# EUROPEAN PATENT SPECIFICATION

(11) **EP 1 581 505 B1**
(45) Date of publication and mention of the grant of the patent: **01.08.2007**
(21) Application number: 03775372.0
(22) Date of filing: 19.11.2003
(51) Int. Cl.: C07D 237/24, C07D 401/04, C07D 401/14, C07D 413/14, C07D 401/12, C07D 403/04, C07D 405/14, C07D 409/14, C07D 409/04, C07D 413/04, C07D 513/04, C07D 417/04, A61K 31/50, A61P 25/00

(54) **PYRIDAZINONE DERIVATIVES AS GSK-3BETA INHIBITORS**
PYRIDAZINON-DERIVATE ALS GSK-3BETA-HEMMER
DERIVES DE PYRIDAZINONES EN TANT QU'INHIBITEURS DE GSK-3BETA

(30) Priority: 19.11.2002 FR 0214443
(43) Date of publication of application: 05.10.2005
(73) Proprietor: Sanofi-Aventis Deutschland GmbH, 65929 Frankfurt am Main (DE)
(72) Inventor: HOELDER, Swen, 60594 Frankfurt (DE); NAUMANN, Thorsten, 63697 Hirzenhain (DE); SCHOENAFINGER, Karl, 63755 Alzenau (DE); WILL, David William, 65926 Frankfurt am Main (DE); MATTER, Hans, 63505 Langenselbold (DE); MUELLER, Guenter, 65843 Sulzbach (DE); LE SUISSE, Dominique, F-93100 Montreuil (FR); BAUDOIN, Bernard, F-92370 Chaville (FR); ROONEY, Thomas, F-91400 Orsay (FR); HALLEY, Franck, F-92310 Sevres (FR); TIRABOSCHI, Gilles, Vitry-sur-Seine Vitry/Alforville 94403 Cedex (FR)
(86) International application number: PCT/EP2003/012950
(87) International publication number: WO 2004/046117

(56) References cited:
- EP-A- 1 061 077
- WO-A-02/22587
- WO-A-02/50065
- US-A- 5 418 233
- PATENT ABSTRACTS OF JAPAN vol. 1997, no. 12, 25 December 1997 (1997-12-25) & JP 09 216883 A (FUJISAWA PHARMACEUT CO LTD), 19 August 1997 (1997-08-19)

## Description

The present invention relates to compounds according to the general formula **(I),** with the definitions of the substituents A and Ar given below in the text, as well as their physiologically acceptable salts, methods for producing these compounds and their use for the preparation of a medicament.

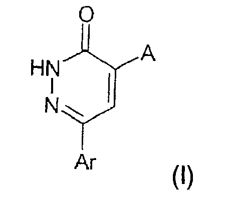

These compounds are kinase inhibitors, in particular inhibitors of the kinase GSK-3β (glycogen synthase kinase-3β).

It is known from literature that in the case of metabolic diseases such as diabetes or neurodegenerative diseases such as Alzheimer's disease, there is a connection between the therapy of said diseases and the inhibition of GSK-3β or the phosphorylation of the tau-protein (S.E. Nikoulina. Diabetes 51, 2190-2198, 2002; Henrikson. Am. J. Physiol. 284, E892-900, 2003). Many compounds or pharmaceuticals, respectively, are already known to be employed for the treatment of said diseases, which compounds interfere at different places of the biochemical processes causing the respective disease. However, there are no compounds known until now, which effect inhibition of GSK-3β.

Pyridazinone derivatives are well known pharmaceuticals, but it has not been reported so far that pyridazinone derivatives can be employed for the inhibition of GSK-3β or tau-phosporylation, respectively. Pyridazinone derivatives described in literature differ from those of the present invention due to a different substitution pattern and (partially) different indications.

WO 03/059891 discloses pyridazinone derivatives that are useful for treating diseases and conditions caused or exacerbated by unregulated p38 MAP Kinase activity and/or TNF activity. The compounds described therein can be used, for example, for the treatment of inflammatory conditions, diabetes, Alzheimer's disease or cancer. They differ from the compounds of the present invention in the substitution of the pyridazinone cycle, since the nitrogen at position 2 of the cycle is mostly substituted with alky, aryl or heteroaryl and at position 4 of the cycle there is no amido group defined as substituent (equals substituent A of the compounds of the present invention).

The documents EP-A 075 436, US 4,734,416 and US 4,353,905 describe pyridazinone derivatives as antihypertensive agents and as agents which increase cardiac contractibility. These pyridazinone derivatives have a phenyl residue at position 6 of the pyridazinone cycle, said phenyl residue is additionally substituted with a heterocycle containing at least one nitrogen atom. Whereas the pyridazinone derivatives described in the documents EP-A 075 436 and US 4,353,905 do not have a substituent at position 4 of the pyridazinone cycle, those disclosed in US 4,734,415 may have an amido group substituted with lower alkyl at this position. Compounds as such, explicitly disclosed by US 4,743,415, are not a subject of the present invention.

JP 09 216883A (Fujisawa Pharmaceutical Co., Ltd.) describes novel pyrazolo pyridine compounds having adenosine antagonistic action. By accidental disclosure compounds of Examples 8, 9, and 10 are mentioned, which are 3-{4-(3,4,5-trimethoxyanilino-carbonyl)-3-oxo-2,3-dihydropyrrdazine-6-yl}-2-phenyl-pyrazolo[1,5-a]pyridine, 3-{4-N-ethoxycarbonylmethyl)-carbamoyl-3-oxo-2,3,-dihydro-pyridazine-6-yl}-2-phenyl-pyrazolo[1,5-a]pyridine, 3-{4-N-carboxymethyl}-carbamoyl-3-oxo-2,3-dihydro-pyridazine-6-yl}-2-phenyl-pyrazolo[1,5-a]pyridine. The said compounds are excluded from the subject matter of claim 1 by a proviso.

EP 1 0161 077 A1 describes novel pyridazine derivatives and drugs. As an intermediate 6-(4-methoxyphenyl)-4-methylcarbamoyl-(2H)-pyridazin-3-one is mentioned, which is excluded from the subject matter of claim 1 by a proviso.

Thus, there exists a strong need for compounds having an inhibitory effect, for GSK-3β and/or the phosphorylation of the tau-protein. The object of the present invention is to provide compounds showing this ability.

This object is attained by pyridazinone derivatives according to the below-mentioned formula (I) 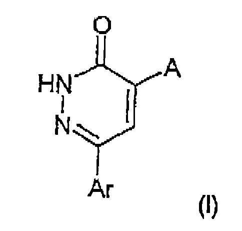 wherein A represents A1 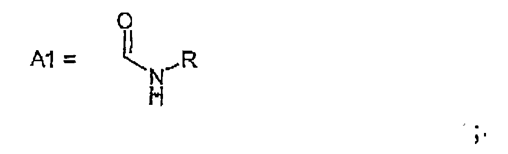
- R: is unsubstituted or at least monosubstituted C₁-C₁₀-alkyl, aryl, aryl-(C₁-C₁₀-alkyl)-, heteroaryl, heteroaryl-(C₁-C₁₀-alkyl)-, heterocyclyl, heterocyclyl-(C₁-C₁₀-alkyl)-, C₃-C₁₀-cycloalkyl, polycycloalkyl, C₂-C₁₀-alkenyl or C₂-C₁₀-alkinyl,
where the substituents are selected from halogen, -CN, C₁-C₁₀-alkyl, -NO₂,OR₁, -C(O)OR1, -O-C(O)R1, -NR1R2, -NHC(O)R1, -C(O)NR1R2, -SR1,S(O)R1, -SO₂R1, -NHSO₂R1, -SO₂NR1R2, -C(S)NR1R2, -NHC(S)R1, -O-SO₂R1, -SO₂-O-R1, oxo, -C(O)R1, -C(NH)NH₂, heterocyclyl, C₃-C₁₀-cycloalkyl, aryl-(C₁-C₆-alkyl)-, aryl, heteroaryl, trifluoromethyl, trifluoromethylsulfanyl and trifluoromethoxy,
and aryl, heterocyclyl and heteroaryl may in turn be at least monosubstituted with C₁-C₆-alkyl, C₁-C₆-alkoxy, halogen, trifluoromethyl, trifluoromethoxy or OH;
- Ar: is unsubstituted or at least monosubstituted aryl or heteroaryl;
where the substituents are selected from halogen, -CN, NO₂, C₁-C₁₀-alkyl, -OR1, -C(O)OR1, -O-C(O)R1, -NR1R2, -NHC(O)R1, -C(O)NR1R2, -NHC(S)R1, -C(S)NR1R2, -SR1, -S(O)R1, -SO₂R1, -NHSO₂R1, -SO₂NR1R2, -O-SO₂R1, -SO₂-O-R1, aryl, heteroaryl, aryl-(C₁-C₆-alkyl)-, formyl, trifluoromethyl and trifluoromethoxy,
and aryl and heteroaryl may in turn be at least monosubstituted with C₁-C₆-alkyl, C₁-C₆-alkoxy, halogen, trifluoromethyl, trifluoromethoxy or OH;
- R1 and R2: are independently from each other
hydrogen;
unsubstituted or at least monosubstituted C₁-C₁₀-alkyl, C₃-C₁₀-cycloalkyl, aryl, aryl-(C₁-C₁₀-alkyl)-, C₂-C₁₀-alkenyl, C₂-C₁₀-alkinyl, heterocyclyl, heterocyclyl-(C₁-C₁₀-alkyl)- or heteroaryl, where the substituents are selected from halogen, C₁-C₆-alkyl, C₁-C₆)alkoxy, ON, NO₂ , NH₂, (C₁-C₆-alkyl)amino-, di(C₁-C₆-alkyl)amino-, OH, COOH, -COO-(C₁-C₆-alkyl), -CONH₂, formyl, trifluoromethyl and trifluoromethoxy;
heteroaryl is a 5 to 10-membered, aromatic, mono- or bicyclic heterocycle containing one or more heteroatoms selected from N, O und S;
aryl is phenyl, indanyl, indenyl or naphthyl;
heterocyclyl is a 5 to 10-membered, aliphatic, mono- or bicyclic heterocycle containing one or more heteroatoms selected from N, O and S;
or the racemates, enantiomers, diasteromeres and mixtures thereof, the tautomers or the physiologically acceptable salts thereof.
with the proviso that A is not -C(O)NH(C₁-C₆-alkyl), in case Ar is phenyl which is at least monosubstituted with heterocyclyl or heteroaryl containing nitrogen, and that the following compounds are excluded: 3-{4-(3,4,5-trimethoxyanilino-carbonyl)-3-oxo-2,3-dihydropyrrdazine-6-yl}-2-phenyl-pyrazolo[1,5-a]pyridine, 3-{4-N-ethoxycarbonylmethyl)-carbamoyl-3-oxo-2,3,-dihydro-pyridazine-6-yl}-2-phenyl-pyrazolo[1,5-a]pyridine, 3-{4-N-carboxymethyl}-carbamoyl-3-oxo-2,3-dihydro-pyridazine-6-yl}-2-phenyl-pyrazolo[1,5-a]pyridine, 6-(4-cyanophenyl)-4-[(4-carboxybutyl)-aminocarbonyl]-(2H)-pyridazin-3-one and 6-(4-methoxyphenyl)-4-methylcarbamoyl-(2H)-pyridazin-3-one.

If in the compounds of formula (I), groups, fragments, residues or substituents such as, for example, aryl, heteroaryl, alkyl etc., may be present several times, they all independently from each other have the meanings indicated and may hence, in each individual case, be identical with or different from each other. The following comments apply to (for example) aryl as well as to any other residue independently from its classification as aryl group, -substituent, -fragment or - residue. One example is the di(C₁-C₁₀-alkyl)amino group in which the alkyl substitutents may be identical or different (for instance 2 x ethyl or 1 x propyl and 1 x heptyl).

If in the above-mentioned definitions of compounds according to formula (I) a substituent, for example aryl, may be unsubstituted or at least mono-substituted with a group of further substituents, for example, (C₁-C₆-alkyl, C₁-C₆-alkoxy, halogen etc., it applies in such cases, where there is a poly-substitution of aryl, that the selection from the group of further substituents is independently from each other. Thus, all combinations of further substituents are comprised in the case of, for example, a double-substitution of aryl, Therefore, aryl may be substituted twice with ethyl, aryl may be mono-substituted with methyl or ethoxy, respectively, aryl may be mono-substituted with ethyl or fluoro, respectively, aryl may be substituted twice with methoxy, etc..

Alkyl, alkenyl and alkynyl residues may be linear or branched. This also applies when they are part of other groups, for example in alkoxy groups (C₁-C₁₀-alkyl-O-), alkoxycarbonyl groups or amino groups, or when they are substituted.

Examples for alkyl groups are: methyl, ethyl, propyl, butyl, pentyl, hexyl, heptyl, octyl, nonyl, decyl. This comprises both the n-isomers of these residues and isopropyl, isobutyl, isopentyl, sec-butyl, tert-butyl, neopentyl, 3,3-dimethylbutyl etc.. Furthermore, unless stated otherwise, the term alkyl here also includes unsubstituted alkyl residues as well as alkyl residues which are substituted by one or more, for example one, two, three or four, identical or different residues, for example aryl, heteroaryl, alkoxy or halogen. The substituents may be present in any desired position of the alkyl group.

Examples for cycloalkyl residues are: cyclopropyl, cyclobutyl, cyclopentyl, cyclohexyl, cycloheptyl and cyclooctyl. All cycloalkyl groups may be unsubstituted or optionally substituted by one or more further residues, as exemplified above in the case of the alkyl groups.

Examples for alkenyl and alkynyl groups are the vinyl residue, the 1-propenyl residue, the 2-propenyl residue (allyl residue), the 2-butenyl residue, the 2-methyl-2-propenyl residue, the 3-methyl-2-butenyl residue, the ethynyl residue, the 2-propynyl residue (propargyl residue), the 2-butynyl residue or the 3-butynyl residue. The term alkenyl here also expressly includes cycloalkenyl residues and cycloalkenyl-alkyl-residues (alkyl substituted by cycloalkenyl) containing at least three carbon atoms. Examples for cycloalkenyl residues are cyclopentenyl, cyclohexenyl, cycloheptenyl and cyclooctenyl.

The alkenyl residues may have 1 to 3 conjugated or unconjugated double bonds in a straight or branched chain; the same applies to alkynyl residues in respect of triple bonds. The alkenyl and alkinyl residues may be unsubstituted or optionally substituted by one or more further residues, as exemplified above in the case of the alkyl groups.

Examples for polycycloalkyl residues are: adamantyl, quinuclidinyl, bornanyl, norbornanyl, bornenyl and norbornenyl.

As already stated the above-mentioned aryl, heteroaryl and heterocyclic residues may be unsubstituted or may carry one or more, for example one, two, three or four of the substituents indicated in the above definition, which substituents may be in any desired position. In monosubstituted phenyl residues, for example, the substituent may be in the 2-position, the 3-position or the 4-position, in disubstituted phenyl residues the substituents may be in 2,3-position, 2,4-position, 2,5-position, 2,6-position, 3,4-position or 3,5-position. In trisubstituted phenyl residues the substituents may be in 2,3,4-position, 2,3,5-position, 2,3,6-position, 2,4,5-position, 2,4,6-position or 3,4,5-position. In fourfold substituted phenyl residues, the substituents may be in the 2,3,4,5-position, the 2,3,4,6-position, or the 2, 3,5,6-position.

The above definitions as well as the following definitions relating to monovalent residues equally apply to the divalent residues phenylene, naphthylene and heteroarylene. Those divalent residues (fragments) may be attached to the adjacent groups by any ring carbon atom. In the case of a phenylene residue, these may be in 1,2-position (ortho-phenylene), 1,3-position (meta-phenylene) or 1,4-position (para-phenylene). In the case of 5-membered ring aromatics containing one heteroatom such as, for example, thiophene or furan, the two free bonds may be in 2,3-position, 2,4-position, 2,5-position or 3,4-position. A divalent residue derived from pyridine may be a 2,3-, 2,4-, 2,5-, 2,6-, 3,4- or 3,5-pyridinediyl residue. In the case of unsymmetrical divalent residues the present invention includes all positional isomers, i. e., in the case of a 2,3-pyridinediyl residue, for example, it includes the compound in which the one adjacent group is present in the 2-position and the other adjacent group is present in the 3-position as well as the compound in which the one adjacent group is present in the 3-position and the other adjacent group is present in the 2-position.

Unless stated otherwise, heteroaryl residues, heteroarylene residues, heterocyclyl residues, heterocyclylen residues and rings which are formed by two groups bonded to a nitrogen are preferably derived from completely saturated, partially saturated or completely unsaturated heterocycles (i.e. heterocycloalkanes, heterocycloalkenes, heteroaromatics), which contain one, two, three or four heteroatoms, which may be identical or different; more preferably they are derived from heterocycles which contain one, two, or three, in particular one or two, heteroatoms, which may be identical or different. Unless stated otherwise, the heterocycles may be monocyclic or polycyclic, for example monocyclic, bicyclic or tricyclic. Preferably they are monocyclic or bicyclic. The rings preferably are 5-membered rings, 6-membered rings or 7-membered rings. Examples of monocyclic and bicyclic heterocyclic systems from which residues occuring in the compounds of the formula (I) may be derived, are pyrrole, furan, thiophene, imidazole, pyrazole, 1,2,3-triazole, 1,2,4-triazole, 1,3-dioxole, 1,3-oxazole (= oxazole), 1,2-oxazole (= isoxazole), 1,3-thiazole (= thiazole), 1,2-thiazole (= isothiazole), tetrazole, pyridine, pyridazine, pyrimidine, pyrazine, pyran, thiopyran, 1,4-dioxine, 1,2-oxazine, 1,3-oxazine, 1,4-oxazine, 1,2-thiazine, 1,3-thiazine, 1,4-thiazine, 1,2,3-triazine, 1,2,4-triazine, 1,3,5-triazine, 1,2,4,5-tetrazine, azepine, 1,2-diazepine, 1,3-diazepine, 1,4-diazepine, 1,3-oxazepine, 1,3-thiazepine, indole, benzothiophene, benzofuran, benzothiazole, benzimidazole, benzodioxol, quinoline, isoquinoline, quinazoline, quinoxaline, phthalazine, thienothiophenes, 1,8-naphthyridine and other naphthyridines, pteridin, or phenothiazine, each of them in saturated form (perhydro form) or in partially unsaturated form (for example in the dihydro form or the tetrahydro form) or in maximally unsaturated form, in case the respective forms are known and stable. The term "aryl" and the term "heteroaryl" as used herein comprise bicyclic residues in which both cycles are aromatic as well as bicyclic residues in which only one cycle is aromatic. Suitable aliphatic heterocycles include, for example, the saturated heterocycles pyrrolidine, piperidine, piperazine, imidazolidine, pyrazolidine, isothiazolidine, thiazolidine, isoxazolidine, oxazolidine, tetrahydrofuran, dioxolane, 2-oxo-azepane, morpholine and thiomorpholine as well as the partially unsaturated heterocycles isochromamyl, chromamyl, 1,2,3,4-tetrahydroisochinolyl and 1,2,3,4-tetrahydrochinolyl. The degree of saturation of heterocyclic groups is indicated in their individual definitions.

Substituents which may be derived from these heterocycles may be attached via any suitable carbon atom. Residues derived from nitrogen heterocycles may carry a hydrogen atom or a substituent on a ring nitrogen atom, and examples include pyrrole, imidazole, pyrrolidine, morpholine, piperazine residues, etc. Those nitrogen heterocyclic residues may also be attached via a ring nitrogen atom, in particular if the respective heterocyclic residue is bonded to a carbon atom. For example, a thienyl residue may be present as 2-thienyl or 3-thienyl, a piperidinyl residue as 1-piperidinyl (= piperidino), 2-piperidinyl, 3-piperidinyl or 4-piperidinyl. Suitable nitrogen heterocycles may also be present as N-oxides or as quarternary salts containing a counterion which is derived from a physiologically acceptable acid. Pyridyl residues, for example, may be present as pyridine-N-oxides.

Arylalkyl means an alkyl residue, which in turn is substituted by an aryl residue. Heteroarylalkyl means an alkyl residue, which in turn is substituted by a heteroaryl residue. Heterocyclylalkyl means an alkyl residue, which in turn is substituted by a heterocyclyl residue. For the definitions and possible substitutions of alkyl, heteroaryl, heterocyclyl and aryl it is referred to the above-mentioned definitions.

Halogen is fluorine, chlorine, bromine or iod, preferably fluorine, chlorine or bromine, most preferably fluorine or chlorine.

The present invention includes all stereoisomeric forms of the compounds of the formula (I). Centers of asymmetry that are present in the compounds of formula (I) all independently of one another have S configuration or R configuration. The invention includes all possible enantiomers and diastereomers and mixtures of two or more stereoisomers, for example mixtures of enantiomers and/or diastereomers, in all ratios. Thus, compounds according to the present invention which may exist as enantiomers may be present in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. All these forms are an object of the present invention. The preparation of individual stereoisomers may be carried out, if desired, by separation of a mixture by customary methods, for example by chromatography or crystallization, by the use of stereochemically uniform starting materials for the synthesis or by stereoselective synthesis. Optionally, a derivatization may be carried out before a separation of stereoisomers. The separation of a mixture of stereoisomers may be carried out at the stage of the compounds of the formula (I) or at the stage of an intermediate during the synthesis. The present invention also includes all tautomeric forms of the compounds of formula (I), in particular keto-enol tautomerism, i.e. the respective compounds may be present either in their keto form or in their enol form or in mixtures thereof in all ratios.

In case the compounds according to formula (I) contain one or more acidic or basic groups, the invention also comprises their corresponding physiologically or toxicologically acceptable salts.

Physiologically acceptable salts are particularly suitable for medical applications, due to their greater solubility in water compared with the starting or base compounds. Said salts must have a physiologically acceptable anion or cation. Suitable physiologically acceptable acid addition salts of the compounds of the invention are salts of inorganic acids such as hydrochloric acid, hydrobromic acid, phosphoric acid, metaphosphoric acid, nitric acid, sulfonic acid and sulfuric acid and also of organic acids such as, for example, acetic acid, theophyllinacetic acid, methylene-bis-b-oxynaphthoic acid, benzenesulfonic acid, benzoic acid, citric acid, ethanesulfonic acid, salicylic acid, fumaric acid, gluconic acid, glycolic acid, isethionic acid, lactic acid, lactobionic acid, maleic acid, malic acid, methanesulfonic acid, succinic acid, p-toluenesulfonic acid, tartaric acid and trifluoroacetic acid. Suitable pharmaceutically acceptable basic salts are ammonium salts, alkali metal salts (such as sodium salts and potassium salts) and alkaline earth metal salts (such as magnesium salts and calcium salts).

Salts having a pharmaceutically unacceptable anion are likewise included within the scope of the present invention as useful intermediates for preparing or purifying pharmaceutically acceptable salts and/or for use in nontherapeutic applications, for example in-vitro applications.

If the compounds of the formula (I) simultaneously contain acidic and basic groups in the molecule, the invention also includes, in addition to the salt forms mentioned, inner salts or betaines (zwitterions).

The respective salts according to the formula (I) may be obtained by customary methods which are known to the person skilled in the art like, for example by contacting these with an organic or inorganic acid or base in a solvent or dispersant, or by anion exchange or cation exchange with other salts.

The present invention furthermore includes all solvates of compounds of the formula (I), for example hydrates or adducts with alcohols, active metabolites of the compounds of the formula (I), and also derivatives, which contain physiologically tolerable and cleavable groups, for example esters or amides.

The compounds of the invention may also be present in various polymorphous forms, for example as amorphous and crystalline polymorphous forms. All polymorphous forms of the compounds of the invention are included within the scope of the invention and are another aspect of the invention.

In an embodiment, compounds of formula (I) are preferred, wherein
- R: is unsubstituted or at least monosubstituted C₁-C₁₀-alkyl, aryl, aryl-(C₁-C₁₀-alkyl)-, heterocyclyl, heterocyclyl-(C₁-C₁₀-alkyl)-, C₃-C₁₀-cycloalkyl, heteroaryl or heteroaryl-(C₁-C₁₀-alkyl)-,
where the substituents are selected from halogen, -CN, C₁-C₁₀-Alkyl, -NO₂, OR1, -C(O)OR1, -O-C(O)R1, -NR1R2, -NHC(O)R1, -C(O)NR1R2, -SR1, -S(O)R1, -SO₂R1, -NHSO₂R1, -SO₂NR1R2, -C(S)NR1R2, -NHC(S)R1, -O-SO₂R1, -SO₂-O-R1, oxo, -C(O)R1, -C(NH)NH₂, heterocyclyl, C₃-C₁₀-cycloalkyl, aryl-(C₁-C₆-alkyl)-, aryl, heteroaryl, trifluoromethyl, trifluoromethylsulfanyl and trifluoromethoxy,
and aryl, heterocyclyl and heteroaryl may in turn be at least monosubstituted with C₁-C₆-alkyl, C₁-C₆-alkoxy, halogen, trifluoromethyl, trifluoroethoxy or OH;
- R1 and R2: are independently from each other
hydrogen;
unsubstituted or at least monosubstituted C₁-C₁₀-alkyl, C₃-C₁₀-cycloalkyl, aryl, aryl-(C₁-C₁₀-alkyl)-, C₂-C₁₀-alkenyl, C₂-C₁₀-alkinyl, heterocyclyl, heterocyclyl-(C₁-C₁₀-alkyl)- or heteroaryl, where the substituents are selected from halogen, C₁-C₆-alkyl, C₁-C₆-alkoxy, CN, NO₂, NH₂, (C₁-C₆-alkyl)amino-, di(C₁-C₆-alkyl)amino-, OH, COOH, -COO-(C₁-C₆-alkyl), -CONH₂, formyl, trifluoromethyl and trifluoromethoxy,
heteroaryl is a 5 to 10-membered, aromatic, mono- or bicyclic heterocycle containing one or more heteroatoms selected from N, O and S;
aryl is phenyl, indanyl, indenyl or naphthyl;
heterocyclyl is a 5 to 10-membered, aliphatic, mono- oder bicyclic heterocycle, containing one or more heteroatoms selected from N, O and S;

In another embodiment, compounds of formula (I) are preferred, wherein
- Ar: is unsubstituted or at least monosubstituted phenyl, pyridinyl, pyrimidinyl, pyrazolyl, thiophenyl, isoxaloyl, benzo[b]thiophemyl, benzodioxolyl or thiazolo[3,2-b][1,2,4]-tiazolyl,
where the substituents are selected from halogen, -CN, NO₂, C₁-C₁₀-alkyl, -OR1, -C(C)OR1, -O-C(O)R1, -NR1R2, -NHO(O)R1, -C(O)NR1R2, -NHC(S)R1, -C(S)NR1R2, -SR1, -S(O)R1, -SO₂R1, -NHSO₂R1, -SO₂NR1R2, -O-SO₂R1, -SO₂-O-R1, aryl, heteroaryl, aryl-(C₁-C₆-alkyl)-, formyl, trifluoromethyl and trifluoromethoxy,
and aryl and heteroaryl may in turn be at least monosubstituted with C₁-C₆-alkyl, C₁-C₆-alkoxy, halogen, trifluoromethyl, trifluoromethoxy or OH;
- R1 and R2: are independently from each other
hydrogen;
unsubstituted or at least monosubstituted C₁-C₁₀-alkyl, C₃-C₁₀-cycloalkyl, aryl, aryl-(C₁-C₁₀-alkyl)-, C₂-C₁₀-alkenyl, C₂-C₁₀-alkinyl, heterocyclyl, heterocyclyl-(C₁-C₁₀-alkyl)- or heteroaryl, where the substituents are selected from halogen, C₁-C₈-alkyl, C₁-C₆-alkoxy, CN, NO₂ , NH₂, (C₁-C₆-alkyl) amino-, di(C₁-C₆-alkyl)amino-, OH, COOH, -COO,(C₁-C₆-alkyl), -CONH₂, formyl, trifluoromethyl and trifluoromethoxy;
heteroaryl is a 5 to 10-membered aromatic, mono- or bicyclic heterocycle, containing one or more heteroatoms selected from N, O and S;
aryl is phenyl, indanyl, indenyl or naphthyl;
heterocyclyl is a 5 to 10-membered aliphatic, mono- oder bicyclic heterocycle, containing one or more heteroatoms selected from N, O and S

Compounds of formula (I) are more preferred, wherein
- R: is unsubstituted or at least monosubstituted C₁-C₁₀-alkyl, aryl, aryl-(C₁-C₁₀-alkyl)-, heterocyclyl, heterocyclyl-(C₁-C₁₀-alkyl)-, C₃-C₁₀-cycloalkyl, heteroaryl or heteroaryl-(C₁-C₁₀-alkyl)-,
where the substituents are selected from halogen. C₁-C₁₀-alkyl, -OR1, -C(O)OR₁, -NR1R2, -C(O)NR1R2, -SR1, -SO₂R1, -SO₂NR1R2, oxo, -C(O)R1, -C(NH)NH₂, heterocyclyl, C₃-C₁₀-cycloalkyl, aryl-(C₁-C₆-alkyl)-, aryl, trifluoromethyl and trifluoromethoxy,
and aryl, heterocyclyl and heteroaryl may in turn be at least monosubtituted with C₁-C₆-alkyl, C₁-C₆-alkoxy, halogen, trifluoromethyl, trifluoromethoxy or OH;
- Ar: is unsubstituted or at least monosubstituted phenyl, pyridinyl, pyrimidinyl, pyrazolyl, thiophenyl, isoxazolyl, benzo[b]thiophenyl, benzodioxolyl or thiazolo[3,2-b][1,2,4]-tiazolyl,
where the substituents are selected from halogen, C₁-C₁₀-alkyl, -OR1, -C(O)OR1, -NR1R2, -C(O)NR1R2, aryl, heteroaryl, aryl-(C₁C₆-alkyl)-, trifluoromethyl and Trifluoromethoxy,
and aryl and heteroaryl may in turn be at least monosubsituted with C₁-C₆-alkyl, C₁-C₆-alkoxy, halogen, trifluoromethyl or OH;
- R1 und R2: are independently from each other
hydrogen;
unsubstituted or at least monosubstituted C₁-C₁₀-alkyl, C₃-C₁₀-cycloalkyl, aryl, aryl-(C₁-C₁₀-alkyl)-, heterocyclyl, heterocyclyl-(C₁-C₁₀-alkyl)- or heteroaryl, where the substituents are selected from halogen, C₁-C₆-alkyl, C₁-C₆-alkoxy, NH₂, (C₁-C₆-alkyl)amino-, di(C₁-C₆-alkyl)amino-, OH, trifluoromethyl and trifluoromethoxy;
heteroaryl is a 5 to 10-membered, aromatic, mono- or bicyclic heterocycle containing one or more heteroatoms selected from N, O und S; heteroaryl is preferably imidazolyl, thiophenyl, furanyl, isoxazolyl, pyridinyl, pyrimidinyl, 1,2,3,4-tetrahydrochinolinyl, benzoimidazolyl, indolyl or benzodioxolyl;
aryl is phenyl, indanyl, indenyl or naphthyl; aryl is preferably phenyl or naphthyl,
heterocyclyl is a 5 to 10-membered, aliphatic, mono- br bicyclic heterocycle containing one or more heteroatoms selected from N, O und S; heterocyclyl is preferably 2-oxo-azepanyl, tetrahydrofuranyl, 1,3-dioxolanyl, morpholinyl, piperazinyl or piperidinyl;

Compounds of formula (I) are even more preferred, wherein
- R: is unsubstituted or at least monosubstituted C₁-C₁₀-alkyl, aryl, aryl-(C₁-C₁₀-alkyl)-, heterocyclyl, heterocyclyl-(C₁-C₁₀-alkyl)-, C₃-C₁₀-cycloalkyl, heteroaryl or heteroaryl-(C₁-C₁₀-alkyl)-,
where the substituents are selected from halogen, C₁-C₁₀-alkyl, -OR1, -C(O)OR1, -NR1R2, -C(O)NR1R2, -SR1, -SO₂R1, -SO₂NR1R2, oxo, -C(O)R1, -C(NH)NH₂, heterocyclyl, C₃-C₁₀-cycloalkyl, aryl-(C₁-C₆-alkyl), aryl, trifluoromethyl and trifluoromethoxy,
and aryl, heterocyclyl and heteroaryl may in turn be at least monosubsituted with C₁-C₆-alkyl, C₁-C₆-alkoxy, halogen, trifluoromethyl, trifluoromethoxy or OH;
- Ar: is unsubstituted or at least monosubstituted phenyl, pyridinyl or pyrimidinyl,
where the substituents are selected from halogen, C₁-C₁₀-alkyl, -OR1, -C(O)OR1, -NR1R2, -C(O)NR1R2, aryl, heteroaryl, aryl-(C₁-C₆-alkyl)-, trifluoromethyl and trifluoromethoxy,
and aryl and heteroaryl may in turn be at least monosubsituted with C₁-C₆-alkyl, C₁-C₆-alkoxy, halogen, trifluoromethyl or OH;
- R1 und R2: are independently from each other
hydrogen;
unsubstituted or at least monosubstituted C₁-C₁₀-alkyl, C₃-C₁₀-cycloalkyl, aryl, aryl-(C₁-C₁₀-alkyl)-, heterocyclyl, heterocyclyl-(C₁-C₁₀-alkyl)- or heteroaryl, where the substituents are selected from halogen, C₁-C₆-alkyl, C₁-C₆-alkoxy, NH₂, (C₁-C₆-alkyl)amino-, di(C₁-C₆-alkyl)amino-, OH, trifluoromethyl and trifluoromethoxy;
heteroaryl is a 5 to 10-membered, aromatic, mono- or bicyclic heterocycle containing one or more heteroatoms selected from N, O und S; heteroaryl is preferably imidazolyl, thiophenyl, furanyl, isoxazolyl, pyridinyl, pyrimidinyl, 1,2,3,4-tetrahydrochinolinyl, benzoimidazolyl, indolyl or benzodioxolyl;
aryl is phenyl, indanyl, indenyl or naphthyl; aryl is preferably phenyl or naphthyl.
heterocyclyl is a 5 to 10-membered, aliphatic, mono- or bicyclic heterocycle containing one or more heteroatoms selected from N, O und S; heterocyclyl is preferably 2-oxo-azepanyl, tetrahydrofuranyl, 1,3-dioxolanyl, morpholinyl, piperazinyl or piperidinyl;

Compounds of formula (1) are even much more preferred, wherein
- R: is unsubstituted or at least monosubstituted aryl-(C₁-C₆-alkyl)- or heteroaryl-(C₁-C₆-alkyl)-,
where the substituents are selected from halogen, C₁-C₆-alkyl, -OH, -O-aryl, C₁-C₆-alkoxy, -O-(C₁-C₆-alkylen)-N(C₁-C₆-alkyl)₂, -C(O)OH, -C(O)O-(C₁-C₆-alkyl), -NH₂, -N(C₁-C₆-alkyl)₂, -NH(C₁-C₆-alkyl), -NH(C₁-C₁₀-cycloalkyl), -C(O)NH₂, -C(O)NH-heteroaryl, -C(O)NH-(C₁-C₆-alkyl), -SO₂(C₁-C₆-alkyl), -SO₂NH₂, -C(O)-heterocyclyl, -C(NH)NH₂, heterocyclyl, aryl-(C₁-C₆-alkyl)-, aryl, trifluoromethyl, and trifluoromethoxy,
and aryl, heterocyclyl and heteroaryl may in turn be at least monosubstituted with C₁-C₃-alkyl, C₁-C₃-alkoxy, fluorine, chlorine, bromine, trifluoromethyl, trifluoromethoxy or OH;
heteroaryl is imidazolyl, thiophenyl, furanyl, isoxazolyl, pyridinyl, pyrimidinyl, benzoimidazolyl, indolyl or benzodioxolyl;
aryl is phenyl or naphthyl;
hetrocyclyl is morpholinyl, piperazinyl or piperidinyl

In another embodiment, compounds of formula (I) are even much more preferred, wherein
- Ar: is unsubstituted or at least monosubstituted phenyl, pyridin-4-yl or pyrimidin-4-yl,
where the substituents are selected from halogen, C₁-C₆-alkyl, -OH, C₁-C₆-alkoxy, -C(O)OH, -C(O)O-(C₁-C₆-alkyl), -NH₂, -N(C₁-C₆-alkyl)₂, -NH(C₁-C₆-alkyl), -NH(C₁-C₁₀-cycloalkyl), -NH(heterocyclyl-(C₁-C₆-alkyl-)), -NH(aryl-(C₁-C₆-alkyl-)), -C(O)NH₂, -C(O)NH-(C₁-C₆-alkyl), aryl, and heteroaryl,
and aryl, heterocyclyl and heteroaryl may in turn be at least monosubstituted C₁-C₃-Alkyl, C₁-C₃-alkoxy, fluorine, chlorine, bromine, trifluoromethyl,trifluoromethoxy or OH;
heteroaryl is pyridinyl or pyrimidinyl;
aryl is phenyl or naphthyl;
hetrocyclyl is morpholinyl, piperazinyl or piperidinyl

Compounds of formula (I) are particularly preferred, wherein
- R: is unsubstituted or at least monosubstituted benzyl, phenylethyl-, phenylpropyl-, pyridinylmethyl-, pyridinylethyl- or pyridinylpropyl-,
where the substituents are selected from chlorine, bromine, fluorine, trifluoromethyl and carboxy;
- Ar: is unsubstituted or at least monosubstituted pyridin-4-yl, pyrimidin-4-yl or phenyl,
where the substituents are selected from methylamino-, ethylamino-, propylamino-, butylamino-, hydroxy, methoxy, ethoxy, methyl, ethyl, propyl, (phenylethyl)amino-, benzylamino- and (morpholinylethyl)amino- .

Compounds of formula (I) are exceptionally preferred, which are selected from the group consisting of
6-(2-butylamino-pyrimidin-4-yl)-3-oxo-2,3-dihydro-pyridazine-4-carboxylic acid (3-pyridin- 3-yl-propyl)-amide,
6-(4-hydroxy-3-methoxy-phenyl)-3-oxo-2,3-dihydro-pyridazine-4-carboxylic acid (3-pyridin-3-yl-propyl)-amide,
6-(4-hydroxy-3,5-dimethyl-phenyl)-3-oxo-2,3-dihydro-pyridazine-4-carboxylic acid (3-pyridin-3-yl-propyl)-amide,
6-(4-hydroxy-phenyl)-3-oxo-2,3-dihydro-pyridazine-4-carboxylic acid (3-pyridin-3-yl-propyl)-amide,
6-(2-ethylamino-pyrimidin-4-yl)-3-oxo-2,3-dihydro-pyridazine-4-carboxylic acid 4-chloro- benzylamide,
6-(3-chloro-4-hydroxy-phenyl)-3-oxo-2,3-dihydro-pyridazine-4-carboxylic acid 4-chloro-benzylamide,
6-(4-hydroxy-3,5-dimethyl-phenyl)-3-oxo-2,3-dihydro-pyridazine-4-carboxylic acid 4-chloro-benzylamide,
4-({[6-(4-hydroxy-3,5-dimethyl-phenyl)-3-oxo-2,3-dihydro-pyridazine-4-carbonyl]-aminol- methyl)-benzoic acid,
24-({[6-(4-hydroxy-3-methoxy-phenyl)-3-oxo-2,3-dihydro-pyridazine-4-carbonyl]-aminol- methyl)-benzoic acid,
6-(2-butylamino-pyrimidin-4-yl)-3-oxo-2,3-dihydro-pyridazine-4-carboxylic acid (pyridin-3- ylmethyl)-amide,
6-(3-fluoro-4-hydroxy-phenyl)-3-oxo-2,3-dihydro-pyridazine-4-carboxylic acid 4-chloro-benzylamide,
6-(4-hydroxy-3-methyl-phenyl)-3-oxo-2,3-dihydro-pyridazine-4-carboxylic acid 4-chloro-benzylamide,
6-[2-(2-morpholin-4-yl-ethylamino)-pyrimidin-4-yl]-3-oxo-2,3-dihydro-pyridazine-4- carboxylic acid 4-chloro-benzylamide,
6-(4-hydroxy-3-methoxy-phenyl)-3-oxo-2,3-dihydro-pyridazine-4-carboxylic acid 4-chloro-benzylamide,
6-(2-methylamino-pyrimidin-4-yl)-3-oxo-2,3-dihydro-pyridazine-4-carboxylic acid 4-chloro-benzylamide,
R-3-oxo-6-[2-(1-phenyl-ethylamino)-pyrimidin-4-yl]-2,3-dihydro-pyridazine-4-carboxylic acid (3-phenyl-propyl)-amide,
6-(4-hydroxy-phenyl)-3-oxo-2,3-dihydro-pyridazine-4-carboxylic acid 4-chloro-benzylamide,
3-oxo-6-pyridin-4-yl-N-[4-(trifluoromethyl)benzyl]-2,3-dihydropyridazine-4-carboxamide,
3-oxo-6-pyridin-4-yl-2,3-dihydro-pyridazine-4-carboxylic acid 4-bromo-benzylamide,
3-oxo-6-pyridin-4-yl-N-(pyridin-3-ylmethyl)-2,3-dihydropyridazine-4-carboxamide,
N-(2,4-dichlorobenzyl)-3-oxo-6-pyridin-4-yl-2,3-dihydropyridazine-4-carboxamide,
3-oxo-6-pyridin-4-yl-2,3-dihydro-pyridazine-4-carboxylic acid 4-chloro-2-fluoro-benzylamide, and
N-(4-chlorobenzyl)-3-oxo-6-pyridin-4-yl-2,3-dihydropyridazine-4-carboxamide;

It is explicitly indicated once more that also in case of the preferred, more preferred, even more preferred, even much more preferred, particularly preferred and exceptionally preferred compounds according to formula (I) the above-mentioned explanations also apply in respect of the salts, stereoisomers, N-oxides etc.; in particular, the respective physiologically acceptable salts are included.

The compounds of formula (I) may be obtained from compounds according to formula (II), where X is a functional group, preferably - OH, C₁-C₁₀-alkoxy, chloro or -O-C(O)-(C₁-C₁₀-alkyl). The convertion with the amine (III) may be carried out using an inert solvent at 0 to 150 °C.

In case X is OH, the compounds of formula (I) may be obtained by acylation of the amine derivatives, either using an acid chloride to be added beforehand or by reaction in the presence of an activating agent.

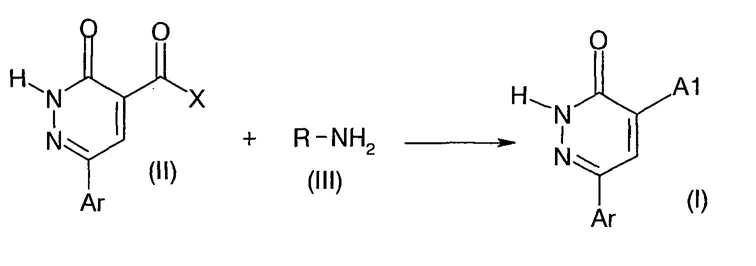

The reaction may be carried out by forming an acid chloride according to any methods known to persons skilled in the art or more precisely by the action of oxalyl chloride in toluene, dichloromethane (R.D.MILLER, J. Org. Chem, 56, (4) 1453, (1991)) which, thus formed, will react with the amine (III) in the presence of a base such as pyridine, triethylamine, diisopropylethylamine; the reaction can start at 0°C and when the addition of the acid chloride is complete, the medium is kept stirred at room temperature (G. DAIDONE, Heterocycles, 43, (11), 2385-96, (1996)) or it is heated if necessary.

The reaction may also be carried out in the presence of an activating agent of the carbodiimide type alone (DCC, EDAC) (M. C. DESAI, Tetrahedron Lett., 34, 7685, (1993)) or in the presence of hydroxybenzotriazole and dimethylaminopyridine (J. P. GAMET, Tetrahedron, 40, 1995, (1984), K. BARLOS, J. Org. Chem., 50, 696, (1985)) or according to well known methods of coupling in peptide chemistry (M. BODANSZKY, Principles of Peptide Synthesis; Springer-Verlag, New York, NY, pages 9-58, (1984)) or of forming the amide bond.

The derivatives of formula (II) are obtained by the method described in patent F.R 2481284 and by Y. Shojiro. Chem. Pharm. Bull; 19 (11) p 2354. It is necessary to protect the reactive functional groups. The protecting groups are introduced according to any methods known to persons skilled in the art and in particular those described by T.W. GREENE, Protective groups in Organic Synthesis, J. Wiley-Interscience Publication (1991). For the phenols, there will be preferably chosen more particularly a benzyl group introduced in the presence of an inorganic base such as sodium carbonate at the reflux temperature of acetone and of acetonitrile (A. R Mac Kenzie, Tetrahedron, 42, 3259, (1986)), which may then be removed by catalytic hydrogenation or more particularly using trifluoroacetic acid under reflux, described in patent W O 9727846.

The products of general formula (III) may be obtained commercially or by functionalization and protection of the reactive functional groups of commercially available products according to the methods described by Larock, Comprehensive Organic Transformations, VCH, New York, 1999. The nitrile functional groups are reduced with hydrogen in the presence of catalysts, BH₃ or more precisely lithium aluminum hydride in solvents such as dioxane or THF (T.M. Koening, Tetrahedron Letters, 35, 1339, (1994)). The phenol functional groups are protected with trimethylsilylethoxymethyl by reacting the starting compound with trimethylsilylethoxymethyl chloride in the presence of sodium hydride in a solvent such as dimethylformamide at room temperature (J. P. WHITTEN, J. Org. Chem., 51, 1891, (1986); M. P. EDWARDS, Tetrahedron, 42, 3723, (1986)). The deprotection is carried out according to methods known to persons skilled in the art and described by T.W. GREENE, Protective groups in Organic Synthesis, J. Wiley-Interscience Publication (1991).

The derivatives of formula (I) for which the protecting group is trimethylsilylethoxymethyl can be deprotected by reaction with tetrabutylammonium fluoride under reflux in solvents such as tetrahydrofuran, dioxane. (J. P. WHITTEN, J. Org. Chem., 51, 1891, (1986); B. H. LIPSHUTZ, Tetrahedron Lett., 4095, (1986)).

The derivatives of formula (I) for which the protecting group is an ester can be saponified according to any methods known to persons skilled in the art and in particular by the action of sodium hydroxide on the reflux (L. Anzalone, J. Org. Chem., 50, 2128, (1985).

Furthermore, compounds according to general formula (I) can be prepared by palladium catalyzed coupling according to a reaction of Suzuki (I. Parrot et al., Synthesis; 7; 1999; 1163-1168). A compound of formula (IV), where Y1 is halogen, B(OH)₂ or Sn(C₁-C₁₀-alkyl) and Y2 is H or a protecting group, is hereby converted with a compound of formula (V).

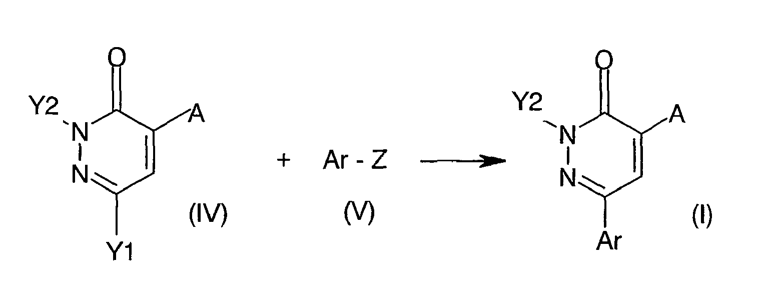

Z may be, for example, B(OH)₂, B(C₁-C₁₀-alkyl)₂, Sn(C₁-C₁₀-alkyl)₃, Zn(C₁-C₁₀-alkyl) or halogen. In case Y2 is a protecting group, said group is removed after the reaction of (IV) and (V) using methods known by a person skilled in the art. All protecting groups known by a person skilled in the art can be used as protecting groups, preferably trimethylsilylethoxymethyl-. For performing the palladium catalyzed coupling all palladium complexes known by a person skilled in the art can be employed, preferably Pd(triphenylphosphin)₄ (Pd-tetrakis-catalyst) is employed, which is preferably obtained in situ from palladium acetate.

The compounds of formula (I) are isolated and may be purified by known methods, for example by crystallization, chromatography or extraction.

The compounds according to the general formula (I) can be used as as pharmaceuticals or medicaments, respectively. With respect to the definition of the substituents A and Ar (as well as all further substituents defined by the before-mentioned substituents) the same explanations as laid out above in the context with the compounds as such apply. This serves as well for the embodiments as of above.

The compounds of general formula (I) are kinase inhibitors and can therefore be employed for the treatment of diseases, which may result from an abnormal activity of kinases. As abnormal kinase activity, there may be mentioned, for example, that of Pl3K, AkT, GSK-3β and the like.

In particular, compounds according to the present invention can be used for the inhibition of the kinase GSK-3β. This effect is particularly relevant for the treatment of metabolic diseases such as type II diabetes or neurodegenerative diseases such as Alzheimer's disease.

Furthermore, compounds according to the general formula (I) have an inhibitory effect in respect of the phosphorylation of the tau-protein. This effect is particularly relevant for the treatment of neurodegenerative diseases such as Alzheimer's disease.

Examples of diseases, which can be treated with the compounds according to the present invention, include: neurodegenerative diseases, strokes, cranial and spinal traumas and peripheral neuropathies, obesity, metabolic diseases, type II diabetes, essential hypertension, atherosclerotic cardiovascular diseases, polycystic ovary syndrome, syndrome X, immunodeficiency or cancer. Neurodegenerative diseases are preferably: Alzheimer's disease, Parkinson's disease, frontoparietal dementia, corticobasal degeneration and Pick's disease.

Compounds according to the present invention are preferably employed for the treatment of metabolic diseases, in particular of type II diabetes.

The compounds according to the general formula (I) can preferably be employed for the treatment of neurodegenerative diseases, in particular of Alzheimer's disease.

In the above-mentioned explanation the item treatment also includes prophylaxis, therapy or curing of the above-mentioned diseases.

All references to "compound(s) according to formula (I)" refer hereinbelow to a compound/compounds of the formula (I) as described above and also to their salts, solvates as described herein.

The compounds of the formula (I) can be administered to animals, preferably to mammals, and in particular to humans. The compounds of the formula (I) can be administered as pharmaceuticals by themselves, in mixtures with one another or in mixtures with other pharmaceuticals or in the form of pharmaceutical preparations. Further subjects of the present invention therefore also are the use of the compounds of the formula (I) for preparing one or more medicaments for prophylaxis and/or treatment of the before-mentioned diseases, pharmaceutical preparations (or pharmaceutical compositions) comprising an effective dose of at least one compound of the formula (I) as well as pharmaceutical preparations comprising an effective dose of at least one compound of the formula (I) for prophylaxis and/or treatment of the before-mentioned diseases

The amount of a compound according to formula (I) which is required in order to attain the desired biological effect depends on a number of factors, for example the specific compound selected, the intended use, the type of administration and the clinical state of the patient. In general, the daily dose is in the range from 0.3 mg to 100 mg (typically from 3 mg to 50 mg) per day per kilogram of body weight, for example 3-10 mg/kg/day. An intravenous dose can be, for example, in the range from 0.3 mg to 1.0 mg/kg and can be administered in a suitable manner as an infusion of 10 ng to 100 ng per kilogram per minute. Suitable infusion solutions for these purposes may contain, for example, from 0.1 ng to 10 mg, typically from 1 ng to 10 mg per milliliter. Individual doses may contain, for example, from 1 mg to 10 g of the active compound. Thus, ampoules for injections can contain, for example, from 1 mg to 100 mg, and orally administerable individual dose formulations such as, for example, tablets or capsules can contain, for example, from 1.0 to 1000 mg, typically from 10 to 600 mg. In the case of pharmaceutically acceptable salts, the abovementioned masses relate to the mass of the free compound on which the salt is based. The compound used for the prophylaxis or therapy of the abovementioned conditions may be the compounds according to formula (I) themselves, but they are preferably present in the form of a pharmaceutical composition together with an acceptable carrier. The carrier must be naturally acceptable, in the sense that it is compatible with the other ingredients of said composition and is not harmful to the patient's health. The carrier may be a solid or a liquid or both and is preferably formulated with the compound as an individual dose, for example as a tablet which may contain from 0.05% to 95% by weight of the active compound. Further pharmaceutically active substances may also be present, including further compounds according to formula (I). The pharmaceutical compositions of the invention may be prepared according to any of the known pharmaceutical methods which essentially comprise mixing the ingredients with pharmacologically acceptable carriers and/or excipients.

Besides at least one compound according to formula (I) as well as one or more carriers, the pharmaceutical preparations can also contain additives. As additives can be employed, for example: fillers, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The pharmaceutical compositions of the invention may be in form of a pill, tablet, lozenge, coated tablet, granule, capsule, hard or soft gelatin capsule, aqueous solution, alcoholic solution, oily solution, syrup, emulsion suspension pastille suppository, solution for injection or infusion, ointment, tincture, cream, lotion, powder, spray, transdermal therapeutic systems, nasal spray, aerosol mixture, microcapsule, implant, rod or plaster.

Pharmaceutical compositions of the invention are those which are suitable for oral, rectal, topical, peroral (e.g. sublingual) and parenteral (e.g. subcutaneous, intramuscular, intradermal or intravenous) administration, although the most suitable manner of administration depends in each individual case on the nature and severity of the condition to be treated and on the nature of the compound according to formula (I) used in each case. Sugar-coated formulations and sugar-coated delayed-release formulations, too, are included within the scope of the invention. Preference is given to acid-resistant and enteric formulations. Suitable enteric coatings include cellulose acetate phthalate, polyvinyl acetate phthalate, hydroxypropyl-methylcellulose phthalate and anionic polymers of methacrylic acid and methyl methacrylate.

Suitable pharmaceutical compounds for oral administration may be present in separate units as, for example, capsules, cachets, lozenges or tablets, which in each case contain a particular amount of the compound according to formula (I); as powders (gelatin capsules or cachets) or granules; as solution or suspension in an aqueous or nonaqueous liquid; or as an oil-in-water or water-in-oil emulsion. As already mentioned, said compositions can be prepared according to any suitable pharmaceutical method which includes a step in which the active compound and the carrier (which may comprise one or more additional components) are contacted. In general, the compositions are prepared by uniform and homogeneous mixing of the active compound with a liquid and/or finely dispersed solid carrier, after which the product is shaped, if necessary. Thus a tablet, for example, may be prepared by pressing or shaping a powder or granules of the compound, where appropriate with one or more additional components. Pressed tablets can be prepared by tableting the compound in free-flowing form, for example a powder or granules, mixed, where appropriate, with a binder, lubricant, inert diluent and/or one or more surface active/dispersing agents in a suitable machine. Shaped tablets can be prepared by shaping the pulverulent compound, moistened with an inert liquid diluent, in a suitable machine. As diluents can be used, for example, starch, cellulose, saccharose, lactose or silica. The pharmaceutical compositions of the invention may also comprise substances other than diluents, for example one or more lubricants such as magnesium stearate or talc, a coloring, a coating (sugar-coated tablets) or a varnish.

Pharmaceutical compositions which are suitable for peroral (sublingual) administration include lozenges which contain a compound according to formula (I) with a flavoring, usually sucrose and gum arabic or tragacanth, and pastilles which comprise the compound in an inert base such as gelatin and glycerol or sucrose and gum arabic.

Suitable pharmaceutical compositions for parenteral administration preferably comprise sterile aqueous preparations of a compound according to formula (I) which are preferably isotonic with the blood of the intended recipient. These preparations are preferably administered intravenously, although they may also be administered subcutaneously, intramuscularly or intradermally as an injection. Said preparations may preferably be prepared by mixing the compound with water and rendering the obtained solution sterile and isotonic with the blood. Injectable compositions of the invention generally contain from 0.1 to 5% by weight of the active compound.

These sterile compositions for parenteral administration may be preferably solutions which are aqueous or non aqueous, suspensions or emulsions. As solvent or vehicle, there may be used water, propylene glycol, polyethylene glycol, vegetable oils, in particular olive oil, organic esters for injection, for example ethyl oleate or other suitable organic solvents. These compositions may also contain adjuvants, in particular wetting, isotonizing, emulsifying, dispersing and stabilizing mediums. The sterilization may be carried out in several ways, for example by an aseptic filtration, by incorporating sterilizing agents into the composition, by irradiation or by heating. They may also be prepared in the form of sterile solid compositions which may be dissolved at the time of use in sterile water or in any other sterile medium for injection.

Suitable pharmaceutical compositions for rectal administration are preferably present as individual dose suppositories. These may be prepared by mixing a compound according to formula (I) with one or more conventional solid carriers, for example cocoa butter, and shaping the resulting mixture.

Suitable pharmaceutical compositions for topical application to the skin are preferably present as ointment, cream, lotion, paste, spray, aerosol or oil. Carriers which may be used are petroleum jelly, lanolin, polyethylene glycols, alcohols and combinations of two or more of these substances. In general, the active compound is present at a concentration of from 0.1 to 15%, for example from 0.5 to 2%, by weight of the composition.

Transdermal administration is also possible. Suitable pharmaceutical compositions for transdermal administration may be present as individual patches which are suitable for long-term close contact with the epidermis of the patient. Such patches suitably contain the active compound in an optionally buffered aqueous solution, dissolved and/or dispersed in an adhesive or dispersed in a polymer. A suitable active compound concentration is from approx. 1 % to 35%, preferably approx. 3% to 15%. A particular possibility is the release of the active compound by electro-transport or iontophoresis, as described, for example, in Pharmaceutical Research, 2(6): 318 (1986).

The following examples illustrate compositions according to the invention:

### EXAMPLE A

Gelatin capsules containing a dose of 50 mg of active product and having the following composition are prepared according to the usual technique:
- Compound of formula (I) 50 mg
- Cellulose 18 mg
- Lactose 55 mg
- Colloidal silica 1 mg
- Sodium carboxymethylstarch 10 mg
- Talc 10 mg
- Magnesium stearate 1 mg

### EXAMPLE B

Tablets containing a dose of 50 mg of active product and having the following composition are prepared according to the usual technique:
- Compound of formula (I)..... 50 mg
- Lactose 104 mg
- Cellulose 40 mg
- Polyvidone 10 mg
- Sodium carboxymethylstarch 22 mg
- Talc 10 mg
- Magnesium stearate 2 mg
- Colloidal silica 2 mg
- Mixture of hydroxymethylcellulose, glycerin, titanium oxide (72-3.5-24.5) qs 1 finished film-coated tablet of 245 mg

### EXAMPLE C

A solution for injection containing 10 mg of active product and having the following composition is prepared:
- Compound of formula (I)..... 10 mg
- Benzoic acid 80 mg
- Benzyl alcohol 0.06 ml
- Sodium benzoate 80 mg
- Ethanol at 95 % 0.4 ml
- Sodium hydroxide 24 mg
- Propylene glycol 1.6 ml
- Water qs 4 ml

Another subject of the present invention is the combination of compounds of the formula (I) with other pharmaceutically active substances not covered by formula (I).

The compounds of the formula (I) are distinguished by beneficial actions on the metabolism of lipids, and they are particularly suitable for weight reduction and, after weight reduction, for maintaining a reduced weight in mammals and as anorectic agents. The compounds are distinguished by their low toxicity and their few side effects.

The compounds may be employed alone or in combination with other weight-reducing or anorectic active compounds. Further anorectic active compounds of this kind are mentioned, for example, in the Rote Liste, Chapter 01 under weight-reducing agents/appetite suppressants, and may also include those active compounds which increase the energy turnover of the organism and thus lead to weight reduction or else those which influence the general metabolism of said organism such that increased calorie intake does not cause an enlargement of the fat depots and a normal calorie intake causes a reduction in the fat depots of said organism. The compounds are suitable for the prophylaxis and, in particular, for the treatment of problems of excess weight or obesity.

The compounds of formula (I) have a beneficial effect on the glucose metabolism, they particularly lower the blood-sugar level and can be used for treatment of type I and type II diabetes. The compounds can therefore be used alone or in combination with other blood-sugar lowering active compounds (antidiabetics). In a further aspect of the invention, the compounds of the formula I may be administered in combination with one or more further pharmacologically active substances which may be selected, for example, from the group consisting of antidiabetics, antiadipose agents, blood-pressure-lowering active compounds, lipid reducers and active compounds for the treatment and/or prevention of complications caused by diabetes or associated with diabetes.

Suitable antidiabetics include insulins, amylin, GLP-1 and GLP-2 derivatives such as, for example, those disclosed by Novo Nordisk A/S in WO 98/08871 and also oral hypoglycemic active compounds.

Said oral hypoglycemic active compounds preferably include sulfonyl ureas, biguanidines, meglitinides, oxadiazolidinediones, thiazolidinediones, glucosidase inhibitors, glucagon receptor antagonists, GLP-1 agonists, potassium channel openers such as, for example, those disclosed by Novo Nordisk A/S in WO 97/26265 and WO 99/03861, insulin sensitizers, activators of insulin receptor kinase, inhibitors of liver enzymes involved in the stimulation of gluconeogenesis and/or glycogenolysis, for example glycogen phosphorase inhibitors, modulators of glucose uptake and glucose elimination, lipid metabolism-modifying compounds such as antihyperlipidemic active compounds and antilipidemic active compounds, for example HMGCoA-reductase inhibitors, inhibitors of cholesterol transport/cholesterol uptake, inhibitors of the reabsorption of bile acid or inhibitors of microsomal triglyceride transfer protein (MTP), compounds which reduce food intake, PPAR and RXR agonists and active compounds which act on the ATP-dependent potassium channel of beta cells.

The present compounds can be administered in combination with insulin.

In another embodiment, the compounds of the invention are administered in combination with a sulfonylurea such as, for example, tolbutamide, glibenclamide, glimepiride, glipizide, gliquidone, glisoxepide, glibornuride or gliclazide. In another embodiment, the compounds of the present invention are administered in combination with a biguanidine such as, for example, metformin. In another embodiment, the compounds of the present invention are administered in combination with a meglitinide such as, for example, repaglinide. In yet another embodiment, the compounds of the present invention are administered in combination with a thiazolidinedione such as, for example, troglitazone, ciglitazone, pioglitazone, rosiglitazone or the compounds disclosed by Dr. Reddy's Research Foundation in WO 97/41097, in particular 5-[[4-[(3,4-dihydro-3-methyl-4-oxo-2-quinazolinylmethoxy]phenyl]methyl]-2,4-thiazolidinedione.

The compounds of the present invention can also be administered in combination with an α-glucosidase inhibitor such as, for example, miglitol or acarbose.

The compounds of the present invention can be administered in combination with an active compound which acts on the ATP-dependent potassium channel of the beta cells, such as, for example, tolbutamide, glibenclamide, glimepiride, glipizide, gliclazide or repaglinide. In yet another embodiment, the compounds of the present invention are administered in combination with an antihyperlipidemic active compound or an antilipidemic active compound such as, for example, cholestyramine, colestipol, clofibrate, fenofibrate, gemfibrozil, lovastatin, pravastatin, simvastatin, atorvastatin, cerivastatin, fluvastatin, probucol, ezetimibe or dextrothyroxine.

The compounds of the present invention can be administered in combination with more than one of the aforementioned compounds, for example in combination with a sulfonylurea and metformin, a sulfonylurea and acarbose, repaglinide and metformin, insulin and a sulfonylurea, insulin and metformin, insulin and troglitazone, insulin and lovastatin, etc.

Furthermore, the compounds of the invention can be be administered in combination with one or more antiadipose agents or appetite-controlling active compounds.

Such active compounds may be selected from the group consisting of CART agonists, NPY antagonists, MC4 agonists, orexin antagonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed serotonin and noradrenalin reuptake inhibitors, 5HT modulators, MAO inhibitors, bombesin agonists, galanin antagonists, growth hormone, growth-hormone-releasing compounds, TRH agonists, uncoupling protein 2 or 3 modulators, leptin agonists, dopamine agonists (bromocriptine, doprexin), lipase/amylase inhibitors, cannabinoid receptor 1 antagonists, modulators of acylation-stimulating protein (ASP), PPAR modulators, RXR modulators, hCNTF mimetics or TR-β agonists.

The antiadipose agent can be leptin or modified leptin. In another embodiment, the antiadipose agent is dexamphetamine or amphetamine. In another embodiment, the antiadipose agent is fenfluramine or dexfenfluramine. In yet another embodiment, the antiadipose agent is sibutramine or the mono- and bis-demethylated active metabolite of sibutramine. In another embodiment, the antiadipose agent is orlistate. In another embodiment, the antiadipose agent is mazindol, diethylpropione or phentermine.

Furthermore, the compounds of the present invention may be administered in combination with one or more antihypertensive active compounds. Examples of antihypertensive active compounds are betablockers such as alprenolol, atenol, timolol, pindolol, propanolol and metoprolol, ACE (angiotensin-converting enzyme) inhibitors such as, for example, benazepril, captopril, enalapril, fosinopril, lisinopril, quinapril and rampril, calcium channel blockers such as nifedipine, felodipine, nicardipine, isradipine, nimodipine, diltiazem and verapamil, and also alphablockers such as doxazosin, urapidil, prazosin and terazosin. Furthermore, reference may be made to Remington: The Science and Practice of Pharmacy, 19th edition, Gennaro, editor, Mack Publishing Co., Easton, PA, 1995.

It is self-evident that every suitable combination of the compounds of the invention with one or more of the aforementioned compounds and optionally one or more other pharmacologically active substances is to be regarded as covered by the scope of protection of the present invention.

The following examples illustrate the invention without limitation.

### Example 1

### N-(2,4-dichlorobenzyl)-3-oxo-6-phenyl-4-yl-2,3-dihydropyridazine-4-carboxamide

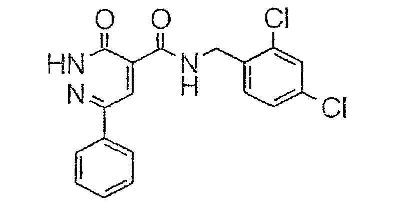

0.14 g of 1-hydroxybenzotriazole, 0.14 cm³ of 2,4-dichlorobenzylamine and 0.14 cm³ of triethylamine are added to 0.2 g of 3-oxo-6-phenyl-2,3-dihydro-pyridazine-4-carboxylic acid prepared as described by Y. Shojiro et al., Chem. Pharm. Bull; 19, (11), p. 2354, in 10 cm³ of dichloromethane. 0.2 g of 1-(3-dimethylaminopropyl)-3-ethylcarbodiimide hydrochloride is then added. The mixture is stirred for 48 hours at 19°C. 10 cm³ of distilled water are added. The organic phase is washed again with 3 times 10 cm³ of aqueous normal hydrochloric acid solution and then with 10 cm³ of saturated aqueous sodium chloride solution. The organic phase is then dried over magnesium sulfate. The mixture is filtered through a sinter funnel and then evaporated under reduced pressure (2 kPa; 45°C). The residue is taken up with 10 cm³ of diisopropyl ether. The insoluble material is filtered off through a sinter funnel and then rinsed again with 10 cm³ of diisopropyl ether. After drying (10 kPa; 20°C), 28 mg of N-(2,4-dichlorobenzyl)-3-oxo-6-phenyl-4-yl-2,3-dihydropyridazine-4-carboxamide are obtained in the form of a cream-colored solid melting at about 258°C.

¹H NMR spectrum (300 MHz, (CD₃)₂SO d6, δ in ppm): 4.64 (d, J = 6 Hz: 2H); from 7.40 to 7.60 (mt: 5H); 7.66 (broad s: 1 H); 7.92 (mt: 2H); 8.55 (s: 1 H); 10.04 (broad t, J = 6 Hz: 1 H); from 13.80 to 14.15 (broad unresolved peak: 1 H).

[M+1]-peak: 374

### Example 2

### N-(2,4-dichlorobenzyl)-3-oxo-6-pyridin-4-yl-2,3-dihydropyridazine-4-carboxamide

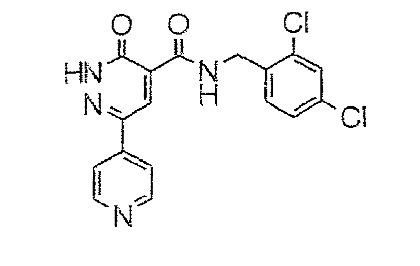

0.02 cm³ of dimethylformamide and then 0.12 cm³ of oxalyl chloride are added to 0.3 g of 3-oxo-6-(pyridin-4-yl)-2,3-dihydropyridazine-4-carboxylic acid prepared as described in patent FR 2 481 284, dissolved in 10 cm³ of dichloromethane. The reaction medium is stirred for 3 hours at 19°C. A further 0.12 cm³ of oxalyl chloride is then added and the mixture is stirred for a further one hour at 19°C. The reaction mixture is then poured onto a solution of 10 cm³ of dichloromethane containing 0.19 cm³ of triethylamine and 0.21 cm³ of 2,4-dichlorobenzylamine. The reaction medium is stirred for 12 hours at 19°C and then filtered through a sinter funnel, rinsed with 10 cm³ of dichloromethane, 10 cm³ of distilled water and with 10 cm³ of aqueous normal hydrochloric acid solution. After drying (10 kPa; 20°C), 0.25 g of N-(2,4-dichlorobenzyl)-3-oxo-6-pyridin-4-yl-2,3-dihydropyridazine-4-carboxamide is obtained in the form of a white solid melting at 233°C.

¹H NMR spectrum (300 MHz, (CD₃)₂SO d6, δ in ppm): 4.64 (d, J = 6 Hz: 2H); 7.45 (mt: 2H); 7.66 (broad s: 1 H); 7.91 (broad d, J = 5 Hz: 2H); 8.62 (s: 1 H); 8.73 (broad d, J = 5 Hz: 2H); 9.95 (broad t, J = 6 Hz: 1 H); 14.25 (unresolved peak: 1H).

[M+1]-peak: 375,03

### Example 3

### N-benzyl-3-oxo-6-pyridin-4-yl-2,3-dihydropyridazine-4-carboxamide

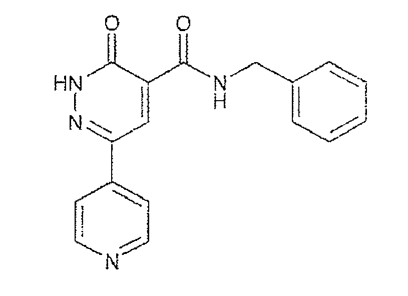

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.17 cm³ of benzylamine and 0.19 cm³ of triethylamine, 0.22 g of N-benzyl-3-oxo-6-pyridin-4-yl-2,3-dihydropyridazine-4-carboxamide is obtained in the form of a cream-colored solid melting at 258°C.

¹H NMR spectrum (300 MHz, (CD₃)₂SO d6, δ in ppm): 4.61 (d, J = 6 Hz: 2H); from 7.25 to 7.45 (mt: 5H); 7.92 (broad d, J = 6 Hz: 2H); 8.64 (s: 1 H); 8.73 (broad d, J = 6 Hz: 2H); 9.93 (broad t, J = 6 Hz: 1 H).

[M+1]-peak: 307

### Example 4

### N-(4-chlorobenzyl)-3-oxo-6-pyridin-4-yl-2,3-dihydropyridazine-4-carboxamide

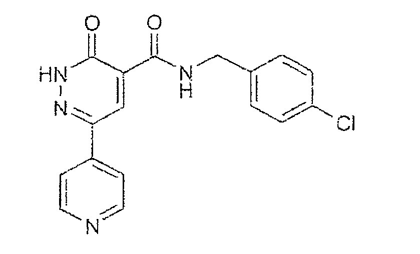

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.19 cm³ of 4-chlorobenzylamine and 0.19 cm³ of triethylamine, 0.2 g of N-(4-chlorobenzyl)-3-oxo-6-pyridin-4-yl-2,3-dihydropyridazine-4-carboxamide is obtained in the form of a cream-colored solid melting at 250°C.

¹H NMR spectrum (300 MHz, (CD₃)₂SO d6, δ in ppm): 4.60 (d, J = 6 Hz: 2H); 7.41 (mt: 4H); 7.92 (broad d, J = 6 Hz: 2H); 8.64 (s: 1 H); 8.73 (broad d, J = 6 Hz: 2H); 9.91 (broad t, J = 6 Hz: 1 H).

[M+1]-peak: 341

### Example 5

### N-(2-chlorobenzyl)-3-oxo-6-pyridin-4-yl-2,3-dihydropyridazine-4-carboxamide

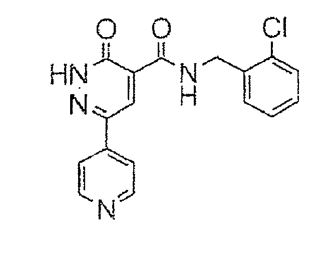

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.19 cm³ of 2-chlorobenzylamine and 0.19 cm³ of triethylamine, 0.25 g of N-(2-chlorobenzyl)-3-oxo-6-pyridin-4-yl-2,3-dihydropyridazine-4-carboxamide is obtained in the form of a white solid melting above 260°C.

¹H NMR spectrum (300 MHz, (CD₃)₂SO d6, δ in ppm): 4.67 (d, J = 6 Hz: 2H); 7.35 (mt: 2H); from 7.40 to 7.55 (mt: 2H); 7.92 (broad d, J = 6 Hz: 2H); 8.63 (s: 1 H); 8.72 (broad d, J = 6 Hz: 2H); 9.95 (broad t, J = 6 Hz: 1 H); 14.25 (s: 1 H).

[M+1]-peak: 341

### Example 6

### N-[2-(2,4-dichlorophenyl)ethyl]-3-oxo-6-pyridin-4-yl-2,3-dihydropyridazine-4-carboxamide

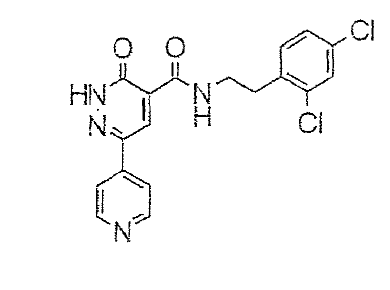

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.23 cm³ of 2,4-dichlorophenylethylamine and 0.19 cm³ of triethylamine, 0.23 g of N-[2-(2,4-dichlorophenyl)ethyl]-3-oxo-6-pyridin-4-yl-2,3-dihydropyridazine-4-carboxamide is obtained in the form of a cream-colored solid melting at 202°C.

¹H NMR spectrum (300 MHz, (CD₃)₂SO d6, δ in ppm): 3.00 (t, J = 7 Hz: 2H); 3.63 (q, J = 7 Hz: 2H); 7.38 (dd, J = 8.5 and 2 Hz: 1 H); 7.44 (d, J = 8 Hz: 1 H); 7.60 (d, J = 2 Hz: 1 H); 7.90 (d mt, J = 6 Hz: 2H); 8.49 (s: 1 H); 8.68 (d mt, J = 6 Hz: 2H); 9.96 (unresolved peak: 1H); from 13.50 to 14.50 (very broad unresolved peak: 1 H).

[M+1]-peak: 389

### Example 7

### N-(2,4-dichlorophenyl)-3-oxo-6-pyridin-4-yl-2,3-dihydropyridazine-4-carboxamide

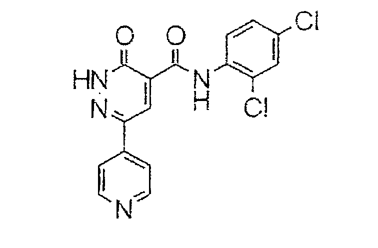

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.036 g of 2,4-dichloroaniline and 0.19 cm³ of triethylamine, 0.16 g of N-(2,4-dichlorophenyl)-3-oxo-6-pyridin-4-yl-2,3-dihydropyridazine-4-carboxamide is obtained in the form of a cream-colored solid melting above 260°C.

¹H NMR spectrum (400 MHz, (CD₃)₂SO d6 with addition of a few drops of CD₃COOD d4, δ in ppm): 7.52 (dd, J = 8.5 and 2.5 Hz: 1 H); 7.75 (d, J = 2.5 Hz: 1 H); 7.96 (d mt, J = 6 Hz: 2H); 8.60 (d, J = 8.5 Hz: 1 H); 8.75 (broad d, J = 6 Hz: 2H); 8.77 (s: 1 H).

[M+1]-peak: 361

### Example 8

### 3-oxo-6-pyridin-4-yl-N-(pyridin-4-ylmethyl)-2,3-dihydropyridazine-4-carboxamide

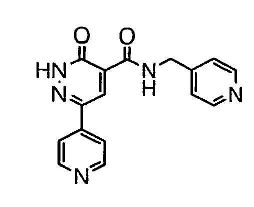

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.15 cm³ of 4-(aminomethyl)pyridine and 0.19 cm³ of triethylamine, 0.14 g of 3-oxo-6-pyridin-4-yl-N-(pyridin-4-ylmethyl)-2,3-dihydropyridazine-4-carboxamide is obtained in the form of a cream-colored solid melting at 254°C.

¹H NMR spectrum (300 MHz, (CD₃)₂SO d6, δ in ppm): 4.63 (d, J = 6 Hz: 2H); 7.35 (broad d, J = 6 Hz: 2H); 7.92 (d mt, J = 6 Hz: 2H); 8.53 (broad d, J = 6 Hz: 2H); 8.62 (s: 1 H); 8.72 (broad d, J = 6 Hz: 2H); 9.99 (t, J = 6 Hz: 1 H); 14.26 (unresolved peak: 1H).

[M+1]-peak: 308

### Example 9

### 3-oxo-6-pyridin-4-yl-N-[3-(trifluoromethyl)benzyl]-2,3-dihydropyridazine-4-carboxamide

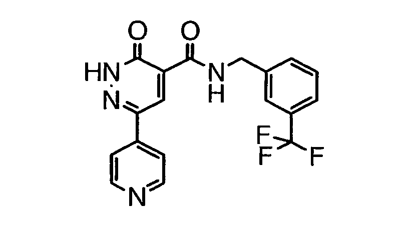

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.21 cm³ of 3-(trifluoromethyl)benzylamine and 0.19 cm³ of triethylamine, 0.22 g of 3-oxo-6-pyridin-4-yl-N-[3-(trifluoromethyl)benzyl]-2,3-dihydropyridazine-4-carboxamide is obtained in the form of a cream-colored solid melting at 224°C.

¹H NMR spectrum (300 MHz, (CD₃)₂SO d6, δ in ppm): 4.68 (d, J = 6 Hz: 2H); from 7.55 to 7.75 (mt: 3H); 7.74 (broad s: 1 H); 7.91 (d mt, J = 6 Hz: 2H); 8.63 (s: 1 H); 8.72 (d mt, J = 6 Hz: 2H); 9.96 (broad t, J = 6 Hz: 1 H); 14.21 (unresolved peak: 1H).

[M+1]-peak: 375

### Example 10

### 3-oxo-6-pyridin-4-yl-N-[4-(trifluoromethyl)benzyl]-2,3-dihydropyridazine-4-carboxamide

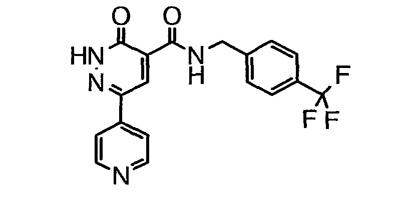

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.21 cm³ of 4-(trifluoromethyl)benzylamine and 0.19 cm³ of triethylamine, 0.22 g of 3-oxo-6-pyridin-4-yl-N-[4-(trifluoromethyl)benzyl]-2,3-dihydropyridazine-4-carboxamide is obtained in the form of a cr2am-colored solid melting at 227°C.

¹H NMR spectrum (300 MHz, (CD₃)₂SO d6, δ in ppm): 4.69 (d, J = 6 Hz: 2H); 7.59 (broad d, J = 8 Hz: 2H); 7.73 (broad d, J = 8 Hz: 2H); 7.91 (d mt, J = 6 Hz: 2H); 8.62 (s: 1 H); 8.72 (d mt, J = 6 Hz: 2H); 10.04 (very broad t, J = 6 Hz: 1 H); 14.24 (unresolved peak: 1 H).

[M+1]-peak: 375

### Example 11

### N-(3,5-dichlorobenzyl)-3-oxo-6-pyridin-4-yl-2,3-dihydropyridazine-4-carboxamide

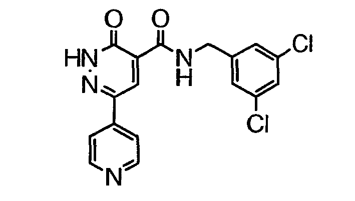

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.19 cm³ of 3,5-dichlorobenzylamine and 0.19 cm³ of triethylamine, 0.025 g of N-(3,5-dichlorobenzyl)-3-oxo-6-pyridin-4-yl-2,3-dihydropyridazine-4-carboxamide is obtained in the form of a white solid melting above 260°C.

¹H NMR spectrum (300 MHz, (CD₃)₂SO d6, δ in ppm): 4.59 (d, J = 6 Hz: 2H); 7.43 (mt: 2H); 7.51 (mt: 1 H); 7.91 (d mt, J = 6 Hz: 2H); 8.57 (s: 1 H); 8.70 (d mt, J = 6 Hz: 2H); 10.14 (unresolved peak: 1 H); 14.18 (broad unresolved peak: 1H).

[M+1]-peak: 375

### Example 12

### 3-oxo-6-pyridin-4-yl-N-(n-butyl)-2,3-dihydropyridazine-4-carboxamide

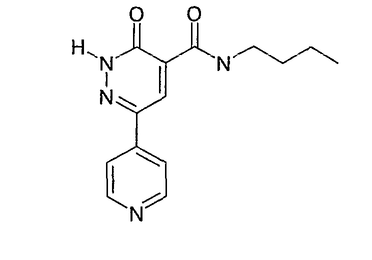

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-4-yl-2,3-dihydropyridazine-4-carboxylic acid, 10 cm³ of dichloromethane, 0.02 cm³ of dimethylformamide, 0.12 cm³ of oxalyl chloride, 0.15 cm³ of n-butylamine and 0.19 cm³ of triethylamine, and after purification by chromatography on silica gel (particle size 40-63 µm, under an argon pressure of 150 kPa), eluting with a mixture of dichloromethane and methanol (97.5/2.5 by volume), 0.23 g of 3-oxo-6-pyridin-4-yl-N-(n-butyl)-2,3-dihydropyridazine-4-carboxamide is obtained in the form of a white solid melting at 209°C.

¹H NMR spectrum (300 MHz, (CD₃)₂SO d6, δ in ppm): 0.93 (t, J = 7 Hz: 3H); 1.38 (mt: 2H); 1.55 (mt: 2H); 3.37 (mt: 2H); 7.90 (d mt, J = 6 Hz: 2H); 8.60 (s: 1 H); 8.72 (broad d, J = 6 Hz: 2H); 9.50 (t, J = 6 Hz: 1 H); 14.20 (unresolved peak: 1 H).

[M+1]-peak: 273

### Example 13

### Ethyl 3-[(3-oxo-6-pyridin-4-yl-2,3-dihydropyridazine-4-carbonyl)amino]propionate

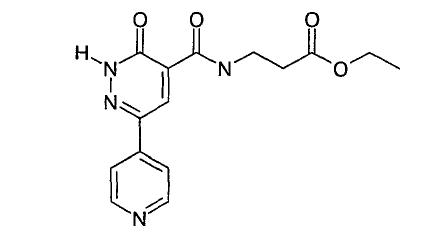

0.733 g of 1-hydroxybenzotriazole, 0.833 g of β-alanine ethyl ester hydrochloride, 0.96 cm³ of N,N-diisopropylethylamine and 2.06 g of O-(7-azabenzotriazol-1-yl)-N,N,N',N'-tetramethyluronium hexafluorophosphate are successively added to 0.94 g of 3-oxo-6-pyridin-4-yl-2,3-dihydropyridazine-4-carboxylic acid dissolved in 100 cm³ of N,N-dimethylformamide. The reaction medium is stirred for 12 hours at 19°C. The solvent is evaporated off under reduced pressure (2 kPa; 55°C). The solid residue is triturated in 20 cm³ of dichloromethane, suction-filtered and oven-dried under reduced pressure (10 kPa; 20°C). After purification by chromatography on silica gel (particle size 40-63 µm, under an argon pressure of 150 kPa) eluting with dichloromethane, 0.45 g of ethyl 3-[(3-oxo-6-pyridin-4-yl-2,3-dihydropyridazine-4-carbonyl)amino]propionate is obtained in the form of a white solid melting at 180°C.

1 H NMR spectrum (300 MHz, (CD3)₂SO d6, δ in ppm): 1.22 (t, J = 7 Hz: 3H); 2.63 (broad t, J = 6.5 Hz: 2H); 3.62 (q, J = 6.5 Hz: 2H); 4.12 (q, J = 7 Hz: 2H); 7.92 (broad d, J = 6 Hz: 2H); 8.61 (s: 1 H); 8.73 (broad d, J = 6 Hz: 2H); 9.69 (broad t, J = 6.5 Hz: 1H).

[M+1]-peak: 317

### Example 14

### 3-oxo-6-pyridin-4-yl-N-(pyridin-3-ylmethyl)-2,3-dihydropyridazine-4-carboxamide

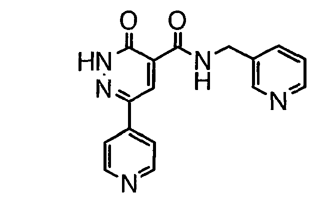

Working as in example 13 for the preparation of ethyl 3-[(3-oxo-6-pyridin-4-yl-2,3-dihydropyridazine-4-carbonyl)amino]propionate, but starting with 0.3 g of 3-oxo-6-pyridin-4-yl-2,3-dihydropyridazine-4-carboxylic acid, 30 cm³ of N,N-dimethylformamide, 0.233 g of 1-hydroxybenzotriazole, 0.18 cm³ of 3-(aminomethyl)pyridine, 0.31 cm³ of N,N-diisopropylethylamine and 0.65 g of O-(7-azabenzotriazol-1-yl)-N,N,N',N'-tetramethyluronium hexafluorophosphate, 0.046 g of 3-oxo-6-pyridin-4-yl-N-(pyridin-3-ylmethyl)-2,3-dihydropyridazine-4-carboxamide is obtained in the form of a yellow solid melting at 262°C.

¹H NMR spectrum (300 MHz, (CD₃)₂SO d6, δ in ppm): 4.62 (d, J = 6 Hz: 2H); 7.38 (broad dd, J = 8 and 5 Hz: 1 H); 7.79 (very broad d, J = 8 Hz: 1 H); 7.91 (broad d, J = 6 Hz: 2H); 8.48 (broad d, J = 5 Hz: 1 H); 8.60 (broad s: 1 H); 8.62 (s: 1 H); 8.72 (broad d, J = 6 Hz: 2H); 9.94 (broad t, J = 6 Hz: 1 H); 14.15 (unresolved peak: 1H).

[M+1]-peak: 308

### Example 15

### 3-oxo-6-pyridin-4-yl-N-(pyridin-2-ylmethyl)-2,3-dihydropyridazine-4-carboxamide

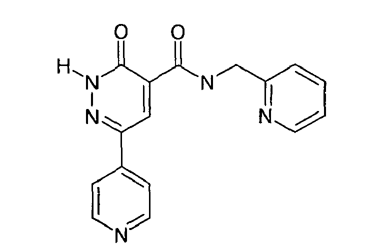

Working as in example 13 for the preparation of ethyl 3-[(3-oxo-6-pyridin-4-yl-2,3-dihydropyridazine-4-carbonyl)amino]propionate, but starting with 0.3 g of 3-oxo-6-pyridin-4-yl-2,3-dihydropyridazine-4-carboxylic acid, 30 cm³ of N,N-dimethylformamide, 0.233 g of 1-hydroxybenzotriazole, 0.18 cm³ of 2-(aminomethyl)pyridine, 0.31 cm³ of N,N-diisopropylethylamine and 0.65 g of O-(7-azabenzotriazol-1-yl)-N,N,N',N'-tetramethyluronium hexafluorophosphate, 0.125 g of 3-oxo-6-pyridin-4-yl-N-(pyridin-2-ylmethyl)-2,3-dihydropyridazine-4-carboxamide is obtained in the form of a white solid melting at 242°C.

¹H NMR spectrum (300 MHz, (CD₃)₂SO d6, δ in ppm): 4.71 (d, J = 6 Hz: 2H); 7.33 (broad dd, J = 8 and 5.5 Hz: 1 H); 7.42 (broad d, J = 8 Hz: 1 H); 7.81 (resolved t, J = 8 and 2 Hz: 1 H); 7.93 (broad d, J = 6 Hz: 2H); 8.57 (broad d, J = 5.5 Hz: 1 H); 8.63 (s: 1 H); 8.73 (broad d, J = 6 Hz: 2H); 10.24 (broad t, J = 6 Hz: 1 H); from 14.00 to 14.50 (very broad unresolved peak: 1H).

[M+1]-peak: 308

### Example 16

### N-(3,4-dichlorobenzyl)-3-oxo-6-pyridin-4-yl-2,3-dihydropyridazine-4-carboxamide

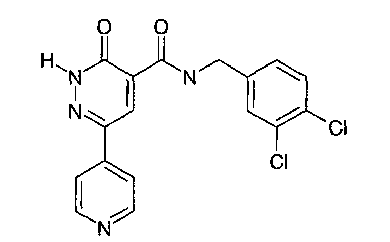

Working as in example 13 for the preparation of ethyl 3-[(3-oxo-6-pyridin-4-yl-2,3-dihydropyridazine-4-carbonyl)amino]propionate, but starting with 0.3 g of 3-oxo-6-pyridin-4-yl-2,3-dihydropyridazine-4-carboxylic acid, 30 cm³ of N,N-dimethylformamide, 0.233 g of 1-hydroxybenzotriazole, 0.19 cm³ of 3,4-dichlorobenzylamine, 0.31 cm³ of N,N-diisopropylethylamine and 0.65 g of O-(7-azabenzotriazol-1-yl)-N,N,N',N'-tetramethyluronium hexafluorophosphate, 0.28 g of N-(3,4-dichlorobenzyl)-3-oxo-6-pyridin-4-yl-2,3-dihydropyridazine-4-carboxamide is obtained in the form of a cream-colored solid melting at 265°C.
¹H NMR spectrum (300 MHz, (CD₃)₂SO d6, δ in ppm): 4.58 (d, J = 6 Hz: 2H); 7.37 (dd, J = 8 and 1.5 Hz: 1 H); 7.62 (d, J = 8 Hz: 1 H); 7.64 (mt: 1 H); 7.91 (broad d, J = 6 Hz: 2H); 8.62 (s: 1 H); 8.72 (broad d, J = 6 Hz: 2H); 9.92 (broad t, J = 6 Hz: 1 H); from 14.00 to 14.40 (very broad unresolved peak: 1 H).

[M+1]-peak: 375

### Example 17

### N-(4-morpholin-4-ylbenzyl)-3-oxo-6-pyridin-4-yl-2,3-dihydropyridazine-4-carboxamide

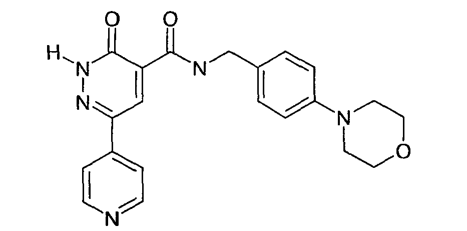

Working as in example 13 for the preparation of ethyl 3-[(3-oxo-6-pyridin-4-yl-2,3-dihydropyridazine-4-carbonyl)amino]propionate, but starting with 0.3 g of 3-oxo-6-pyridin-4-yl-2,3-dihydropyridazine-4-carboxylic acid, 30 cm³ of N,N-dimethylformamide, 0.233 g of 1-hydroxybenzotriazole, 0.265 g of 4-morpholino-benzylamine, 0.31 cm³ of N,N-diisopropylethylamine and 0.65 g of O-(7-azabenzotriazol-1-yl)-N,N,N',N'-tetramethyluronium hexafluorophosphate, and after purification by chromatography on silica gel (particle size 40-63 µm, under an argon pressure of 150 kPa), eluting with dichloromethane, 0.13 g of N-(4-morpholin-4-ylbenzyl)-3-oxo-6-pyridin-4-yl-2,3-dihydropyridazine-4-carboxamide is obtained in the form of a yellow solid melting at 252°C.

¹H NMR spectrum (300 MHz, (CD₃)₂SO d6, δ in ppm): 3.09 (t, J = 5 Hz: 4H); 3.74 (t, J = 5 Hz: 4H); 4.48 (d, J = 6 Hz: 2H); 6.93 (d, J = 8 Hz: 2H); 7.24 (d, J = 8 Hz: 2H); 7.91 (broad d, J = 6 Hz: 2H); 8.62 (s: 1 H); 8.72 (broad d, J = 6 Hz: 2H); 9.85 (very broad t, J = 6 Hz: 1 H); 14.22 (unresolved peak: 1 H).

[M+1]-peak:392,16

### Example 18

### (Trimethylsilyl)-2-ethoxvmethoxv-4-benzonitrile

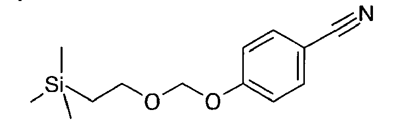

0.085 g of 4-dimethylaminopyridine and 4.9 cm³ of chloromethylethoxy(trimethylsilyl) are added successively to 3 g of 4-hydroxybenzonitrile dissolved in 60 cm³ of dichloromethane, followed by addition of 5.62 cm³ of triethylamine. The reaction medium is stirred for 12 hours at 19°C and then washed with three times 10 cm³ of aqueous normal hydrochloric acid solution, then with 10 cm³ of water, then with 10 cm³ of normal sodium hydroxide and finally with 10 cm³ of saturated sodium chloride solution. The organic phase is dried over magnesium sulfate, filtered through a sinter funnel and then evaporated under reduced pressure (2 kPa; 45°C). After purification by chromatography on silica gel (particle size 40-63 µm, under an argon pressure of 150 kPa), eluting with dichloromethane, 3.5 g of (trimethylsilyl)-2-ethoxymethoxy-4-benzonitrile are obtained in the form of a colorless oil.

Mass spectrum: EI, m/z = 206 (M - SiCH₃)⁺, m/z = 191 (M - Si(CH₃)₂)⁺, m/z = 176 (M - Si(CH₃)₃)⁺ base peak, m/z = 103 (PhCN)⁺, m/z = 73 (Si(CH3)₃)⁺

¹H NMR spectrum (300 MHz, (CD₃)₂SO d6, δ in ppm): - 0.03 (s: 9H); 0.89 (t, J = 8 Hz: 2H); 3.72 (t, J = 8 Hz: 2H); 5.35 (s: 2H); 7.18 (d, J = 9 Hz: 2H); 7.79 (d, J = 9 Hz: 2H).

### (Trimethylsilyl)-2-ethoxymethoxy-4-benzylamine

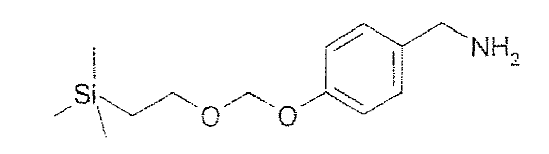

15.5 cm³ of molar lithium aluminum hydride solution are added, at a temperature in the region of 19°C, to 3.5 g of (trimethylsilyl)-2-ethoxymethoxy-4-benzonitrile dissolved in 70 cm³ of tetrahydrofuran. The reaction medium is heated and maintained at the reflux point of the tetrahydrofuran for 4 hours. After cooling to a temperature in the region of 19°C, 0.6 cm³ of water is added to the reaction medium, followed by 0.6 cm³ of aqueous 0.5 N sodium hydroxide solution and 1.8 cm³ of water. The suspension obtained is filtered through a sinter funnel and the residue is washed with 5 times 1.8 cm³ of tetrahydrofuran. The organic phase is dried over magnesium sulfate, filtered through a sinter funnel and then evaporated under reduced pressure (2 kPa; 45°C). 3.3 g of (trimethylsilyl)-2-ethoxymethoxy-4-benzylamine are obtained in the form of a yellow oil.

Mass spectrum: El, m/z = 253 M⁺, m/z = 194 (M - CH₃CH₂0CH₂)⁺ base peak, m/z = 180 (M - Si(CH₃)₃⁺, m/z = 73 (Si(CH₃)₃)⁺

¹H NMR spectrum (400 MHz, (CD₃)₂SO d6, δ in ppm): 0.00 (s: 9H); 0.90 (t, J = 8 Hz: 2H); 3.66 (s: 2H); 3.71 (t, J = 8 Hz: 2H); 5.20 (s: 2H); 6.95 (broad d, J = 8.5 Hz: 2H); 7.24 (broad d, J = 8.5 Hz: 2H).

### N-(4-hydroxybenzyl)-3-oxo-6-pyridin-4-yl-2,3-dihydropyridazine-4-carboxamide

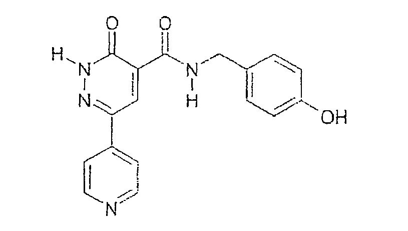

Working as in example 13 for the preparation of ethyl 3-[(3-oxo-6-pyridin-4-yl-2,3-dihydropyridazine-4-carbonyl)amino]propionate, but starting with 0.6 g of 3-oxo-6-pyridin-4-yl-2,3-dihydropyridazine-4-carboxylic acid, 60 cm³ of N,N-dimethylformamide, 0.466 g of 1-hydroxybenzotriazole, 0.91 g of (trimethylsilyl)-2-ethoxymethoxy-4-benzylamine, 0.6 cm³ of N,N-diisopropylethylamine and 1.31 g of O-(7-azabenzotriazol-1-yl)-N,N,N',N'-tetramethyluronium hexafluorophosphate, and after purification by high performance liquid chromatography on a 100 x 30 mm HyPURITY® 5 *µ*m column, eluting with a mixture increasing from 25% to 95% of acetonitrile/water (containing 0.05% trifluoroacetic acid), 0.16 g of N-(4-hydroxybenzyl)-3-oxo-6-pyridin-4-yl-2,3-dihydropyridazine-4-carboxamide is obtained in the form of a cream-colored solid melting at a temperature above 260°C.

¹H NMR spectrum (300 MHz, (CD₃)₂SO d6, δ in ppm): 4.48 (d, J = 6 Hz: 2H); 6.75 (d, J = 8 Hz: 2H); 7.19 (d, J = 8 Hz: 2H); 8.05 (broad d, J = 6 Hz: 2H); 8.69 (s: 1 H); 8.79 (broad d, J = 6 Hz: 2H); from 9.00 to 9.60 (broad unresolved peak: 1 H); 9.74 (t, J = 6 Hz: 1 H); 14.28 (broad s: 1H).

[M+1]-peak: 323.11

### Example 19

### 4-benzyloxyacetophenone

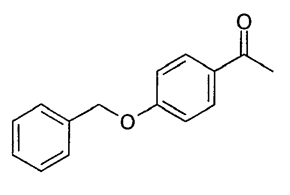

14.5 cm³ of benzyl bromide and 16.75 g of potassium carbonate are added, at a temperature in the region of 19°C, to 15 g of 4-hydroxyacetophenone dissolved in 180 cm³ of acetone. The reaction medium is heated and maintained at the reflux temperature of the acetone for 4 hours. After cooling to a temperature in the region of 19°C, the reaction medium is suction-filtered through a sinter funnel and the insoluble material is rinsed again with 10 cm³ of acetone. The organic phase is evaporated under reduced pressure (2 kPa; 45°C) and the solid obtained is dissolved in 300 cm³ of ethyl acetate. The organic solution is washed with twice 100 cm³ of water and then with 100 cm³ of saturated sodium chloride solution. The organic phase is dried over magnesium sulfate, filtered through a sinter funnel and then evaporated under reduced pressure (2 kPa; 45°C). The solid residue is triturated in 20 cm³ of pentane, suction-filtered through a sinter funnel and oven-dried under reduced pressure (10 kPa; 20°C). 23.1 g of 4-benzyloxyacetophenone are obtained in the form of a white solid melting at 99°C.

¹H NMR spectrum (300 MHz, (CD₃)₂SO d6, δ in ppm): 2.52 (s: 3H); 5.21 (s: 2H); 7.13 (d, J = 9 Hz: 2H); from 7.30 to 7.55 (mt: 5H); 7.95 (d, J = 9 Hz: 2H).

### Diethyl hydroxy[2-(4-benzyloxyphenyl)-2-oxoethyl]malonate

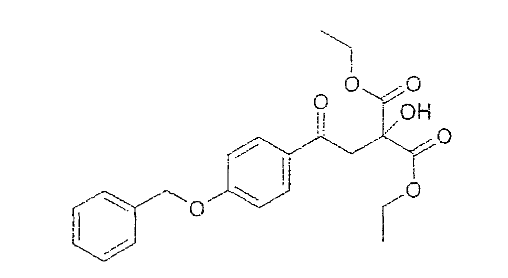

19 cm³ of ethyl ketomalonate and 2.5 cm³ of pyridine are added to 23.1 g of 4-benzyloxyacetophenone. The reaction medium is heated and maintained at reflux for 12 hours. After cooling, the reaction medium is purified by chromatography on silica gel (particle size 40-63 µm, under an argon pressure of 150 kPa), eluting with a mixture of dichloromethane and methanol (99/1 by volume). The fractions containing the product are combined and then concentrated under reduced pressure (45°C; 5 kPa). The product obtained is triturated in 150 cm³ of ethanol, filtered through a sinter funnel, washed with twice 50 cm³ of ethanol and 50 cm³ of isopropyl ether to give, after drying under reduced pressure (2 kPa; 55°C), 5.6 g of diethyl hydroxy[2-(4-benzyloxyphenyl)-2-oxoethyl]malonate melting at 80°C.
¹H NMR spectrum (300 MHz, (CD₃)₂SO d6, δ in ppm): 1.21 (t, J = 7 Hz: 6H); 3.65 (s: 2H); 4.18

(q, J = 7 Hz: 4H); 5.22 (s: 2H); 6.25 (s: 1 H); 7.14 (d, J = 9 Hz: 2H); from 7.30 to 7.55 (mt: 5H); 7.94 (d, J = 9 Hz: 2H).

### Ethyl 6-[4-(benzyloxy)phenyl]-3-oxo-2,3-dihydropyridazine-4-carboxylate

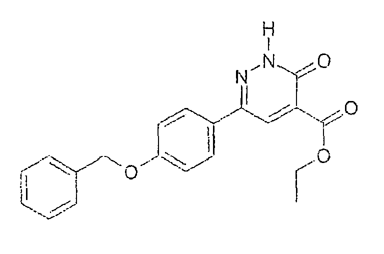

1.69 g of hydrazine dihydrochloride are added, at a temperature in the region of 19°C, to 5.6 g of diethyl hydroxy[2-(4-benzyloxyphenyl)-2-oxoethyl]malonate dissolved in 180 cm³ of ethanol. The reaction medium is heated and maintained at reflux for 12 hours. After cooling, the solvent is removed under reduced pressure (2 kPa; 55°C). The solid residue is triturated in 20 cm³ of ethanol, suction-filtered through a sinter funnel and oven-dried under reduced pressure (10 kPa; 50°C). 3.85 g of ethyl 6-[4-(benzyloxy)phenyl]-3-oxo-2,3-dihydropyridazine-4-carboxylate are obtained in the form of a green solid melting at 239°C.

¹H NMR spectrum (300 MHz, (CD₃)₂SO d6, δ in ppm): 1.32 (t, J = 7 Hz: 3H); 4.32 (q, J = 7 Hz: 2H); 5.19 (s: 2H); 7.14 (d, J = 9 Hz: 2H); from 7.30 to 7.55 (mt: 5H); 7.84 (d, J = 9 Hz: 2H); 8.30 (s: 1 H); 13.51 (broad s: 1H).

### 6-[4-(benzyloxy)phenyl]-3-oxo-2,3,4,5-tetrahydropyridazine-4-carboxylic acid

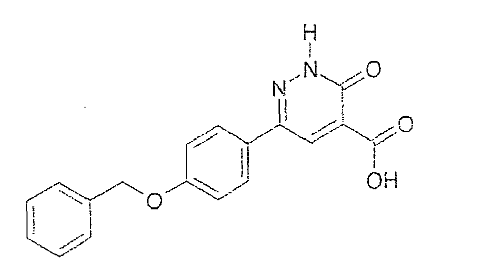

33 cm³ of one molar sodium hydroxide solution are added to 3.85 g of ethyl 6-[4-(benzyloxy)phenyl]-3-oxo-2,3-dihydropyridazine-4-carboxylate. The reaction medium is heated and maintained at reflux for 30 minutes. After cooling, 33 cm³ of one molar hydrochloric acid solution are added.

The suspension obtained is filtered through a sinter funnel and the residue is washed with twice 25 cm³ of water and oven-dried under reduced pressure (10 kPa; 50°C). 3.05 g of 6-[4-(benzyloxy)phenyl]-3-oxo-2,3,4,5-tetrahydropyridazine-4-carboxylic acid are obtained in the form of a yellow solid melting above 260°C.

¹H NMR spectrum (300 MHz, (CD₃)₂SO d6, δ in ppm): 5.20 (s: 2H); 7.15 (d, J = 9 Hz: 2H); from 7.30 to 7.55 (mt: 5H); 7.92 (d, J = 9 Hz: 2H); 8.42 (s: 1H).

### N-(2,4-dichlorobenzyl)-3-oxo-6-[4-(benzyloxy)phenyl]-2,3-dihydropyridazine-4-carboxamide

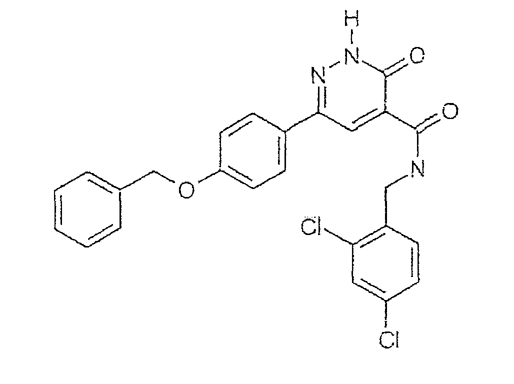

Working as in example 13 for the preparation of ethyl 3-[(3-oxo-6-pyridin-4-yl-2,3-dihydropyridazine-4-carbonyl)amino]propionate, but starting with 1 g of 6-[4-(benzyloxy)phenyl]-3-oxo-2,3,4,5-tetrahydropyridazine-4-carboxylic acid, 100 cm³ of N,N-dimethylformamide, 0.523 g of 1-hydroxybenzotriazole, 0.56 cm³ of 2,4-dichlorobenzylamine, 1.1 cm³ of N,N-diisopropylethylamine and 1.47 g of O-(7-azabenzotriazol-1-yl)-N,N,N',N'-tetramethyluronium hexafluorophosphate, 1.02 g of N-(2,4-dichlorobenzyl)-3-oxo-6-[4-(benzyloxy)phenyl]-2,3-dihydropyridazine-4-carboxamide are obtained in the form of a yellow solid melting at 225°C.

¹H NMR spectrum (300 MHz, (CD₃)₂SO d6, δ in ppm): 4.53 (d, J = 6 Hz: 2H); 5.19 (s: 2H); 7.15 (d, J = 9 Hz: 2H); from 7.30 to 7.55 (mt: 7H); 7.66 (broad s: 1 H); 7.86 (d, J = 9 Hz: 2H); 8.47 (s: 1 H); 10.24 (unresolved peak: 1 H); from 13.75 to 13.95 (broad unresolved peak: 1 H).

### N-benzyl-3-oxo-6-[4-(hydroxy)phenyl]-2,3-dihydropyridazine-4-carboxamide

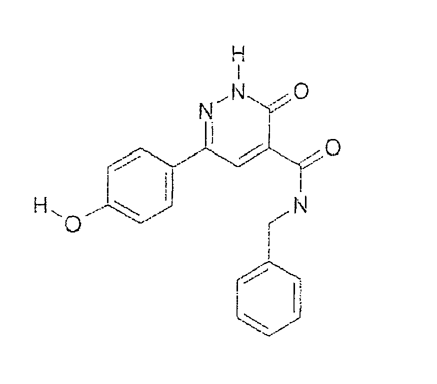

0.525 g of ammonium formate and 0.022 g of palladium hydroxide are added to 0.4 g of N-(2,4-dichlorobenzyl)-3-oxo-6-[4-(benzyloxy)phenyl]-2,3-dihydropyrida-zine-4-carboxamide dissolved in 10 cm³ of methanol. The reaction medium is heated and maintained at reflux for 2 hours. After cooling, the reaction medium is suction-filtered through a sinter funnel and the insoluble material is rinsed again with three times 10 cm³ of hot methanol. The organic phase is evaporated under reduced pressure (2 kPa; 45°C). The residue is recrystallized from methanol. After filtration through a sinter funnel, washing with twice 10 cm³ of methanol and oven-drying under reduced pressure (10 kPa; 50°C), 0.022 g of N-benzyl-3-oxo-6-[4-(hydroxy)phenyl]-2,3-dihydropyridazine-4-carboxamide is obtained in the form of a yellow solid melting at a temperature above 260°C.

¹H NMR spectrum (300 MHz, (CD₃)₂SO d6, δ in ppm): 4.53 (d, J = 6 Hz: 2H); 6.89 (broad d, J = 9 Hz: 2H); from 7.20 to 7.45 (mt: 5H); 7.75 (broad d, J = 9 Hz: 2H); 8.50 (s: 1 H); 10.02 (t, J = 6 Hz: 1H).

[M+1]-peak: 322

### N-(2,4-dichlorobenzyl)-3-oxo-6-[4-(hydroxy)phenyl]-2,3-dihydropyridazine-4-carboxamide and N-(2,4-dichlorobenzyl)-3-oxo-6-[3-benzyl-4-hydroxyphenyl]-2,3-dihydropyridazine-4-carboxamide

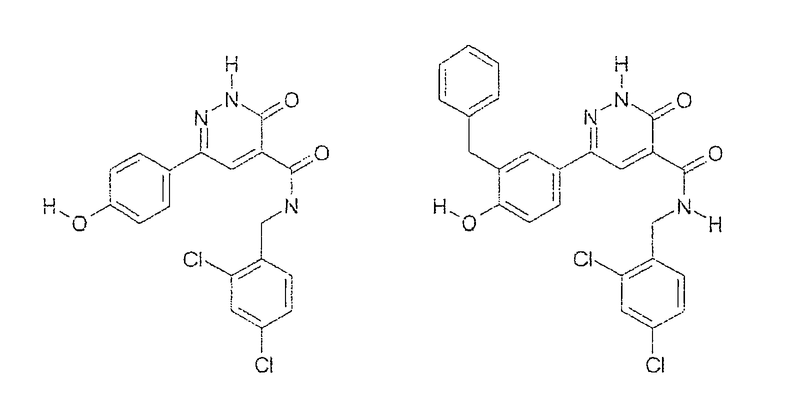

5 cm³ of trifluoroacetic acid are added to 0.4 g of N-(2,4-dichlorobenzyl)-3-oxo-6-[4-(benzyloxy)phenyl]-2,3-dihydropyridazine-4-carboxamide. The reaction medium is heated and maintained at reflux for 2 hours. After cooling, the solvent is removed under reduced pressure (2 kPa; 55°C). The residue is purified by high performance liquid chromatography on a 100 x 30 mm HyPURITY® 5 µm column, eluting with a mixture increasing from 5% to 95% of acetonitrile/water (containing 0.05% trifluoroacetic acid).

0.021 g of N-(2,4-dichlorobenzyl)-3-oxo-6-[4-(hydroxy)phenyl]-2,3-dihydropyridazine-4-carboxamide is obtained in the form of a yellow solid melting at a temperature above 260°C.

¹H NMR spectrum (300 MHz, (CD₃)₂SO d6, δ in ppm): 4.63 (d, J = 6 Hz: 2H); 6.88 (d, J = 8 Hz: 2H); 7.45 (s: 2H); 7.65 (s: 1 H); 7.75 (d, J = 8 Hz: 2H); 8.47 (s: 1 H); 9.90 (unresolved peak: 1 H); 10.11 (broad t, J = 6 Hz: 1 H); 13.78 (broad unresolved peak: 1H).

0.040 g of N-(2,4-dichlorobenzyl)-3-oxo-6-[3-benzyl-4-hydroxyphenyl]-2,3-dihydropyridazine-4-carboxamide is also obtained, in the form of a yellow solid melting at a temperature in the region of 270°C.

¹H NMR spectrum (300 MHz, (CD₃)₂SO d6, δ in ppm): 3.95 (s: 2H); 4.62 (d, J = 6 Hz: 2H); 6.94 (d, J = 8.5 Hz: 1 H); from 7.10 to 7.35 (mt: 5H); 7.44 (mt: 2H); 7.60 (dd, J = 8.5 and 2 Hz: 1 H); 7.65 (mt: 2H); 8.45 (s: 1 H); 9.95 (broad s: 1 H); 10.07 (broad t, J = 6 Hz: 1 H); 13.81 (unresolved peak: 1 H).

### Example 20

### Diethyl hydroxy(pyridin-2-oxoethyl)malonate

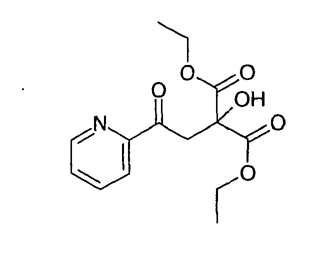

Working as in example 19 for the preparation of diethyl hydroxy[2-(4-benzyloxyphenyl)-2-oxoethyl]malonate, but starting with 13 cm³ of 2-acetylpyridine, 21 cm³ of ethyl ketomalonate and 2.5 cm³ of pyridine, and after purification by chromatography on silica gel (particle size 40-63 µm, under an argon pressure of 150 kPa), eluting with dichloromethane, 31.1 g of diethyl hydroxy(pyridin-2-oxoethyl)malonate are obtained in the form of a brown oil.

¹H NMR spectrum (300 MHz, (CD₃)₂SO d6, δ in ppm): 1.20 (t, J = 7 Hz: 6H); 3.90 (s: 2H); 4.19 (q, J = 7 Hz: 4H); 6.38 (s: 1 H); 7.71 (ddd, J = 7.5 - 5 and 1.5 Hz: 1 H); 7.97 (broad d, J = 7.5 Hz: 1 H); 8.04 (resolved t, J = 7.5 and 1.5 Hz: 1 H); 8.76 (broad d, J = 5 Hz: 1 H).

### Ethyl 6-pyridin-2-yl-3-oxo-2,3-dihydropyridazine-4-carboxylate

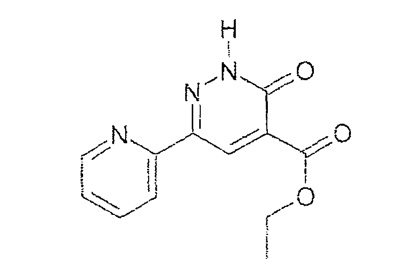

Working as in example 19 for the preparation of ethyl 6-[4-(benzyloxy)phenyl]-3-oxo-2,3-dihydropyridazine-4-carboxylate, but starting with 31.1 g of diethyl hydroxy(pyridin-2-oxoethyl)malonate, 11.55 g of hydrazine dihydrochloride and 700 cm³ of ethanol, and after purification by chromatography on silica gel (particle size 40-63 µm, under an argon pressure of 150 kPa), eluting with dichloromethane, followed by recrystallization from ethanol, 6.9 g of ethyl 6-pyridin-2-yl-3-oxo-2,3-dihydropyridazine-4-carboxylate are obtained in the form of a yellow solid melting at 182°C.

¹H NMR spectrum (300 MHz, (CD₃)₂SO d6, δ in ppm): 1.33 (t, J = 7 Hz: 3H); 4.33 (q, J = 7 Hz: 2H); 7.50 (ddd, J = 8 - 5 and 1.5 Hz: 1 H); 7.97 (resolved t, J = 8 and 2 Hz: 1 H); 8.08 (broad d, J = 8 Hz: 1 H); 8.67 (s: 1 H); 8.70 (ddd, J = 5 - 2 and 1.5 Hz: 1 H); 13.72 (broad s: 1 H).

### 6-pyridin-2-yl-3-oxo-2,3,4,5-tetrahydropyridazine-4-carboxylic acid

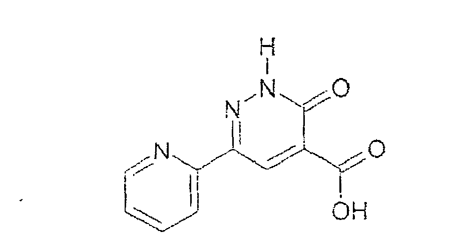

Working as in example 19 for the preparation of 6-[4-(benzyloxy)phenyl]-3-oxo-2,3,4,5-tetrahydropyridazine-4-carboxylic acid, but starting with 4 g of ethyl 6-pyridin-2-yl-3-oxo-2,3-dihydropyridazine-4-carboxylate, 49 cm³ of one molar sodium hydroxide solution and 50 cm³ of one molar hydrochloric acid solution, 3.44 g of 6-pyridin-2-yl-3-oxo-2,3,4,5-tetrahydropyridazine-4-carboxylic acid are obtained in the form of a beige-colored solid melting at a temperature above 260°C.

¹H NMR spectrum (300 MHz, (CD₃)₂SO d6, δ in ppm): 7.53 (broad dd, J = 7.5 and 5 Hz: 1 H); 7.99 (resolved t, J = 7.5 and 1.5 Hz: 1 H); 8.13 (broad d, J = 7.5 Hz: 1 H); 8.72 (broad d, J = 5 Hz: 1H); 8.83 (s: 1 H); from 13.55 to 14.30 (unresolved peak: 2H).

### N-(2,4-dichlorobenzyl)-3-oxo-6-pyridin-2-yl-2,3-dihydropyridazine-4-carboxamide

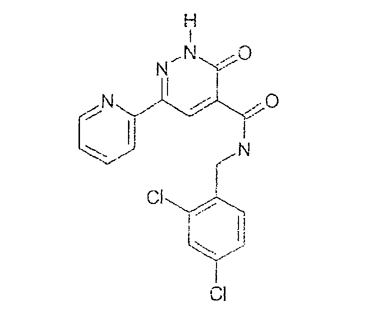

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-2-yl-2,3-dihydropyridazine-4-carboxylic acid, 10 cm³ of dichloromethane, 1 cm³ of dimethylformamide, 0.12 cm³ of oxalyl chloride, 0.21 cm³ of 2,4-dichlorobenzylamine and 0.22 cm³ of triethylamine, 0.22 g of N-(2,4-dichlorobenzyl)-3-oxo-6-pyridin-2-yl-2,3-dihydropyridazine-4-carboxamide is obtained in the form of a cream-colored solid melting at a temperature above 260°C.

¹H NMR spectrum (300 MHz, (CD₃)₂SO d6, δ in ppm): 4.65 (d, J = 6 Hz: 2H); 7.46 (s: 2H); 7.55 (broad dd, J = 8 and 5 Hz: 1 H); 7.67 (broad s: 1 H); 8.31 (ddd, J = 8 - 2.5 and 2 Hz: 1 H); 8.59 (s: 1 H); 8.68 (dd, J = 5 and 2 Hz: 1 H); 9.10 (broad d, J = 2.5 Hz: 1 H); 10.09 (t, J = 6 Hz: 1 H).

[M+1]-peak: 375

### Example 21

### Diethyl hydroxy(pyridin-3-oxoethyl)malonate

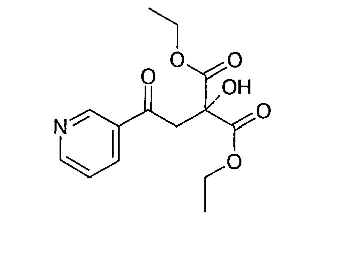

Working as in example 19 for the preparation of diethyl hydroxy[2-(4-benzyloxyphenyl)-2-oxoethyl]malonate, but starting with 8 cm³ of 3-acetylpyridine, 14 cm³ of ethyl ketomalonate and 2 cm³ of pyridine, and after purification by chromatography on silica gel (particle size 40-63 µm, under an argon pressure of 150 kPa), eluting with dichloromethane, 8.85 g of diethyl hydroxy(pyridin-3-oxoethyl)malonate are obtained in the form of a brown oil.

¹H NMR spectrum (300 MHz, (CD₃)₂SO d6, δ in ppm): 1.21 (t, J = 7 Hz: 6H); 3.76 (s: 2H); 4.20 (q, J = 7 Hz: 4H); 6.44 (s: 1 H); 7.59 (broad dd, J = 8 and 5 Hz: 1 H); 8.31 (ddd, J = 8 - 2.5 and 2 Hz: 1 H); 8.83 (dd, J = 5 and 2 Hz: 1 H); 9.12 (broad d, J = 2.5 Hz: 1H).

### Ethyl 6-pyridin-3-yl-3-oxo-2,3-dihydropyridazine-4-carboxylate

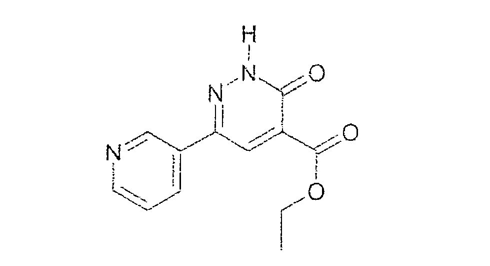

Working as in example 19 for the preparation of ethyl 6-[4-(benzyloxy)phenyl]-3-oxo-2,3-dihydropyridazine-4-carboxylate, but starting with 8.85 g of diethyl hydroxy(pyridin-3-oxoethyl)malonate, 3.67 g of hydrazine dihydrochloride and 250 cm³ of ethanol, and after recrystallization from ethanol, 3.6 g of ethyl 6-pyridin-3-yl-3-oxo-2,3-dihydropyridazine-4-carboxylate are obtained in the form of a green solid melting at a temperature in the region of 150°C.

¹H NMR spectrum (300 MHz, (CD₃)₂SO d6, δ in ppm): 1.33 (t, J = 7 Hz: 3H); 4.34 (q, J = 7 Hz: 2H); 7.54 (broad dd, J = 8 and 5 Hz: 1 H); 8.28 (ddd, J = 8 - 2.5 and 2 Hz: 1 H); 8.41 (s: 1 H); 8.67 (dd, J = 5 and 2 Hz: 1 H); 9.09 (broad d, J = 2.5 Hz: 1 H); 13.75 (unresolved peak: 1H).

### 6-pyridin-3-yl-3-oxo-2,3,4,5-tetrahydropyridazine-4-carboxylic acid

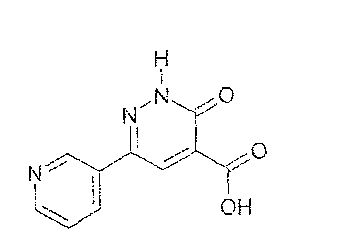

Working as in example 19 for the preparation of 6-[4-(benzyloxy)phenyl]-3-oxo-2,3,4,5-tetrahydropyridazine-4-carboxylic acid, but starting with 2 g of ethyl 6-pyridin-3-yl-3-oxo-2,3-dihydropyridazine-4-carboxylate, 24.5 cm³ of one molar sodium hydroxide solution and 25 cm³ of one molar hydrochloric acid solution, 1 .65 g of 6-pyridin-3-yl-3-oxo-2,3,4,5-tetrahydropyridazine-4-carboxylic acid are obtained in the form of a cream-colored solid melting at a temperature above 260°C.

¹H NMR spectrum (300 MHz, (CD₃)₂SO d6, δ in ppm): 7.55 (broad dd, J = 8 and 5 Hz: 1 H); 8.33 (ddd, J = 8 - 2.5 and 2 Hz: 1 H); 8.56 (s: 1 H); 8.68 (dd, J = 5 and 2 Hz: 1 H); 9.12 (broad d, J = 2.5 Hz: 1 H); from 13.50 to 14.80 (very broad unresolved peak: 2H).

### N-(2,4-dichlorobenzyl)-3-oxo-6-pyridin-3-yl-2,3-dihydropyridazine-4-carboxamide

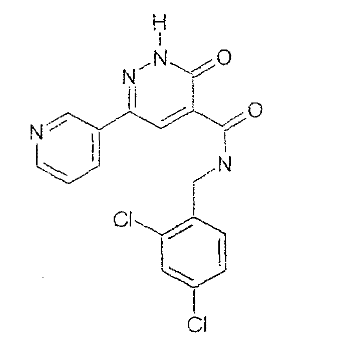

Working as in example 2 for the preparation of N-(2,4-dichlorobenzyl)-3-oxo-6-pyridin-4-yl-2,3-dihydropyridazine-4-carboxamide, but starting with 0.3 g of 3-oxo-6-pyridin-3-yl-2,3-dihydropyridazine-4-carboxylic acid, 10 cm³ of dichloromethane, 1 cm³ of dimethylformamide, 0.12 cm³ of oxalyl chloride, 0.21 cm³ of 2,4-dichlorobenzylamine and 0.22 cm³ of triethylamine, and after purification by chromatography on silica gel (particle size 40-63 µm, under an argon pressure of 150 kPa), eluting with a mixture of dichloromethane and methanol (90/10 by volume), 0.204 g of N-(2,4-dichlorobenzyl)-3-oxo-6-pyridin-3-yl-2,3-dihydropyridazine-4-carboxamide is obtained in the form of a cream-colored solid melting at a temperature in the region of 260°C.

¹H NMR spectrum (300 MHz, (CD₃)₂SO d6, δ in ppm): 4.65 (d, J = 6 Hz: 2H); 7.46 (s: 2H); 7.55 (broad dd, J = 8 and 5 Hz: 1 H); 7.67 (broad s: 1 H); 8.31 (ddd, J = 8 - 2.5 and 2 Hz: 1 H); 8.59 (s: 1 H); 8.68 (dd, J = 5 and 2 Hz: 1 H); 9.10 (broad d, J = 2.5 Hz: 1 H); 10.09 (t, J = 6 Hz: 1H).

[M+1]-peak:375

If not stated otherwise, the examples listed in the following table are synthesized according to the above-mentioned methods.

| Example | structural formula | name | M+1 peak | synthesis according to example |
|---|---|---|---|---|
| 22 | 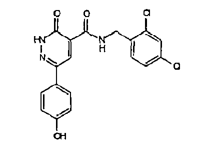 | N-(2,4-Dichlorbenzyl)-3-oxo-6-[4-(hydroxy)phenyl]-2,3-dihydropyridazine-4-carboxylic acid | 390 | 13 |
| 23 | 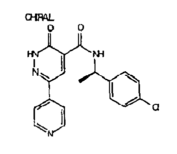 | R-3-Oxo-6-pyridin-4-yl-2,3-dihydro-pyridazine-4-carboxylic acid [1-(4-chloro-phenyl)-ethyl]- amide | 355,09 | 13 |
| 24 | 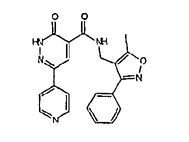 | 3-Oxo-6-pyridin-4-yl-2,3-dihydro-pyridazine-4-carboxylic acid (5-methyl-3-phenyl-isoxazol- 4-ylmethyl)-amide | 388,13 | 13 |
| 25 | 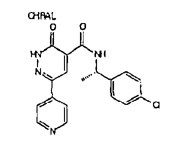 | S-3-Oxo-6-pyridin-4-yl-2,3-dihydro-pyridazine-4-carboxylic acid [1-(4-chloro-phenyl)-ethyl]- amide | 355,09 | 13 |
| 26 | 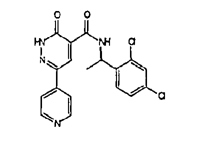 | 3-Oxo-6-pyridin-4-yl-2,3-dihydro-pyridazine-4-carboxylic acid [1-(2,4-dichloro-phenyl)-ethyl]-amide | 389,05 | 13 |
| 27 | 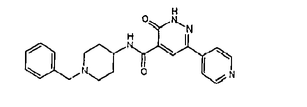 | 3-Oxo-6-pyridin-4-yl-2,3-dihydro-pyridazine-4-carboxylic acid (1-benzyl-piperidin-4-yl)-amide | 390,19 | 13 |
| 28 | 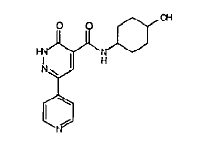 | 3-Oxo-6-pyridin-4-yl-2,3-dihydro-pyridazine-4-carboxylic acid (4-hydroxy-cyclohexyl)-amide | 315,14 | 13 |
| 29 | 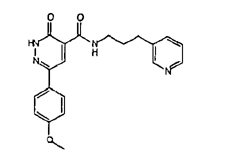 | 6-(4-Methoxy-phenyl)-3-oxo-2,3-dihydro-pyridazine-4-carboxylic acid (3-pyridin-3-yl-propyl)-amide | 365,15 | 13 |
| 30 | 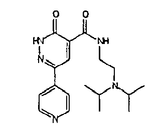 | 3-Oxo-6-pyridin-4-yl-2,3-dihydro-pyridazine-4-carboxylic acid (2-diisopropylamino-ethyl)-amide | 344,2 | 13 |
| 31 | 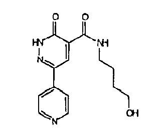 | 3-Oxo-6-pyridin-4-yl-2,3-dihydro-pyridazine-4-carboxylic acid (4-hydroxy-butyl)-amide | 289,12 | 13 |
| 32 | 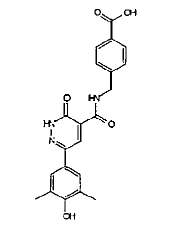 | 4-({[6-(4-Hydroxy-3,5-dimethyl-phenyl)-3-oxo-2,3-dihydro-pyridazine-4-carbonyl]-amino}- methyl)-benzoic acid | 394,13 | 13 |
| 33 | 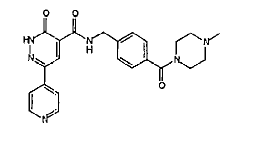 | 3-Oxo-6-pyridin-4-yl-2,3-dihydro-pyridazine-4-carboxylic acid 4-(4-methyl-piperazine-1-carbonyl)-benzylamide; compound with trifluoro-acetic acid | 433,19 | 13 |
| 34 | 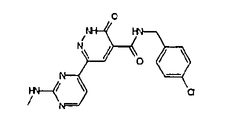 | 6-(2-Methylamino-pyrimidin-4-yl)-3-oxo-2,3-dihydro-pyridazine-4-carboxylic acid 4-chloro-benzylamide | 371,1 | 13 |
| 35 | 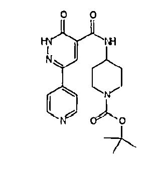 | 4-[(3-Oxo-6-pyridin-4-yl-2,3-dihydro-pyridazine-4-carbonyl)-amino]-piperidine-1-carboxylic acid tert-butyl ester | 400,19 | 13 |
| 36 | 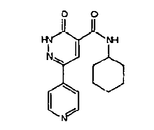 | 3-Oxo-6-pyridin-4-yl-2,3-dihydro-pyridazine-4-carboxylic acid cyclohexylamide | 299,14 | 13 |
| 37 | 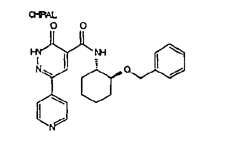 | S,S-3-Oxo-6-pyridin-4-yl-2,3-dihydro-pyridazine-4-carboxylic acid (2-benzyloxy-cyclohexyl)- amide | 405,19 | 13 |
| 38 | 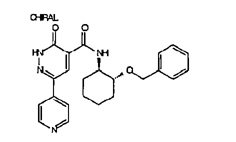 | R,R-3-Oxo-6-pyridin-4-yl-2,3-dihydro-pyridazine-4-carboxylic acid (2-benzyloxy-cyclohexyl)- amide | 405,19 | 13 |
| 39 | 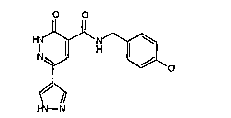 | 3-Oxo-6-(1 H-pyrazol-4-yl)-2,3-dihydro-pyridazine-4-carboxylic acid 4-chloro-benzylamide | 330,07 | 41 |
| 40 | 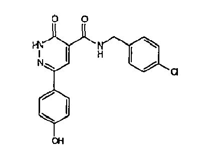 | 6-(4-Hydroxy-phenyl)-3-oxo-2,3-dihydro-pyridazine-4-carboxylic acid 4-chloro-benzylamide | 356,07 | 41 |
| 41 | 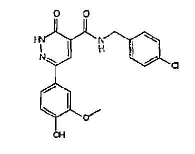 | 6-(4-Hydroxy-3-methoxy-phenyl)-3-oxo-2,3-dihydro-pyridazine-4-carboxylic acid 4-chloro-benzylamide | 386,08 | description of synthesis at the end of this table |
| 42 | 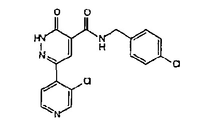 | 6-(3-Chloro-pyridin-4-yl)-3-oxo-2,3-dihydro-pyridazine-4-carboxylic acid 4-chloro-benzylamide | 375,03 | 41 |
| 43 | 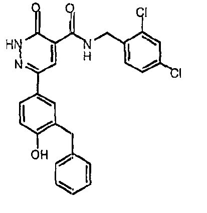 | 6-Chloro-3-oxo-2,3-dihydro-pyridazine-4-carboxylic acid 4-chloro-benzylamide | 298,01 | 13 |
| 44 | 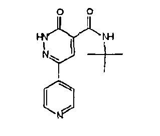 | 3-Oxo-6-pyridin-4-yl-2,3-dihydro-pyridazine-4-carboxylic acid tert-butylamide | 273,13 | 13 |
| 45 | 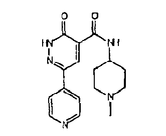 | 3-Oxo-6-pyridin-4-yl-2,3-dihydro-pyridazine-4-carboxylic acid (1-methyl-piperidin-4-yl)-amide | 314,15 | 13 |
| 46 | 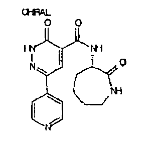 | 3-Oxo-6-pyridin-4-yl-2,3-dihydro-pyridazine-4-carboxylic acid (2-oxo-azepan-3-yl)-amide | 328,13 | 13 |
| 47 | 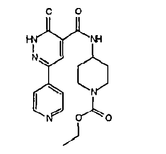 | 4-[(3-Oxo-6-pyridin-4-yl-2,3-dihydro-pyridazine-4-carbonyl)-amino]-piperidine-1-carboxylic acid ethyl ester | 372,16 | 13 |
| 48 | 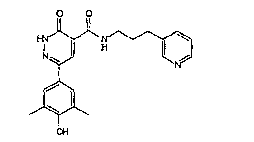 | 6-(4-Hydroxy-3,5-dimethyl-phenyl)-3-oxo-2,3-dihydro-pyridazine-4-carboxylic acid (3-pyridin-3-yl-propyl)-amide | 379,17 | 13 |
| 49 | 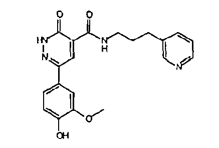 | 6-(4-Hydroxy-3-methoxy-phenyl)-3-oxo-2,3-dihydro-pyridazine-4-carboxylic acid (3-pyridin-3-yl-propyl)-amide | 381,15 | 13 |
| 50 | 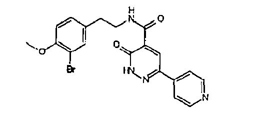 | 3-Oxo-6-pyridin-4-yl-2,3-dihydro-pyridazine-4-carboxylic acid [2-(3-bromo-4-methoxy-phenyl)-ethyl]-amide; compound with trifluoro-acetic acid | 429,05 | 13 |
| 51 | 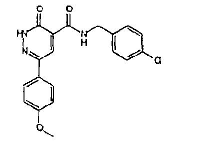 | 6-(4-METHOXY-PHENYL)-3-OXO-2,3-DIHYDRO-PYRIDAZINE-4-CARBOXYLIC ACID 4-CHLORO-BENZYLAMIDE | 370,09 | 41 |
| 52 | 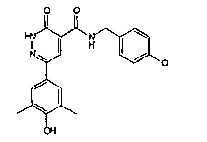 | 6-(4-Hydroxy-3,5-dimethyl-phenyl)-3-oxo-2,3-dihydro-pyridazine-4-carboxylic acid 4-chloro-benzylamide | 384,1 | 41 |
| 53 | 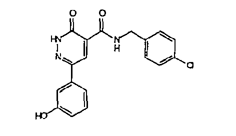 | 6-(3-Hydroxy-phenyl)-3-oxo-2,3-dihydro-pyridazine-4-carboxylic acid 4-chloro-benzylamide | 356,07 | 41 |
| 54 | 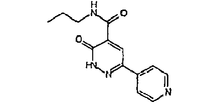 | 3-Oxo-6-pyridin-4-yl-2,3-dihydro-pyridazine-4-carboxylic acid propylamide; compound with trifluoro-acetic acid | 259,11 | 13 |
| 55 | 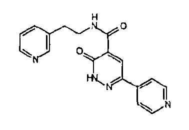 | 3-Oxo-6-pyridin-4-yl-2,3-dihydro-pyridazine-4-carboxylic acid (2-pyridin-3-yl-ethyl)-amide; compound with trifluoro-acetic acid | 322,12 | 13 |
| 56 | 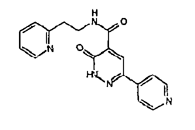 | 3-Oxo-6-pyridin-4-yl-2,3-dihydro-pyridazine-4-carboxylic acid (2-pyridin-2-yl-ethyl)-amide; compound with trifluoro-acetic acid | 322,12 | 13 |
| 57 | 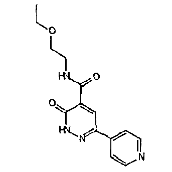 | 3-Oxo-6-pyridin-4-yl-2,3-dihydro-pyridazine-4-carboxylic acid (2-ethoxy-ethyl)-amide; compound with trifluoro-acetic acid | 289,12 | 13 |
| 58 | 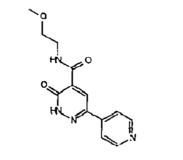 | 3-Oxo-6-pyridin-4-yl-2,3-dihydro-pyridazine-4-carboxylic acid (2-methoxy-ethyl)-amide; compound with trifluoro-acetic acid | 275,11 | 13 |
| 59 | 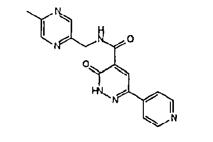 | 3-Oxo-6-pyridin-4-yl-2,3-dihydro-pyridazine-4-carboxylic acid (5-methyl-pyrazin-2-ylmethyl)-amide; compound with trifluoro-acetic acid | 323,12 | 13 |
| 60 | 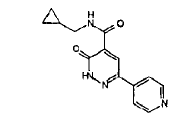 | 3-Oxo-6-pyridin-4-yl-2,3-dihydro-pyridazine-4-carboxylic acid cyclopropylmethyl-amide; compound with trifluoro-acetic acid | 271,11 | 13 |
| 61 | 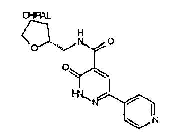 | 3-Oxo-6-pyridin-4-yl-2,3-dihydro-pyridazine-4-carboxylic acid [(R)-1-(tetrahydro-furan-2-yl)methyl]-amide; compound with trifluoro-acetic acid | 301,12 | 13 |
| 62 | 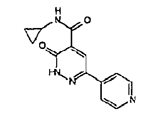 | 3-Oxo-6-pyridin-4-yl-2,3-dihydro-pyridazine-4-carboxylic acid cyclopropylamide; compound with trifluoro-acetic acid | 257,1 | 13 |
| 63 | 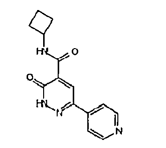 | 3-Oxo-6-pyridin-4-yl-2,3-dihydro-pyridazine-4-carboxylic acid cyclobutylamide; compound with trifluoro-acetic acid | 271,11 | 13 |
| 64 | 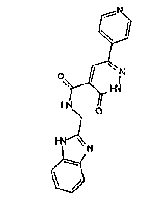 | 3-Oxo-6-pyridin-4-yl-2,3-dihydro-pyridazine-4-carboxylic acid (1 H-benzoimidazol-2-ylmethyl)-amide; compound with trifluoro-acetic acid | 347,12 | 13 |
| 65 | 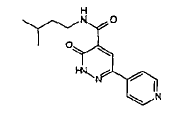 | 3-Oxo-6-pyridin-4-yl-2,3-dihydro-pyridazine-4-carboxylic acid (3-methyl-butyl)-amide; compound with trifluoro-acetic acid | 287,14 | 13 |
| 66 | 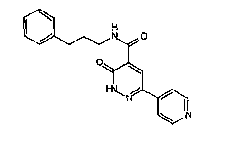 | 3-Oxo-6-pyridin-4-yl-2,3-dihydro-pyridazine-4-carboxylic acid (3-phenyl-propyl)-amide; compound with trifluoro-acetic acid | 335,14 | 13 |
| 67 | 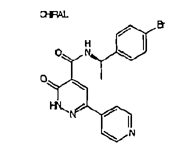 | 3-Oxo-6-pyridin-4-yl-2,3-dihydro-pyridazine-4-carboxylic acid [(R)-1-(4-bromo-phenyl)-ethyl]-amide; compound with trifluoro-acetic acid | 399,04 | 13 |
| 68 | 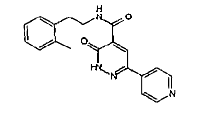 | 3-Oxo-6-pyridin-4-yl-2,3-dihydro-pyridazine-4-carboxylic acid (2-o-tolyl-ethyl)-amide; compound with trifluoro-acetic acid | 335,14 | 13 |
| 69 | 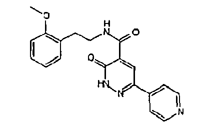 | 3-Oxo-6-pyridin-4-yi-2,3-dihydro-pyridazine-4-carboxylic acid [2-(2-methoxy-phenyl)-ethyl]-amide; compound with trifluoro-acetic acid | 351,14 | 13 |
| 70 | 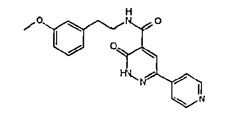 | 3-Oxo-6-pyridin-4-yl-2,3-dihydro-pyridazine-4-carboxylic acid [2-(3-methoxy-phenyl)-ethyl]-amide; compound with trifluoro-acetic acid | 351,14 | 13 |
| 71 | 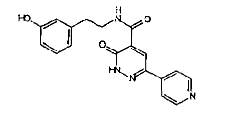 | 3-Oxo-6-pyridin-4-yl-2,3-dihydro-pyridazine-4-carboxylic acid [2-(3-hydroxy-phenyl)-ethyl]-amide; compound with trifluoro-acetic acid | 337,12 | 13 |
| 72 | 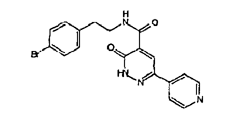 | 3-Oxo-6-pyridin-4-yl-2,3-dihydro-pyridazine-4-carboxylic acid [2-(4-bromo-phenyl)-ethyl]-amide; compound with trifluoro-acetic acid | 399,04 | 13 |
| 73 | 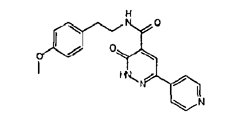 | 3-Oxo-6-pyridin-4-yl-2,3-dihydro-pyridazine-4-carboxylic acid [2-(4-methoxy-phenyl)-ethyl]-amide; compound with trifluoro-acetic acid | 351,14 | 13 |
| 74 | 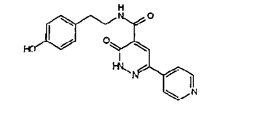 | 3-Oxo-6-pyridin-4-yl-2,3-dihydro-pyridazine-4-carboxylic acid [2-(4-hydroxy-phenyl)-ethyl]-amide; compound with trifluoro-acetic acid | 337,12 | 13 |
| 75 | 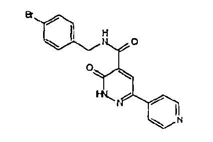 | 3-Oxo-6-pyridin-4-yl-2,3-dihydro-pyridazine-4-carboxylic acid 4-bromo-benzylamide; compound with trifluoro-acetic acid | 385,02 | 13 |
| 76 | 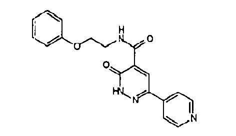 | 3-Oxo-6-pyridin-4-yl-2,3-dihydro-pyridazine-4-carboxylic acid (2-phenoxy-ethyl)-amide; compound with trifluoro-acetic acid | 337,12 | 13 |
| 77 | 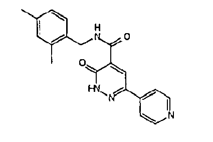 | 3-Oxo-6-pyridin-4-yl-2,3-dihydro-pyridazine-4-carboxylic acid 2,4-dimethyl-benzylamide; compound with trifluoro-acetic acid | 335,14 | 13 |
| 78 | 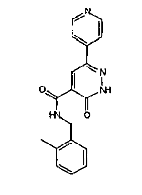 | 3-Oxo-6-pyridin-4-yl-2,3-dihydro-pyridazine-4-carboxylic acid 2-methyl-benzylamide; compound with trifluoro-acetic acid | 321,13 | 13 |
| 79 | 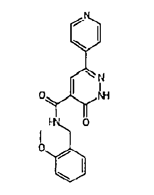 | 3-Oxo-6-pyridin-4-yl-2,3-dihydro-pyridazine-4-carboxylic acid 2-methoxy-benzylamide; compound with trifluoro-acetic acid | 337,12 | 13 |
| 80 | 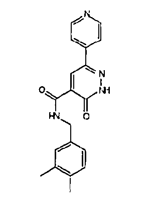 | 3-Oxo-6-pyridin-4-yl-2,3-dihydro-pyridazine-4-carboxylic acid 3,4-dimethyl-benzylamide; compound with trifluoro-acetic acid | 335,14 | 13 |
| 81 | 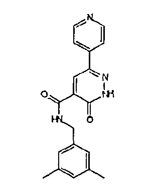 | 3-Oxo-6-pyridin-4-yl-2,3-dihydro-pyridazine-4-carboxylic acid 3,5-dimethyl-benzylamide; compound with trifluoro-acetic acid | 335,14 | 13 |
| 82 | 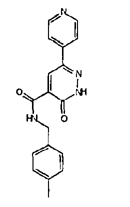 | 3-Oxo-6-pyridin-4-yl-2,3-dihydro-pyridazine-4-carboxylic acid 4-methyl-benzylamide; compound with trifluoro-acetic acid | 321,13 | 13 |
| 83 | 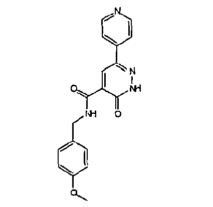 | 3-Oxo-6-pyridin-4-yl-2,3-dihydro-pyridazine-4-carboxylic acid 4-methoxy-benzylamide; compound with trifluoro-acetic acid | 337,12 | 13 |
| 84 | 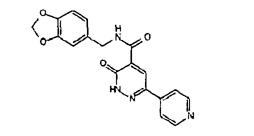 | 3-Oxo-6-pyridin-4-yl-2,3-dihydro-pyridazine-4-carboxylic acid (benzo[1,3]dioxol-5-ylmethyl)-amide; compound with trifluoro-acetic acid | 351,1 | 13 |
| 85 | 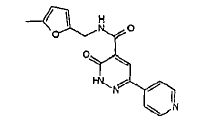 | 3-Oxo-6-pyridin-4-yl-2,3-dihydro-pyridazine-4-carboxylic acid (5-methyl-furan-2-ylmethyl)-amide; compound with trifluoro-acetic acid | 311,11 | 13 |
| 86 | 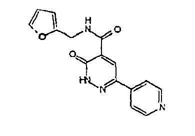 | 3-Oxo-6-pyridin-4-yl-2,3-dihydro-pyridazine-4-carboxylic acid (furan-2-ylmethyl)-amide; compound with trifluoro-acetic acid | 297,09 | 13 |
| 87 | 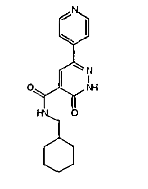 | 3-Oxo-6-pyridin-4-yl-2,3-dihydro-pyridazine-4-carboxylic acid cyclohexylmethyl-amide; compound with trifluoro-acetic acid | 313,16 | 13 |
| 88 | 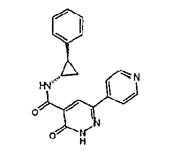 | 3-Oxo-6-pyridin-4-yl-2,3-dihydro-pyridazine-4-carboxylic acid ((1 S,2R)-2-phenyl-cyclopropyl)-amide; compound with trifluoro-acetic acid | 333,13 | 13 |
| 89 | 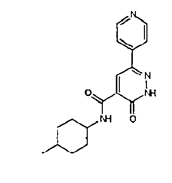 | 3-Oxo-6-pyridin-4-yl-2,3-dihydro-pyridazine-4-carboxylic acid (4-methyl-cyclohexyl)-amide; compound with trifluoro-acetic acid | 313,16 | 13 |
| 90 | 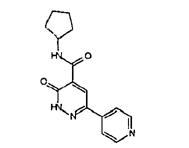 | 3-Oxo-6-pyridin-4-yl-2,3-dihydro-pyridazine-4-carboxylic acid cyclopentylamide; compound with trifluoro-acetic acid | 285,13 | 13 |
| 91 | 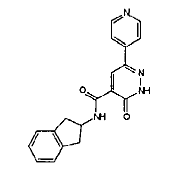 | 3-Oxo-6-pyridin-4-yl-2,3-dihydro-pyridazine-4-carboxylic acid indan-2-ylamide; compound with trifluoro-acetic acid | 333,13 | 13 |
| 92 | 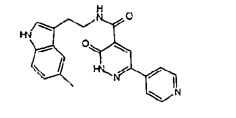 | 3-Oxo-6-pyridin-4-yl-2,3-dihydro-pyridazine-4-carboxylic acid [2-(5-methyl-1 H-indol-3-yl)-ethyl]-amide; compound with trifluoro-acetic acid | 374,15 | 13 |
| 93 | 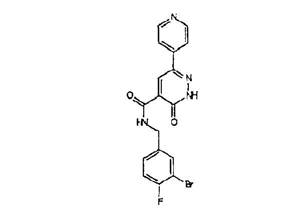 | 3-Oxo-6-pyridin-4-yl-2,3-dihydro-pyridazine-4-carboxylic acid 3-bromo-4-fluoro-benzylamide; compound with trifluoro-acetic acid | 403,01 | 13 |
| 94 | 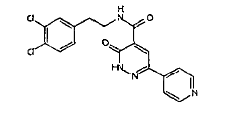 | 3-Oxo-6-pyridin-4-yl-2,3-dihydro-pyridazine-4-carboxylic acid [2-(3,4-dichloro-phenyl)-ethyl]-amide; compound with trifluoro-acetic acid | 389,05 | 13 |
| 95 | 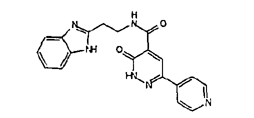 | 3-Oxo-6-pyridin-4-yl-2,3-dihydro-pyridazine-4-carboxylic acid [2-(1 H-benzoimidazol-2-yl)-ethyl]-amide | 361,13 | 13 |
| 96 | 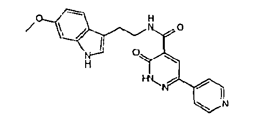 | 3-Oxo-6-pyridin-4-yl-2,3-dihydro-pyridazine-4-carboxylic acid [2-(6-methoxy-1 H-indol-3-yl)-ethyl]-amide; compound with trifluoro-acetic acid | 390,15 | 13 |
| 97 | 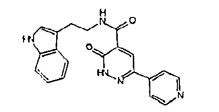 | 3-Oxo-6-pyridin-4-yl-2,3-dihydro-pyridazine-4-carboxylic acid [2-(1 H-indol-3-yl)-ethyl]-amide; compound with trifluoro-acetic acid | 360,14 | 13 |
| 98 | 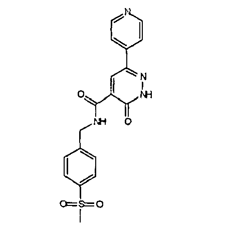 | 3-Oxo-6-pyridin-4-yl-2,3-dihydro-pyridazine-4-carboxylic acid 4-methanesulfonyl-benzylamide; compound with trifluoro-acetic acid | 385,09 | 13 |
| 99 | 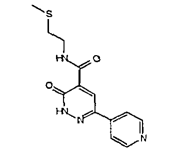 | 3-Oxo-6-pyridin-4-yl-2,3-dihydro-pyridazine-4-carboxylic acid (2-methylsulfanyl-ethyl)-amide; compound with trifluoro-acetic acid | 291,08 | 13 |
| 100 | 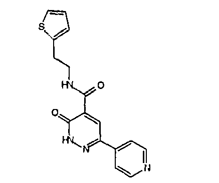 | 3-Oxo-6-pyridin-4-yl-2,3-dihydro-pyridazine-4-carboxylic acid (2-thiophen-2-yl-ethyl)-amide; compound with trifluoro-acetic acid | 327,08 | 13 |
| 101 | 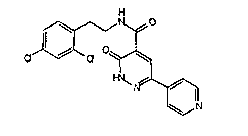 | 3-Oxo-6-pyridin-4-yl-2,3-dihydro-pyridazine-4-carboxylic acid [2-(2,4-dichloro-phenyl)-ethyl]-amide; compound with trifluoro-acetic acid | 389,05 | 13 |
| 102 | 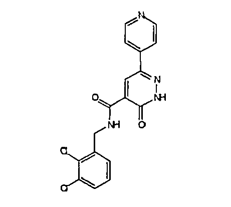 | 3-Oxo-6-pyridin-4-yl-2,3-dihydro-pyridazine-4-carboxylic acid 2,3-dichloro-benzylamide; compound with trifluoro-acetic acid | 375,03 | 13 |
| 103 | 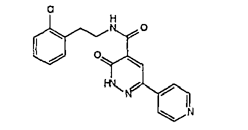 | 3-Oxo-6-pyridin-4-yl-2,3-dihydro-pyridazine-4-carboxylic acid [2-(2-chloro-phenyl)-ethyl]-amide; compound with trifluoro-acetic acid | 355,09 | 13 |
| 104 | 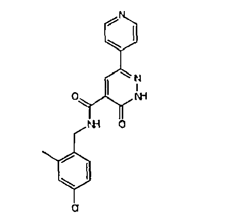 | 3-Oxo-6-pyridin-4-yl-2,3-dihydro-pyridazine-4-carboxylic acid 4-chloro-2-methyl-benzylamide; compound with trifluoro-acetic acid | 355,09 | 13 |
| 105 | 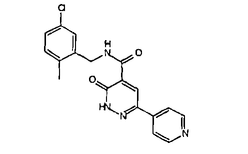 | 3-Oxo-6-pyridin-4-yl-2,3-dihydro-pyridazine-4-carboxylic acid 5-chloro-2-methyl-benzylamide; compound with trifluoro-acetic acid | 355,09 | 13 |
| 106 | 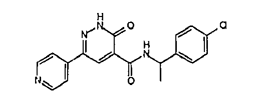 | 3-Oxo-6-pyridin-4-yl-2,3-dihydro-pyridazine-4-carboxylic acid [1-(4-chloro-phenyl)-ethyl]-amide; compound with trifluoro-acetic acid | 355,09 | 13 |
| 107 | 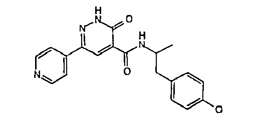 | 3-Oxo-6-pyridin-4-yl-2,3-dihydro-pyridazine-4-carboxylic acid [2-(4-chloro-phenyl)-1-methyl-ethyl]-amide; compound with trifluoro-acetic acid | 369,1 | 13 |
| 108 | 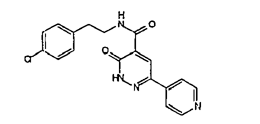 | 3-Oxo-6-pyridin-4-yl-2,3-dihydro-pyridazine-4-carboxylic acid [2-(4-chloro-phenyl)-ethyl]-amide; compound with trifluoro-acetic acid | 355,09 | 13 |
| 109 | 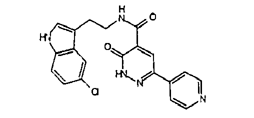 | 3-Oxo-6-pyridin-4-yl-2,3-dihydro-pyridazine-4-carboxylic acid [2-(5-chloro-1 H-indol-3-yl)-ethyl]-amide; compound with trifluoro-acetic acid | 394,1 | 13 |
| 110 | 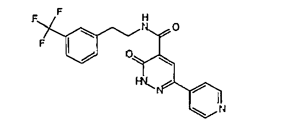 | 3-Oxo-6-pyridin-4-yl-2,3-dihydro-pyridazine-4-carboxylic acid [2-(3-trifluoromethyl-phenyl)-ethyl]-amide; compound with trifluoro-acetic acid | 389,12 | 13 |
| 111 | 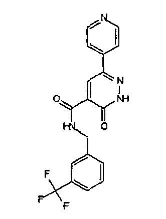 | 3-Oxo-6-pyridin-4-yl-2,3-dihydro-pyridazine-4-carboxylic acid 3-trifluoromethyl-benzylamide; compound with trifluoro-acetic acid | 375,1 | 13 |
| 112 | 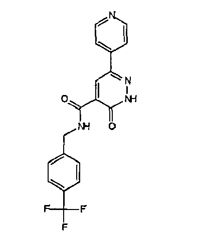 | 3-Oxo-6-pyridin-4-yl-2,3-dihydro-pyridazine-4-carboxylic acid 4-trifluoromethyl-benzylamide; compound with trifluoro-acetic acid | 375,1 | 13 |
| 113 | 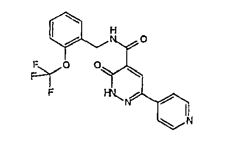 | 3-Oxo-6-pyridin-4-yl-2,3-dihydro-pyridazine-4-carboxylic acid 2-trifluoromethoxy-benzylamide; compound with trifluoro-acetic acid | 391,09 | 13 |
| 114 | 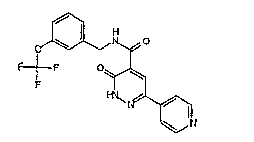 | 3-Oxo-6-pyridin-4-yl-2,3-dihydro-pyridazine-4-carboxylic acid 3-trifluoromethoxy-benzylamide; compound with trifluoro-acetic acid | 391,09 | 13 |
| 115 | 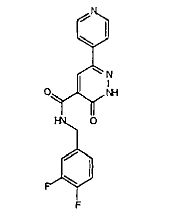 | 3-Oxo-6-pyridin-4-yl-2,3-dihydro-pyridazine-4-carboxylic acid 3,4-difluoro-benzylamide; compound with trifluoro-acetic acid | 343,09 | 13 |
| 116 | 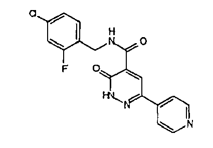 | 3-Oxo-6-pyridin-4-yl-2,3-dihydro-pyridazine-4-carboxylic acid 4-chloro-2-fluoro-benzylamide; compound with trifluoro-acetic acid | 359,06 | 13 |
| 117 | 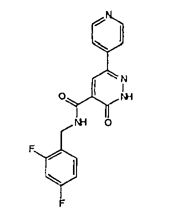 | 3-Oxo-6-pyridin-4-yl-2,3-dihydro-pyridazine-4-carboxylic acid 2,4-difluoro-benzylamide; compound with trifluoro-acetic acid | 343,09 | 13 |
| 118 | 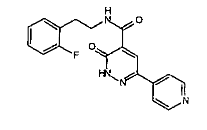 | 3-Oxo-6-pyridin-4-yl-2,3-dihydro-pyridazine-4-carboxylic acid [2-(2-fluoro-phenyl)-ethyl]-amide; compound with trifluoro-acetic acid | 339,12 | 13 |
| 119 | 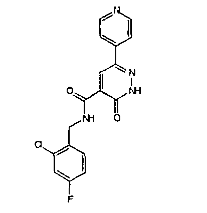 | 3-Oxo-6-pyridin-4-yl-2,3-dihydro-pyridazine-4-carboxylic acid 2-chloro-4-fluoro-benzylamide; compound with trifluoro-acetic acid | 359,06 | 13 |
| 120 | 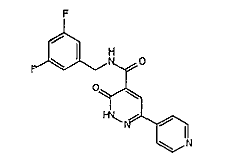 | 3-Oxo-6-pyridin-4-yl-2,3-dihydro-pyridazine-4-carboxylic acid 3,5-difluoro-benzylamide; compound with trifluoro-acetic acid | 343,09 | 13 |
| 121 | 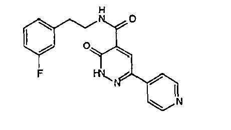 | 3-Oxo-6-pyridin-4-yl-2,3-dihydro-pyridazine-4-carboxylic acid [2-(3-fluoro-phenyl)-ethyl]-amide; compound with trifluoro-acetic acid | 339,12 | 13 |
| 122 | 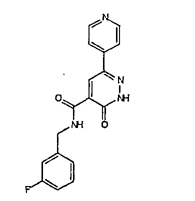 | 3-Oxo-6-pyridin-4-yl-2,3-dihydro-pyridazine-4-carboxylic acid 3-fluoro-benzylamide; compound with trifluoro-acetic acid | 325,1 | 13 |
| 123 | 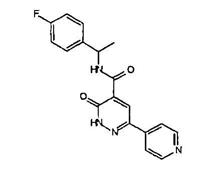 | 3-Oxo-6-pyridin-4-yl-2,3-dihydro-pyridazine-4-carboxylic acid [1-(4-fluoro-phenyl)-ethyl]-amide; compound with trifluoro-acetic acid | 339,12 | 13 |
| 124 | 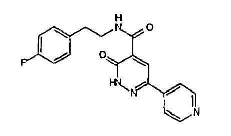 | 3-Oxo-6-pyridin-4-yl-2, 3-dihydro-pyridazine-4-carboxylic acid [2-(4-fluoro-phenyl)-ethyl]-amide; compound with trifluoro-acetic acid | 339,12 | 13 |
| 125 | 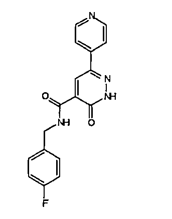 | 3-Oxo-6-pyridin-4-yl-2,3-dihydro-pyridazine-4-carboxylic acid 4-fluoro-benzylamide; compound with trifluoro-acetic acid | 325,1 | 13 |
| 126 | 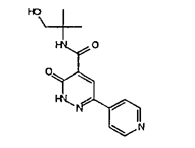 | 3-Oxo-6-pyridin-4-yl-2,3-dihydro-pyridazine-4-carboxylic acid (2-hydroxy-1,1-dimethyl-ethyl)-amide; compound with trifluoro-acetic acid | 289,12 | 13 |
| 127 | 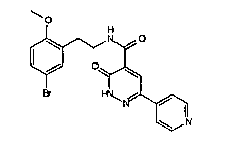 | 3-Oxo-6-pyridin-4-yl-2,3-dihydro-pyridazine-4-carboxylic acid [2-(5-bromo-2-methoxy-phenyl)-ethyl]-amide; compound with trifluoro-acetic acid | 429,05 | 13 |
| 128 | 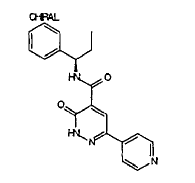 | 3-Oxo-6-pyridin-4-yl-2,3-dihydro-pyridazine-4-carboxylic acid ((R)-1-phenyl-propyl)-amide; compound with trifluoro-acetic acid | 335,14 | 13 |
| 129 | 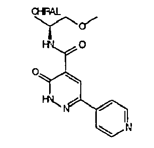 | 3-Oxo-6-pyridin-4-yl-2,3-dihydro-pyridazine-4-carboxylic acid ((S)-2-methoxy-1-methyl-ethyl)-amide; compound with trifluoro-acetic acid | 289,12 | 13 |
| 130 | 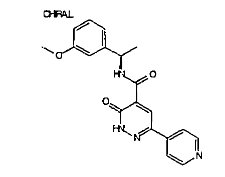 | 3-Oxo-6-pyridin-4-yl-2,3-dihydro-pyridazine-4-carboxylic acid [(R)-1-(3-methoxy-phenyl)-ethyl]-amide; compound with trifluoro-acetic acid | 351,14 | 13 |
| 131 | 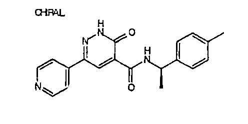 | 3-Oxo-6-pyridin-4-yl-2,3-dihydro-pyridazine-4-carboxylic acid ((R)-1-p-tolyl-ethyl)-amide; compound with trifluoro-acetic acid | 335,14 | 13 |
| 132 | 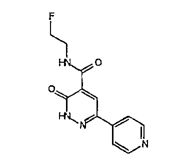 | 3-Oxo-6-pyridin-4-yl-2,3-dihydro-pyridazine-4-carboxylic acid (2-fluoro-ethyl)-amide; compound with trifluoro-acetic acid | 263,09 | 13 |
| 133 | 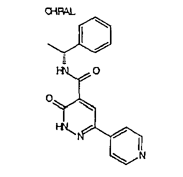 | 3-Oxo-6-pyridin-4-yl-2,3-dihydro-pyridazine-4-carboxylic acid ((R)-1-phenyl-ethyl)-amide; compound with trifluoro-acetic acid | 321,13 | 13 |
| 134 | 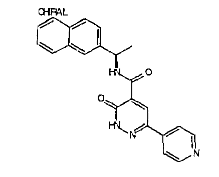 | 3-Oxo-6-pyridin-4-yl-2,3-dihydro-pyridazine-4-carboxylic acid ((R)-1-naphthalen-2-yl-ethyl)-amide; compound with trifluoro-acetic acid | 371,14 | 13 |
| 135 | 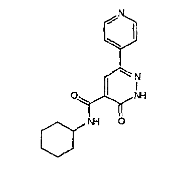 | 3-Oxo-6-pyridin-4-yl-2,3-dihydro-pyridazine-4-carboxylic acid cyclohexylamide; compound with trifluoro-acetic acid | 299,14 | 13 |
| 136 | 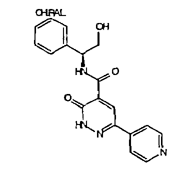 | 3-Oxo-6-pyridin-4-yl-2,3-dihydro-pyridazine-4-carboxylic acid ((S)-2-hydroxy-1-phenyl-ethyl)-amide; compound with trifluoro-acetic acid | 337,12 | 13 |
| 137 | 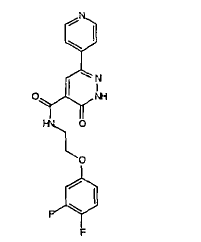 | 3-Oxo-6-pyridin-4-yl-2,3-dihydro-pyridazine-4-carboxylic acid [2-(3,4-difluoro-phenoxy)-ethyl]-amide; compound with trifluoro-acetic acid | 373,1 | 13 |
| 138 | 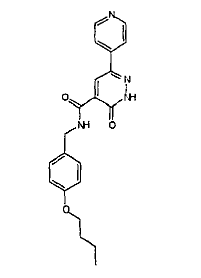 | 3-Oxo-6-pyridin-4-yl-2,3-dihydro-pyridazine-4-carboxylic acid 4-butoxy-benzylamide; compound with trifluoro-acetic acid | 379,17 | 13 |
| 139 | 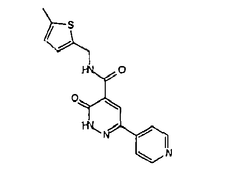 | 3-Oxo-6-pyridin-4-yl-2,3-dihydro-pyridazine-4-carboxylic acid (5-methyl-thiophen-2-ylmethyl)-amide; compound with trifluoro-acetic acid | 327,08 | 13 |
| 140 | 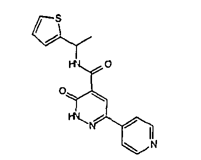 | 3-Oxo-6-pyridin-4-yl-2,3-dihydro-pyridazine-4-carboxylic acid (1-thiophen-2-yl-ethyl)-amide; compound with trifluoro-acetic acid | 327,08 | 13 |
| 141 | 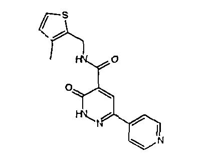 | 3-Oxo-6-pyridin-4-yl-2,3-dihydro-pyridazine-4-carboxylic acid (3-methyl-thiophen-2-ylmethyl)-amide; compound with trifluoro-acetic acid | 327,08 | 13 |
| 142 | 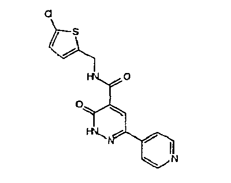 | 3-Oxo-6-pyridin-4-yl-2,3-dihydro-pyridazine-4-carboxylic acid (5-chloro-thiophen-2-ylmethyl)-amide; compound with trifluoro-acetic acid | 347,03 | 13 |
| 143 | 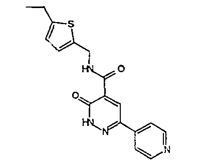 | 3-Oxo-6-pyridin-4-yl-2,3-dihydro-pyridazine-4-carboxylic acid (5-ethyl-thiophen-2-ylmethyl)-amide; compound with trifluoro-acetic acid | 341,1 | 13 |
| 144 | 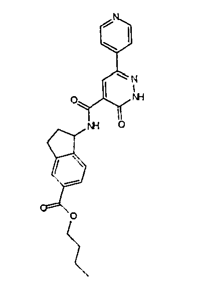 | 1-[(3-Oxo-6-pyridin-4-yl-2,3-dihydro-pyridazine-4-carbonyl)-amino]-indan-5-carboxylic acid butyl ester; compound with trifluoro-acetic acid | 433,18 | 13 |
| 145 | 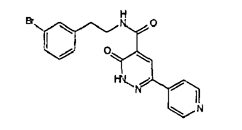 | 3-Oxo-6-pyridin-4-yl-2,3-dihydro-pyridazine-4-carboxylic acid [2-(3-bromo-phenyl)-ethyl]-amide; compound with trifluoro-acetic acid | 399,04 | 13 |
| 146 | 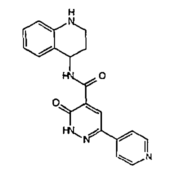 | 3-Oxo-6-pyridin-4-yl-2,3-dihydro-pyridazine-4-carboxylic acid (1,2,3,4-tetrahydro-quinolin-4-yl)-amide; compound with trifluoro-acetic acid | 348,14 | 13 |
| 147 | 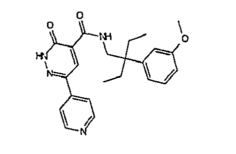 | 3-Oxo-6-pyridin-4-yl-2,3-dihydro-pyridazine-4-carboxylic acid [2-ethyl-2-(3-methoxy-phenyl)-butyl]-amide; compound with trifluoro-acetic acid | 407,2 | 13 |
| 148 | 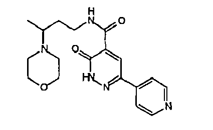 | 3-Oxo-6-pyridin-4-yl-2,3-dihydro-pyridazine-4-carboxylic acid (3-morpholin-4-yl-butyl)-amide; compound with trifluoro-acetic acid | 358,18 | 13 |
| 149 | 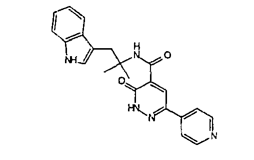 | 3-Oxo-6-pyridin-4-yl-2,3-dihydro-pyridazine-4-carboxylic acid [2-(1 H-indol-3-yl)-1,1-dimethyl-ethyl]-amide; compound with trifluoro-acetic acid | 388,17 | 13 |
| 150 | 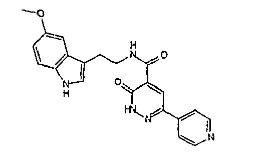 | 3-Oxo-6-pyridin-4-yl-2,3-dihydro-pyridazine-4-carboxylic acid [2-(5-methoxy-1 H-indol-3-yl)-ethyl]-amide; compound with trifluoro-acetic acid | 390,15 | 13 |
| 151 | 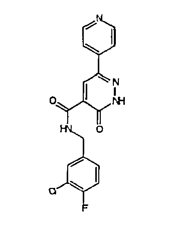 | 3-Oxo-6-pyridin-4-yi-2,3-dihydro-pyridazine-4-carboxylic acid 3-chloro-4-fluoro-benzylamide; compound with trifluoro-acetic acid | 359,06 | 13 |
| 152 | 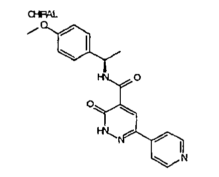 | 3-Oxo-6-pyridin-4-yl-2,3-dihydro-pyridazine-4-carboxylic acid [(R)-1-(4-methoxy-phenyl)-ethyl]-amide; compound with trifluoro-acetic acid | 351,14 | 13 |
| 153 | 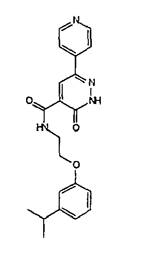 | 3-Oxo-6-pyridin-4-yl-2,3-dihydro-pyridazine-4-carboxylic acid [2-(3-isopropyl-phenoxy)-ethyl]-amide; compound with trifluoro-acetic acid | 379,17 | 13 |
| 154 | 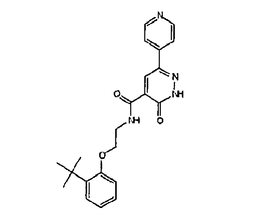 | 3-Oxo-6-pyridin-4-yl-2,3-dihydro-pyridazine-4-carboxylic acid [2-(2-tert-butyl-phenoxy)-ethyl]-amide; compound with trifluoro-acetic acid | 393,19 | 13 |
| 155 | 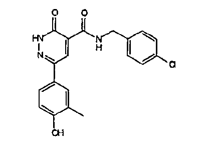 | 6-(4-Hydroxy-3-methyl-phenyl)-3-oxo-2,3-dihydro-pyridazine-4-carboxylic acid 4-chloro-benzylamide | 370,09 | 41 |
| 56 | 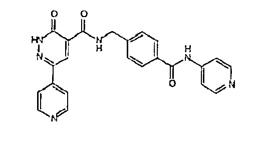 | 3-Oxo-6-pyridin-4-yl-2,3-dihydro-pyridazine-4-carboxylic acid 4-(pyridin-4-ylcarbamoyl)- benzylamide; compound with trifluoro-acetic acid | 427,14 | 13 |
| 157 | 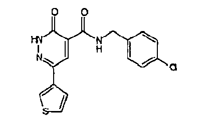 | 3-Oxo-6-thiophen-3-yl-2,3-dihydro-pyridazine-4-carboxylic acid 4-chloro-benzylamide | 346,03 | 41 |
| 158 | 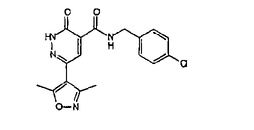 | 6-(3,5-Dimethyl-isoxazol-4-yl)-3-oxo-2,3-dihydro-pyridazine-4-carboxylic acid 4-chloro-benzylamide | 359,08 | 41 |
| 159 | 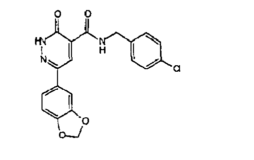 | 6-1,3-Benzodioxol-5-yl-3-oxo-2,3-dihydro-pyridazine-4-carboxylic acid 4-chloro-benzylamide | 384,07 | 41 |
| 160 | 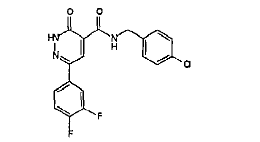 | 6-(3,4-Difluoro-phenyl)-3-oxo-2,3-dihydro-pyridazine-4-carboxylic acid 4-chloro-benzylamide | 376,06 | 41 |
| 161 | 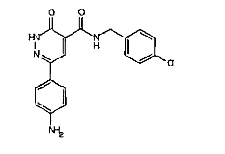 | 6-(4-Amino-phenyl)-3-oxo-2,3-dihydro-pyridazine-4-carboxylic acid 4-chloro-benzylamide | 355,09 | 13 |
| 162 | 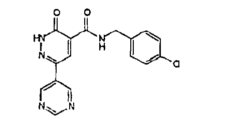 | 3-Oxo-6-pyrimidin-5-yl-2,3-dihydro-pyridazine-4-carboxylic acid 4-chloro-benzylamide | 342,07 | 13 |
| 163 | 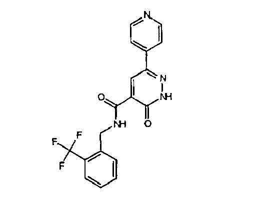 | 3-Oxo-6-pyridin-4-yl-2,3-dihydro-pyridazine-4-carboxylic acid 2-trifluoromethyl-benzylamide; compound with trifluoro-acetic acid | 375,1 | 13 |
| 164 | 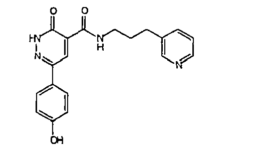 | 6-(4-Hydroxy-phenyl)-3-oxo-2,3-dihydro-pyridazine-4-carboxylic acid (3-pyridin-3-yl-propyl)-amide | 351,14 | 13 |
| 165 | 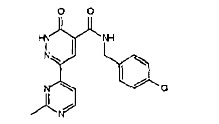 | 6-(2-Methyl-pyrimidin-4-yl)-3-oxo-2,3-dihydro-pyridazine-4-carboxylic acid 4-chloro- benzylamide | 356,08 | 13 |
| 166 | 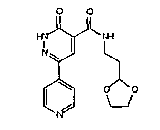 | 3-Oxo-6-pyridin-4-yl-2,3-dihydro-pyridazine-4-carboxylic acid (2-[1,3]dioxolan-2-yl-ethyl)-amide | 317,12 | 13 |
| 167 | 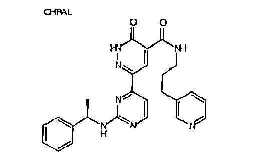 | R-3-Oxo-6-[2-(1-phenyl-ethylamino)-pyrimidin-4-yl]-2,3-dihydro-pyridazine-4-carboxylic acid (3-pyridin-3-yl-propyl)-amide | 456,21 | 13 |
| 168 | 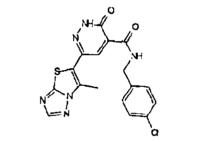 | 6-(6-Methyl-thiazolo[3,2-b][1,2,4]triazol-5-yl)-3-oxo-2,3-dihydro-pyridazine-4- carboxylic acid 4-chloro-benzylamide | 401,05 | 13 |
| 169 | 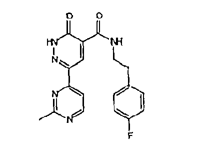 | 6-(2-Methyl-pyrimidin-4-yl)-3-oxo-2,3-dihydro-pyridazine-4-carboxylic acid [2-(4-fluoro- phenyl)-ethyl]-amide | 354,13 | 13 |
| 170 | 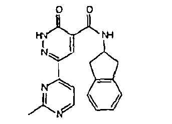 | 6-(2-Methyl-pyrimidin-4-yl)-3-oxo-2,3-dihydro-pyridazine-4-carboxylic acid indan-2- ylamide | 348,14 | 13 |
| 171 | 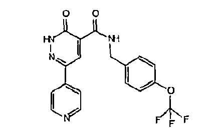 | 3-Oxo-6-pyridin-4-yl-2,3-dihydro-pyridazine-4-carboxylic acid 4-trifluoromethoxy-benzylamide | 391,09 | 13 |
| 172 | 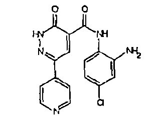 | 3-Oxo-6-pyridin-4-yl-2,3-dihydro-pyridazine-4-carboxylic acid (2-amino-4-chloro-phenyl)-amide | 342,07 | 13 |
| 173 | 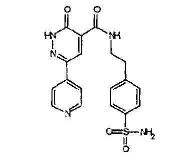 | 3-Oxo-6-pyridin-4-yl-2,3-dihydro-pyridazine-4-carboxylic acid [2-(4-sulfamoyl-phenyl)-ethyl]-amide | 400,1 | 13 |
| 174 | 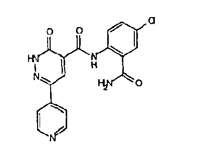 | 3-Oxo-6-pyridin-4-yl-2,3-dihydro-pyridazine-4-carboxylic acid (2-carbamoyl-4-chloro-phenyl)-amide | 370,06 | 13 |
| 175 | 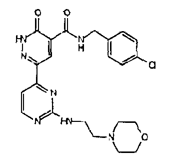 | 6-[2-(2-Morpholin-4-yl-ethylamino)-pyrimidin-4-yl]-3-oxo-2,3-dihydro-pyridazine-4- carboxylic acid 4-chloro-benzylamide; compound with trifluoro-acetic acid | 470,16 | 13 |
| 176 | 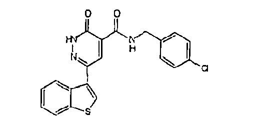 | 6-Benzo[b]thiophen-3-yl-3-oxo-2,3-dihydro-pyridazine-4-carboxylic acid 4-chloro-benzylamide | 396,05 | 41 |
| 177 | 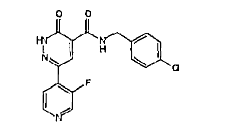 | 6-(3-Fluoro-pyridin-4-yl)-3-oxo-2,3-dihydro-pyridazine-4-carboxylic acid 4-chloro-benzylamide | 359,06 | 41 |
| 178 | 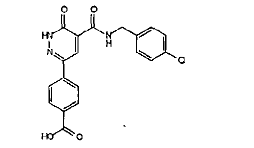 | 4-[5-(4-Chloro-benzylcarbamoyl)-6-oxo-1,6-dihydro-pyridazin-3-yl]-benzoic acid | 384,07 | 41 |
| 179 | 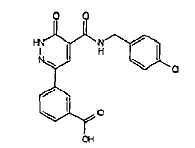 | 3-[5-(4-Chloro-benzylcarbamoyl)-6-oxo-1,6-dihydro-pyridazin-3-yl]-benzoic acid | 384,07 | 41 |
| 180 | 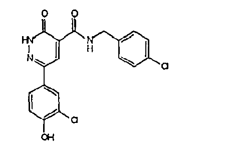 | 6-(3-Chloro-4-hydroxy-phenyl)-3-oxo-2,3-dihydro-pyridazine-4-carboxylic acid 4-chloro-benzylamide | 390,03 | 41 |
| 181 | 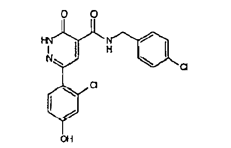 | 6-(2-Chloro-4-hydroxy-phenyl)-3-oxo-2,3-dihydro-pyridazine-4-carboxylic acid 4-chloro-benzylamide | 390,03 | 41 |
| 182 | 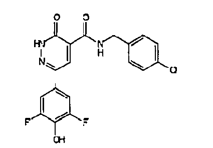 | 6-(3,5-Difluoro-4-hydroxy-phenyl)-3-oxo-2,3-dihydro-pyridazine-4-carboxylic acid 4-chloro-benzylamide | 392,05 | 41 |
| 183 | 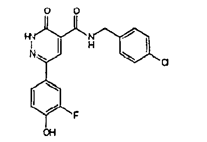 | 6-(3-Fluoro-4-hydroxy-phenyl)-3-oxo-2,3-dihydro-pyridazine-4-carboxylic acid 4-chloro-benzylamide | 374,06 | 41 |
| 184 | 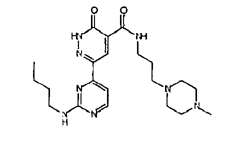 | 6-(2-Butylamino-pyrimidin-4-yl)-3-oxo-2,3-dihydro-pyridazine-4-carboxylic acid [3-(4-methyl-piperazin-1-yl)-propyl]-amide | 429,27 | 13 |
| 185 | 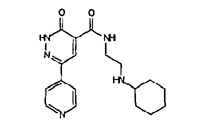 | 3-Oxo-6-pyridin-4-yl-2,3-dihydro-pyridazine-4-carboxylic acid (2-cyclohexylamino-ethyl)-amide | 342,19 | 13 |
| 186 | 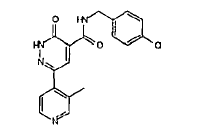 | 6-(3-Methyl-pyridin-4-yl)-3-oxo-2,3-dihydro-pyridazine-4-carboxylic acid 4-chloro-benzylamide; compound with trifluoro-acetic acid | 355,09 | 13 |
| 187 | 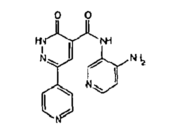 | 3-Oxo-6-pyridin-4-yl-2,3-dihydro-pyridazine-4-carboxylic acid (4-amino-pyridin-3-yl)-amide | 309,1 | 13 |
| 188 | 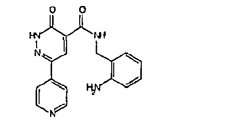 | 3-Oxo-6-pyridin-4-yl-2,3-dihydro-pyridazine-4-carboxylic acid 2-amino-benzylamide | 322,12 | 13 |
| 189 | 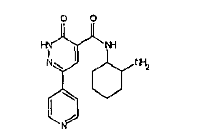 | 3-Oxo-6-pyridin-4-yl-2,3-dihydro-pyridazine-4-carboxylic acid (2-amino-cyclohexyl)-amide | 314,15 | 13 |
| 190 | 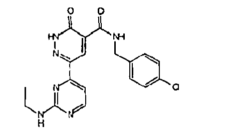 | 6-(2-Ethylamino-pyrimidin-4-yl)-3-oxo-2,3-dihydro-pyridazine-4-carboxylic acid 4-chloro- benzylamide | 385,11 | 13 |
| 191 | 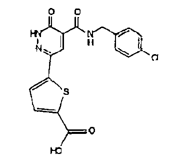 | 5-[5-(4-Chloro-benzylcarbamoyl)-6-oxo-1,6-dihydro-pyridazin-3-yl]-thiophene-2-carboxylic acid | 390,02 | 41 |
| 192 | 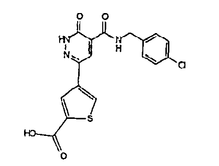 | 4-[5-(4-Chloro-benzylcarbamoyl)-6-oxo-1,6-dihydro-pyridazin-3-yl]-thiophene-2-carboxylic acid | 390,02 | 41 |
| 193 | 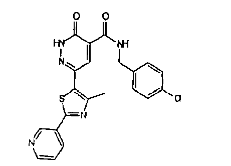 | 6-(4-Methyl-2-pyridin-3-yl-thiazol-5-yl)-3-oxo-2,3-dihydro-pyridazine-4-carboxylic acid 4- chloro-benzylamide | 438,07 | 13 |
| 194 | 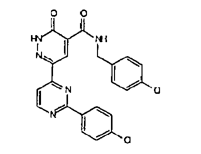 | 6-[2-(4-Chloro-phenyl)-pyrimidin-4-yl]-3-oxo-2,3-dihydro-pyridazine-4-carboxylic acid 4-chloro-benzylamide | 452,06 | 13 |
| 195 | 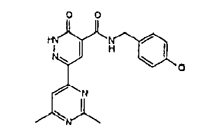 | 6-(2,6-Dimethyl-pyrimidin-4-yl)-3-oxo-2,3-dihydro-pyridazine-4-carboxylic acid 4-chloro- benzylamide-hydrochloride | 370,1 | 13 |
| 196 | 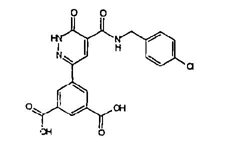 | 5-[5-(4-Chloro-benzylcarbamoyl)-6-oxo-1,6-dihydro-pyridazin-3-yl]-isophthalic acid | 428,06 | 13 |
| 197 | 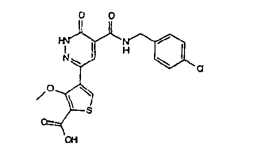 | 4-[5-(4-Chloro-benzylcarbamoyl)-6-oxo-1,6-dihydro-pyridazin-3-yl]-3-methoxy-thiophene-2- carboxylic acid | 420,03 | 41 |
| 198 | 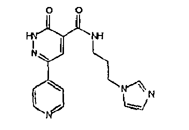 | 3-Oxo-6-pyridin-4-yl-2,3-dihydro-pyridazine-4-carboxylic acid (3-imidazol-1-yl-propyl)-amide | 325,13 | 13 |
| 199 | 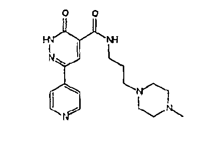 | 3-Oxo-6-pyridin-4-yl-2,3-dihydro-pyridazine-4-carboxylic acid [3-(4-methyl-piperazin-1-yl)-propyl]-amide | 357,2 | 13 |
| 200 | 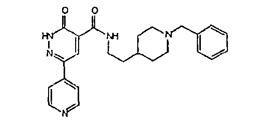 | 3-Oxo-6-pyridin-4-yl-2,3-dihydro-pyridazine-4-carboxylic acid [2-(1-benzyl-piperidin-4-yl)-ethyl]-amide | 418,22 | 13 |
| 201 | 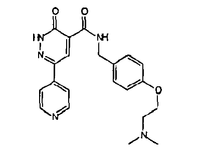 | 3-Oxo-6-pyridin-4-yl-2,3-dihydro-pyridazine-4-carboxylic acid 4-(2-dimethylamino-ethoxy)- benzylamide | 394,18 | 13 |
| 202 | 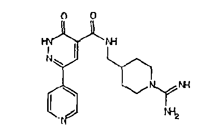 | 3-Oxo-6-pyridin-4-yl-2,3-dihydro-pyridazine-4-carboxylic acid (1-carbamimidoyl-piperidin- 4-ylmethyl)-amide | 356,18 | 13 |
| 203 | 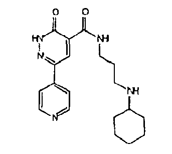 | 3-Oxo-6-pyridin-4-yl-2,3-dihydro-pyridazine-4-carboxylic acid (3-cyclohexylamino-propyl)- amide | 356,2 | 13 |
| 204 | 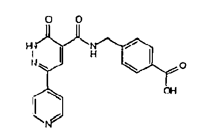 | 4-{[(3-Oxo-6-pyridin-4-yl-2,3-dihydro-pyridazine-4-carbonyl)-amino]-methyl}-benzoic acid | 351,1 | 13 |
| 205 | 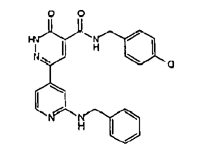 | 6-(2-Benzylamino-pyridin-4-yl)-3-oxo-2,3-dihydro-pyridazine-4-carboxylic acid 4-chloro-benzylamide | 446,13 | 41 |
| 206 | 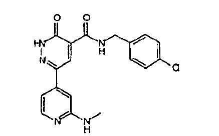 | 6-(2-Methylamino-pyridin-4-yl)-3-oxo-2,3-dihydro-pyridazine-4-carboxylic acid 4-chloro-benzylamide | 370,1 | 41 |
| 207 | 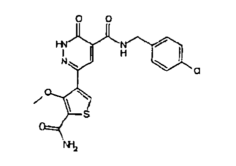 | 6-(5-Carbamoyl-4-methoxy-thiophen-3-yl)-3-oxo-2,3-dihydro-pyridazine-4-carboxylic acid 4- chloro-benzylamide | 419,05 | 41 |
| 208 | 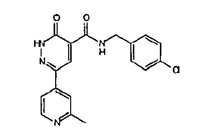 | 6-(2-Methyl-pyridin-4-yl)-3-oxo-2,3-dihydro-pyridazine-4-carboxylic acid 4-chloro-benzylamide; compound with trifluoro-acetic acid | 355,09 | 41 |
| 209 | 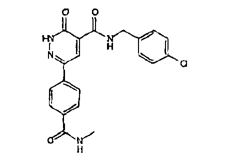 | 6-(4-Methylcarbamoyl-phenyl)-3-oxo-2,3-dihydro-pyridazine-4-carboxylic acid 4-chloro- benzylamide | 397,1 | 41 |
| 210 | 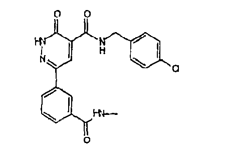 | 6-(3-Methylcarbamoyl-phenyl)-3-oxo-2,3-dihydro-pyridazine-4-carboxylic acid 4-chloro- benzylamide | 397,1 | 41 |
| 211 | 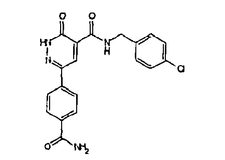 | 6-(4-Carbamoyl-phenyl)-3-oxo-2,3-dihydro-pyridazine-4-carboxylic acid 4-chloro-benzylamide | 383,08 | 41 |
| 212 | 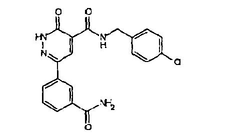 | 6-(3-Carbamoyl-phenyl)-3-oxo-2,3-dihydro-pyridazine-4-carboxylic acid 4-chloro-benzylamide | 383,08 | 41 |
| 213 | 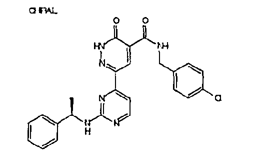 | R-3-Oxo-6-[2-(1-phenyl-ethylamino)-pyrimidin-4-yl]-2,3-dihydro-pyridazine-4-carboxylic acid 4-chloro-benzylamide | 461,14 | 13 |
| 214 | 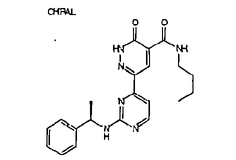 | R-3-Oxo-6-[2-(1-phenyl-ethylamino)-pyrimidin-4-yl]-2,3-dihydro-pyridazine-4-carboxylic acid butylamide | 461,14 | 13 |
| 215 | 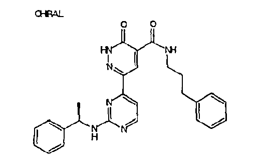 | R-3-Oxo-6-[2-(1-phenyl-ethylamino)-pyrimidin-4-yl]-2,3-dihydro-pyridazine-4-carboxylic acid (3-phenyl-propyl)-amide | 455,21 | 13 |
| 216 | 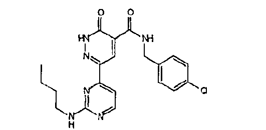 | 6-(2-Butylamino-pyrimidin-4-yl)-3-oxo-2,3-dihydro-pyridazine-4-carboxylic acid 4-chloro- benzylamide | 413,14 | 13 |
| 217 | 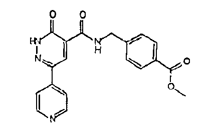 | 4-{[(3-Oxo-6-pyridin-4-yl-2,3-dihydro-pyridazine-4-carbonyl)-amino]-methyl}-benzoic acid methyl ester; compound with trifluoro-acetic acid | 365,12 | 13 |
| 218 | 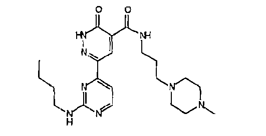 | 6-(2-Butylamino-pyrimidin-4-yl)-3-oxo-2,3-dihydro-pyridazine-4-carboxylic acid [3-(4-methyl-piperazin-1-yl)-propyl]-amide; compound with trifluoro-acetic acid | 429,27 | 13 |
| 219 | 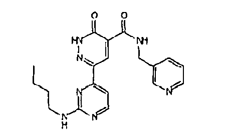 | 6-(2-Butylamino-pyrimidin-4-yl)-3-oxo-2,3-dihydro-pyridazine-4-carboxylic acid (pyridin-3- ylmethyl)-amide | 380,18 | 13 |
| 220 | 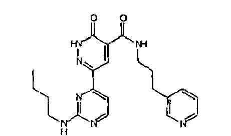 | 6-(2-Butylamino-pyrimidin-4-yl)-3-oxo-2,3-dihydro-pyridazine-4-carboxylic acid (3-pyridin- 3-yl-propyl)-amide | 408,21 | 13 |
| 221 | 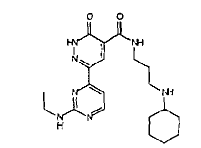 | 6-(2-Ethylamino-pyrimidin-4-yl)-3-oxo-2,3-dihydro-pyridazine-4-carboxylic acid (3-cyclohexylamino-propyl)-amide | 400,24 | 13 |
| 222 | 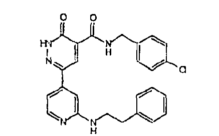 | 3-Oxo-6-(2-phenethylamino-pyridin-4-yl)-2,3-dihydro-pyridazine-4-carboxylic acid 4-chloro-benzylamide | 460,15 | 41 |
| 223 | 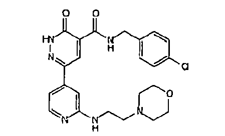 | 6-[2-(2-Morpholin-4-yl-ethylamino)-pyridin-4-yl]-3-oxo-2,3-dihydro-pyridazine-4-carboxylic acid 4-chloro-benzylamide | 469,17 | 41 |
| 224 | 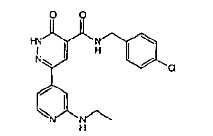 | 6-(2-Ethylamino-pyridin-4-yl)-3-oxo-2,3- dihydro-pyridazine-4-carboxylic acid 4-chloro-benzylamide | 384,12 | 41 |
| 225 | 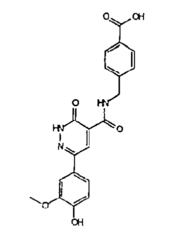 | 4-({[6-(4-Hydroxy-3-methoxy-phenyl)-3-oxo-2,3-dihydro-pyridazine-4-carbonyl]-amino}-methyl)-benzoic acid | 396,11 | 41 |

### Example 41

### 6-(4-Hydroxy-3-methoxy-phenyl)-3-oxo-2,3-dihydro-pyridazine-4-carboxylic acid 4-chloro-benzyl amide

### a) 3,6-Dichloro-pyridazine-4-carboxylic acid

A solution of 24.9 g 3,6-dichloro-4-methylpyridazine and 56,7 g potassium dichromate in 250 ml of concentrated sulphuric acid are stirred at 40°C for 2 h, the reaction mixture is poured onto 1.5 l ice-water and extracted with ethyl acetate. The organic layer is extracted with water and a saturated solution of NaCl, dried over MgSO₄ and evaporated. The raw product is used without any further purification.

Yield: 27.1 g

MS:M+1=193.1

### b) 6-Chloro-3-oxo-2,3-dihydro-pyridazine-4-carboxylic acid

27 g of 3,6-dichloro-pyridazine-4-carboxylic acid are stirred at 50°C for 6 h in a 1:1 mixture of concentrated sulphuric acid and water. The pure product crystallizes after cooling off the reaction mixture and is filtered.

Yield: 12.48 g

MS:M+1=175.1

### c) 6-Chloro-3-oxo-2,3-dihydro-pyridazine-4-carboxylic acid 4-chloro-benzyl amide

Oxalyl chloride is added to a solution a 8.73 g of 6-chloro-3-oxo-2,3-dihydro-pyridazine-4-carboxylic acid and 1 ml DMF in 250 ml THF at 5-10°C and the mixture is stirred at room temperature for 2 h. Afterwards, it is evaporated to dryness, the residue dissolved in 450 ml THF and 13.8 g potassium carbonate and a solution of 7.2 g 4-chloro-benzyl amide in THF are added. The solvent is distilled off after 2 h of stirring at room temperature, the residue suspended in 100 ml water and a pH of 6.4 is adjusted. The obtained precipitate is sucked off, suspended again in 50 ml water and the pH is adjusted to 3. Afterwards, the precipitate is sucked off and dried over phosphorous pentoxide in an exciccator.

Yield: 9.3 g

MS:M+1=298.

### d) 6-Chloro-3-oxo-2-(2-trimethylsilyl-ethoxymethyl)-2,3-dihydropyradizine-4-carboxylic acid 4-chloro-benzyl amide

8.6 g of 6-chloro-3-oxo-2,3-dihydro-pyridazine-4-carboxylic acid 4 chloro-benzyl amide are dissolved in 100 ml of absolute DMF and 4.8 g N,N-diisopropylethyl amine are added, then it is stirred at room temperature for 30 min. Afterwards, 5.9 g of trimethylsilylethoxymethyl chloride is added dropwise and it is stirred at room temperature for 5 h. The mixture is added to 1000 ml water for working up, extracted with ethyl acetate, the organic layer is washed with a saturated solution of NaCl and dried over MgSO₄. After distilling off the solvent the residue is chromatographically purified (silica gel, n-heptane/ethylacetate).

Yield: 7.6 g

MS:M+1=428.18.

### e) 6-(4-Hydroxy-3-methoxy-phenyl)-3-oxo-2-(2-trimethylsilanylethoxymethyl)-2,3-dihydro-pyradizine-4-carboxylic acid 4-chloro-benzyl amide

A solution of 128.5 mg 6-chloro-3-oxo-2-(2-trimethylsilyl-ethoxymethyl)-2,3-dihydropyridazine-4-carboxylic acid 4-chloro-benzyl amide, 91.8 mg 2-methoxy-4-(4,4,5,5-tetramethyl-1,3,2-dioxaborolan-2-yl)-phenol, 82.9 mg K₂CO₃ and 32.1 mg triphenylphosphine in 3.2 ml DME/H₂O (2/1) are degassed by conducting of argon. Afterwards, the mixture is diluted with ethyl acetate and washed with 0.5 N of HCl. After drying over MgSO₄ the solvent is distilled off and the raw product is purified by HPLC (column 125x25, PurospherStar RP18 endcapped, 5 µm; solvent: A: water (0.05 % HCOOH), B: acetonitrile (0.05 % HCOOH)).

Yield: 70.7 mg

MS:M+1=516.31

### f) 6-(4-Hydroxy-3-methoxy-phenyl)-3-oxo-2,3-dihydro-pyridazine-4-carboxylic acid 4-chloro-benzyl amide

70.7 mg of 6-(4-hydroxy-3-methoxy-phenyl)-3-oxo-2-(2-trimethylsilanylethoxymethyl)-2,3-dihydro-pyridazine-4-carboxylic acid 4-chloro-benzyl amide and 60 µl ethandithiol and 60 µl Water are dissolved in 750 µl TFA and stirred at room temperature for 3 h. Afterwards, the solvent is distilled off and the raw product purified by HPLC (column 125x25, PurospherStar RP18 endcapped, 5 µm; solvent: A: water (0.05 % HCOOH), B: acetonitrile (0.05 % HCOOH)).

Yield: 19.7 mg

MS:M+1=386.14

### Functional measurements for determination of IC₅₀-values

### Tau-phosphorylation

Their activities were determined by measuring the inhibition of phosphorylation of the Tau protein in adult rat cortex sections. The cortex sections having a thickness of 300 µm are prepared from 8-10-week old male OFA rats (Iffa-Credo), sacrificed by decapitation. They are incubated in 5 ml of DMEM medium containing pyruvate and glucose 4.5 g/I at 37°C for 40 min. The sections are then washed twice with the medium, distributed into microtubes (50 µl in 500 µl of medium with or without test compounds), and incubated at 37°C, with stirring. Two hours later, the experiment is stopped by centrifugation. The sections are washed, sonicated and centrifuged at 18300 g, for 15 min at 4°C. The concentration of proteins in supernatant is determined by a commercial assay (BCA Protein Assay, Pierce) based on the Lowry method.

The samples, denatured beforehand for 10 min at 70°C, are separated on a 4-12% Bis-Tris vertical gel in the presence of MOPS-SDS buffer and electrotransferred onto nitrocellulose membrane. Immunolabeling is performed with their monoclonal antibody AD2 which specifically recognizes the phosphorylated epitopes Ser396/404 of the Tau protein. The immunoactive proteins are visualized by adding a second antibody directed against the mouse IgGs and coupled to peroxidase and a chemiluminescent substrate. The autoradiograms obtained are finally quantified using the 'GeneTools' software from Syngene (GeneGnome, Ozyme) in order to determine an lC₅₀.

The compounds of formula (I) have a very advantageous activity and in particular some compounds have an IC₅₀ of less than 100 µM.

| Example | IC₅₀[*µ*M] |
|---|---|
| 2 | 22 |
| 4 | 13 |
| 8 | 9.0 |
| 10 | 9.5 |
| 16 | 3.5 |
| 19 | 4.1 |
| 22 | 22 |
| 34 | 0.24 |
| 40 | 0.3 |
| 43 | 2.2 |
| 52 | 19 |
| 53 | 19 |
| 66 | 1.1 |

### GSK-3β

GSK-β activity is measured using human recombinant GSK-3β and a primed (pre-phosphorylated) substrate peptide (derived from glycogen synthase and containing the phosphorylation sites 3a, b, and c) on basis of the AlphaScreen technology in 384-well plate format (small volume plate, white, GREINER). In a final volume of 11 µl, 2 *µ*l of compound (1 nM-100 mM in kinase buffer, DMSO kept constant at 0.9% ), 2 µl of GSK- 3ß solution (0.18 nM) and 2 µl of biot. phospho-glycogen synthase peptide (34 nM) in kinase buffer (20 mM Hepes, pH 7,4, 10 mM MgCl, 200 mM EDTA, 1 mM DTT, 0,1 mg/ml BSA, 10 µM ATP) are incubated at room temperature for 60 min. After adding 2,5 ml Donor beads (20 µg/ml) and 2,5 ml antibody (anti-phospho-glycogen synthase 1:2000) -coated Acceptor beads (40 µg/ml) in AlphaScreen detection buffer (20 mM Hepes, pH 7,4, 10 mM MgCl, 40 mM EDTA, 1 mM DTT, 0,1 mg/ml BSA , plates are incubated at room temperature (in the dark!) over night and then placed in a reader (Alphaquest or Fusion) to measure final fluorescence. IC50-values are calculated form the fitted curvea corrected for blank values (absence of GSK-3ß) and preformed in triplicate.

| Example | IC₅₀[µM] |
|---|---|
| 2 | 0,72 |
| 4 | 0,18 |
| 14 | 0,097 |
| 17 | 0,251 |
| 32 | 0,005 |
| 40 | 2,8 |
| 41 | 2,3 |
| 48 | 0,036 |
| 49 | 0,001 |
| 52 | > 10 |
| 53 | 4,4 |
| 66 | 0,095 |
| 75 | 0,38 |
| 112 | 0,123 |
| 116 | 0,25 |
| 117 | 0,065 |
| 155 | 10,3 |
| 156 | 0,086 |
| 164 | 0,007 |
| 175 | 0,017 |
| 180 | 0,227 |
| 182 | 0,17 |
| 183 | 0,039 |
| 190 | > 1 |
| 206 | 0,03 |
| 215 | 0,024 |
| 218 | 0,022 |
| 219 | 0,029 |
| 220 | 0,036 |
| 225 | 0,009 |

## Claims

1. A compound of formula (I) 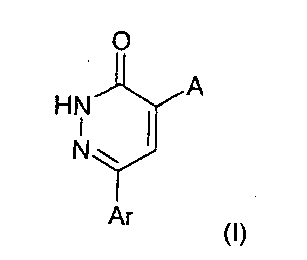 wherein A represents A1 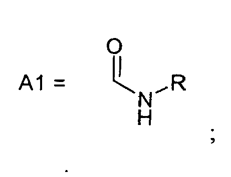
R is unsubstituted or at least monosubstituted C₁-C₁₀-alkyl, aryl, aryl-(C₁-C₁₀-alkyl)-, heteroaryl, heteroaryl-(C₁-C₁₀-alkyl)-, heterocyclyl, heterocyclyl-(C₁-C₁₀-alkyl)-, C₃-C₁₀-cycloalkyl, polycycloalkyl, C₂-C₁₀-alkenyl or C₂-C₁₀-alkinyl,
where the substituents are selected from halogen, -CN, C₁-C₁₀-alkyl, -NO₂, -OR1, -C(O)OR1, -O-C(O)R1, -NR1R2, -NHC(O)R1, -C(O)NR1R2, -SR1, -S(O)R1, -SO₂R1, -NHSO₂R1, -SO₂NR1R2, -C(S)NR1R2, -NHC(S)R1, -O-SO₂R1, -SO₂-O-R1, oxo, -C(O)R1, -C(NH)NH₂, heterocyclyl, C₃-C₁₀-cycloalkyl, aryl-(C₁-C₆-alkyl)-, aryl, heteroaryl, trifluoromethyl, trifluoromethylsulfanyl and trifluoromethoxy,
and aryl, heterocyclyl and heteroaryl may in turn be at least monosubstituted with C₁-C₆-alkyl, C₁-C₆-alkoxy, halogen, trifluoromethyl, trifluoromethoxy or OH;
Ar is unsubstituted or at least monosubstituted aryl or heteroaryl;
where the substituents are selected from halogen, -CN, NO₂ C₁-C₁₀-alkyl, -OR1, -C(O)OR1, -O-C(O)R1, -NR1R2, -NHC(O)R1, -C(O)NR1R2, -NHC(S)R1, -C(S)NR1R2, -SR1, -S(O)R1, -SO₂R1, -NHSO₂R1, -SO₂NR1R2, -O-SO₂R1, -SO₂-O-R1, aryl, heteroaryl, aryl-(C₁-C₆-alkyl)-, formyl, trifluoromethyl and trifluoromethoxy,
and aryl and heteroaryl may in turn be at least monosubstituted with C₁-C₆-alkyl, C₁-C₆-alkoxy, halogen, trifluoromethyl, trifluoromethoxy or OH;
R1 and R2 are independently from each other
hydrogen;
unsubstituted or at least monosubstituted C₁-C₁₀-alkyl, C₃-C₁₀-cycloalkyl, aryl, aryl-(C₁-C₁₀-alkyl)-, C₂-C₁₀-alkenyl, C₂-C₁₀-alkinyl, heterocyclyl, heterocyclyl-(C₁-C₁₀-alkyl)- or heteroaryl, where the substituents are selected from halogen, C₁-C₆-alkyl, C₁-C₆-alkoxy, CN, NO₂ , NH₂, (C₁-C₆-alkyl) amino-, di(C₁-C₆-alkyl)amino-, OH, COOH, -COO-(C₁-C₆-alkyl), -CONH₂, formyl, trifluoromethyl and trifluoromethoxy;
heteroaryl is a 5 to 10-membered, aromatic, mono- or bicyclic heterocycle containing one or more heteroatoms selected from N, O und S;
aryl is phenyl, indanyl, indenyl or naphthyl;
heterocyclyl is a 5 to 10-membered, aliphatic, mono- or bicyclic heterocycle containing one or more heteroatoms selected from N, O and S;
or the racemates, enantiomers, diastereoisomers and mixtures thereof, the tautomers or the physiologically acceptable salts thereof;
with the proviso that A is not -C(O)NH(C₁-C₆-alkyl), in case Ar is phenyl which is at least monosubstituted with heterocyclyl or heteroaryl containing nitrogen and that the following compounds are excluded: 3-{4-(3,4,5-trimethoxyanilino-carbonyl)-3-oxo-2,3-dihydropyridazine-6-yl}-2-phenyl-pyrazolo[1,5-a]pyridine, 3-{4-(N-ethoxycarbonylmethyl)-carbamoyl-3-oxo-2, 3-dihydro-pyridazine-6-yl}-2-phenyl-pyrazolo[1,5-a]pyridine, 3-{4-(N-carboxymethyl)-carbamoyl-3-oxo-2,3-dihydro-pyridazine-6-yl}-2-phenyl-pyrazolo[1,5-a]pyridine, 6-(4-cyanophenyl)-4[(4-carboxybutyl)-aminocarbonyl]-(2H)-pyridazin-3-one and 6-(4-methoxyphenyl)-4-methylcarbamoyl-(2H)-pyridazin-3-one.

2. A compound according to claim 1, wherein the formula (1)
R is unsubstituted or at least monosubstituted C₁-C₁₀-alkyl, aryl, aryl-(C₁-C₁₀-alkyl)-, heterocyclyl, heterocyclyl-(C₁-C₁₀-alkyl)-, C₃-C₁₀-cycloalkyl, heteroaryl or heteroaryl-(C₁-C₁₀-alkyl)-,
where the substituents are selected from halogen, -CN, C₁-C₁₀-Alkyl, -NO₂, -OR1, -C(O)OR1, -O-C(O)R1, -NR1R2, -NHC(O)R1, -C(O)NR1 R2, -SR1, -S(O)R1, -SO₂R1, -NHSO₂R1, -SO₂NR1R2, -C(S)NR1R2, -NHC(S)R1, -O-SO₂R1, -SO₂-O-R1, oxo, -C(O)R1, -C(NH)NH₂, heterocyclyl, C₃-C₁₀-cycloalkyl aryl-(C₁-C₆-alkyl)-, aryl, heteroaryl, trifluoromethyl, trifluoromethylsulfanyl and trifluoromethoxy,
and aryl, heterocyclyl and heteroaryl may in turn be at least monosubstituted with C₁-C₆-alkyl, C₁-C₆-alkoxy, halogen, trifluoromethyl, trifluoroethoxy or OH;
R1 and R2 are independently from each other
hydrogen;
unsubstituted or at least monosubstituted C₁-C₁₀-alkyl, C₃-C₁₀-cycloalkyl, aryl, aryl-(C₁-C₁₀-alkyl)-, C₂-C₁₀-alkenyl, C₂-C₁₀-alkinyl, heterocyclyl, heterocyclyl-(C₁-C₁₀-alkyl)- or heteroaryl, where the substituents are selected from halogen, C₁-C₆-alkyl, C₁-C₆-alkoxy, CN, NO₂, NH₂, (C₁-C₆-alkyl)amino-, di(C₁-C₆-alkyl)amino-, OH, COOH, -COO-(C₁-C₆-alkyl), -CONH₂, formyl, trifluoromethyl and trifluoromethoxy;
heteroaryl is a 5 to 10-membered, aromatic, mono- or bicyclic heterocycle containing one or more heteroatoms selected from N, O and S;
aryl is phenyl, indanyl, indenyl or naphthyl,
heterocyclyl is a 5 to 10-membered, aliphatic, mono- oder bicyclic heterocycle, containing one or more heteroatoms selected from N, O and S;
or the racemates, enantiomers, diastereoisomers and mixtures thereof, the tautomers or the physiologically acceptable salts thereof.

3. A compound according to claim 1 or 2, wherein in formula (I)
Ar is unsubstituted or at least monosubstituted phenyl, pyridinyl, pyrimidinyl, pyrazolyl, thiophenyl, isoxazolyl, benzo[b]thiophenyl, benzodioxolyl or thiazolo[3,2-b][1,2,4]-tiazolyl,
where the substituents are selected from halogen, -CN, NO₂ C₁-C₁₀-alkyl, -OR1, -C(O)OR1, -O-C(O)R1, -NR1R2, -NHC(O)R1, -C(O)NR1 R2, -NHC(S)R1, -C(S)NR1 R2, -SR1, -S(O)R1, -SO₂R1, - NHSO₂R1, -SO₂NR1R2, -O-SO₂R1, -SO₂-O-R1, aryl, heteroaryl, aryl-(C₁-C₆-alkyl)-, formyl, trifluoromethyl and trifluoromethoxy,
and aryl and heteroaryl may in turn be at least monosubstituted with C₁-C₆-alkyl, C₁-C₆-alkoxy, halogen, trifluoromethyl, trifluoromethoxy or OH;
R1 and R2 are independently from each other
hydrogen;
unsubstituted or at least monosubstituted C₁-C₁₀-alkyl, C₃-C₁₀-cycloalkyl, aryl, aryl-(C₁-C₁₀-alkyl)-, C₂-C₁₀-alkenyl, C₂-C₁₀-alkinyl, heterocyclyl, heterocyclyl-(C₁-C₁₀-alkyl)- or heteroaryl, where the substituents are selected from halogen, C₁-C₆-alkyl, C₁-C₆-alkoxy, CN, NO₂ , NH₂, (C₁-C₆-alkyl) amino-, di(C₁-C₆-alkyl)amino-, OH, COOH, -COO-(C₁-C₆-alkyl), -CONH₂, formyl, trifluoromethyl and trifluoromethoxy;
heteroaryl is a 5 to 10-membered aromatic, mono- or bicyclic heterocycle, containing one or more heteroatoms selected from N, O and S;
aryl is phenyl, indanyl, indenyl or naphthyl;
heterocyclyl is a 5 to 10-membered aliphatic, mono- oder bicyclic heterocycle, containing one or more heteroatoms selected from N, O and S;
or the racemates, enantiomers, diastereoisomers and mixtures thereof, the tautomers or the physiologically acceptable salts thereof.

4. A compound according to any of claims 1 to 3, wherein in formula (I)
R is unsubstituted or at least monosubstituted aryl-(C₁-C₆-alkyl)- or heteroaryl-(C₁-C₆-alkyl)-,
where the substituents are selected from halogen, C₁-C₆-alkyl, -OH, -O-aryl, C₁-C₆-alkoxy, -O-(C₁-C₆-alkylen)-N(C₁-C₆-alkyl)₂, -C(O)OH, -C(O)O-(C₁-C₆-alkyl), -NH₂, -N(C₁-C₆-alkyl)₂, -NH(C₁-C₆-alkyl), -NH(C₁-C₁₀-cycloalkyl), -C(O)NH₂, -C(O)NH-heteroaryl, -C(O)NH-(C₁-C₆-alkyl), -SO₂(C₁-C₆-alkyl), -SO₂NH₂, -C(O)-heterocyclyl, -C(NH)NH₂, heterocyclyl, aryl-(C₁-C₆-alkyl)-, aryl, trifluoromethyl, and trifluoromethoxy,
and aryl, aeterocyclyl and heteroaryl may in turn be at least monosubstituted with C₁-C₃-alkyl, C₁-C₃-alkoxy, fluorine, chlorine, bromine, trifluoromethyl, trifluoromethoxy or OH;
heteroaryl is imidazolyl, thiophenyl, furanyl, isoxazolyl, pyridinyl, pyrimidinyl, benzoimidazolyl, indolyl or benzodioxolyl;
aryl is phenyl or naphthyl;
hetrocyclyl is morpholinyl, piperazinyl or piperidinyl;
or the racemates, enantiomers, diastereoisomers and mixtures thereof, the tautomers or the physiologically acceptable salts thereof.

5. A compound according to any of claims 1 to 4, wherein in formula (I)
Ar is unsubstituted or at least monosubstituted phenyl, pyridin-4-yl or pyrimidin-4-yl,
where the substituents are selected from halogen, C₁-C₆-alkyl, -OH, C₁-C₆-alkoxy, -C(O)OH, -C(O)O-(C₁-C₆-alkyl), -NH₂, -N(C₁-C₆-alkyl)₂, -NH(C₁-C₆-alkyl), -NH (C₁-C₁₀-cycloalkyl), -NH(heterocyclyl-(C₁-C₆-alkyl-)), -NH(aryl-(C₁-C₆-alkyl-)), -C(O)NH₂, -C(O)NH-(C₁-C₆-alkyl), aryl, and heteroaryl,
and aryl, heterocyclyl and heteroaryl may in turn be at least monosubstituted with C₁-C₃-Alkyl, C₁-C₃-alkoxy, fluorine, chlorine, bromine, trifluoromethyl, trifluoromethoxy or OH;
heteroaryl is pyridinyl or pyrimidinyl;
aryl is phenyl or naphthyl;
hetrocyclyl is morpholinyl, piperazinyl or piperidinyl;
or the racemates, enantiomers, diastereoisomers and mixtures thereof, the tautomers or the physiologically acceptable salts thereof.

6. A compound according to any of claims 1 to 5, wherein in formula (I)
R is unsubstituted or at least monosubstituted benzyl, phenylethyl-, phenylpropyl-, pyridinylmethyl-, pyridinylethyl- or pyridinylpropyl-,
where the substituents are selected from chlorine, bromine, fluorine, trifluoromethyl and carboxy;
Ar is unsubstituted or at least monosubstituted pyridin-4-yl, pyrimidin-4-yl or phenyl,
where the substituents are selected from methylamino-, ethylamino-, propylamino-, butylamino-, hydroxy, methoxy, ethoxy, methyl, ethyl, propyl, (phenylethyl)amino-, benzylamino- and (morpholinylethyl)amino-;
or the racemates, enantiomers, diastereoisomers and mixtures thereof, the tautomers or the physiologically acceptable salts thereof.

7. A compound according to any of claims 1 to 6 selected from the group consisting of
6-(2-butylamino-pyrimidin-4-yl)-3-oxo-2,3-dihydro-pyridazine-4-carboxylic acid (3-pyridin- 3-yl-propyl)-amide,
6-(4-hydroxy-3-methoxy-phenyl)-3-oxo-2,3-dihydro-pyridazine-4-carboxylic acid (3-pyridin-3-yl-propyl)-amide,
6-(4-hydroxy-3,5-dimethyl-phenyl)-3-oxo-2,3-dihydro-pyridazine-4-carboxylic acid (3-pyridin-3-yl-propyl)-amide,
6-(4-hydroxy-phenyl)-3-oxo-2,3-dihydro-pyridazine-4-carboxylic acid (3-pyridin-3-yl-propyl)-amide,
6-(2-ethylamino-pyrimidin-4-yl)-3-oxo-2,3-dihydro-pyridazine-4-carboxylic acid 4-chloro-benzylamide,
6-(3-chloro-4-hydroxy-phenyl)-3-oxo-2,3-dihydro-pyridazine-4-carboxylic acid 4-chloro-benzylamide,
6-(4-hydroxy-3,5-dimethyl-phenyl)-3-oxo-2,3-dihydro-pyridazine-4-carboxylic acid 4-chloro-benzylamide,
4-({[6-(4-hydroxy-3,5-dimethyl-phenyl)-3-oxo-2,3-dihydro-pyridazine-4-carbonyl]-amino}- methyl)-benzoic acid,
4-({[6-(4-hydroxy-3-methoxy-phenyl)-3-oxo-2,3-dihydro-pyridazine-4-carbonyl]-aminol- methyl)-benzoic acid,
6-(2-butylamino-pyrimidin-4-yl)-3-oxo-2,3-dihydro-pyridazine-4-carboxylic acid (pyridin-3- ylmethyl)-amide,
6-(3-fluoro-4-hydroxy-phenyl)-3-oxo-2,3-dihydro-pyridazine-4-carboxylic acid 4-chloro-benzylamide,
6-(4-hydroxy-3-methyl-phenyl)-3-oxo-2,3-dihydro-pyridazine-4-carboxylic acid 4-chloro-benzylamide,
6-[2-(2-morpholin-4-yl-ethylamino)-pyrimidin-4-yl]-3-oxo-2,3-dihydro-pyridazine-4- carboxylic acid 4-chloro-benzylamide,
6-(4-hydroxy-3-methoxy-phenyl)-3-oxo-2,3-dihydro-pyridazine-4-carboxylic acid 4-chloro-benzylamide,
6-(2-methylamino-pyrimidin-4-yl)-3-oxo-2,3-dihydro-pyridazine-4-carboxylic acid 4-chloro-benzylamide,
R-3-oxo-6-[2-(1-phenyl-ethylamino)-pyrimidin-4-yl]-2,3-dihydro-pyridazine-4-carboxylic acid (3-phenyl-propyl)-amide,
6-(4-hydroxy-phenyl)-3-oxo-2,3-dihydro-pyridazine-4-carboxylic acid 4-chloro-benzylamide,
3-oxo-6-pyridin-4-yl-N-[4-(trifluoromethyl)benzyl]-2,3-dihydropyridazine-4-carboxamide,
3-oxo-6-pyridin-4-yl-2,3-dihydro-pyridazine-4-carboxylic acid 4-bromo-benzylamide,
3-oxo-6-pyridin-4-yl-N-(pyridin-3-ylmethyl)-2,3-dihydropyridazine-4-carboxamide
N-(2,4-dichlorobenzyl)-3-oxo-6-pyridin-4-yl-2,3-dihydropyridazine-4-carboxamide,
3-oxo-6-pyridin-4-yl-2,3-dihydro-pyridazine-4-carboxylic acid 4-chloro-2-fluoro-benzylamide, and
N-(4-chlorobenzyl)-3-oxo-6-pyridin-4-yl-2,3-dihydropyridazine-4-carboxamide;
or the racemates, enantiomers, diastereoisomers and mixtures thereof, the tautomers or the physiologically acceptable salts thereof.

8. A compound according to any of claims 1 to 7 or a physiologically acceptable salt thereof for use as pharmaceutical.

9. The use of a compound according to any of claims 1 to 7 or a physiologically acceptable salt thereof for the manufacture of a medicament for prophylaxis and/or treatment of diseases in which phosphorylation of the Tau protein is observed.

10. The use of a compound according to any of claims 1 to 7 or a physiologically acceptable salt thereof for the manufacture of a medicament which is an inhibitor of GSK-3β.

11. The use of a compound according to any of claims 1 to 7 or a physiologically acceptable salt thereof for the manufacture of a medicament for prophylaxis and/or treatment of neurodegenerative diseases, strokes, cranial and spinal traumas and peripheral neuropathies, obesity, metabolic diseases, type II diabetes, essential hypertension, atherosclerotic cardiovascular diseases, polycystic ovary syndrome, syndrome X, immunodeficiency or cancer.

12. The use according to claim 11, wherein the neurodegenerative disease is Alzheimer's disease, Parkinson's disease, frontoparietal dementia, corticobasal degeneration or Pick's disease.

13. The use according to claim 11 for prophylaxis and/or treatment of type-II-diabetes or Alzheimer's disease.

14. A pharmaceutical preparation comprising an effective dose of at least one compound or a physiologically acceptable salt thereof as defined in any of claims 1 to 7 and a physiologically acceptable carrier.

15. A pharmaceutical preparation according to claim 14, which pharmaceutical preparation is in the form of a pill, tablet, lozenge, coated tablet, granuie, capsule, hard or soft gelatin capsule, aqueous solution, alcoholic solution, oily solution, syrup, emulsion suspension, pastille, suppository, solution for injection or infusion, ointment, tincture, cream, lotion, powder, spray, transdermal therapeutic systems, nasal spray, aerosol mixture, microcapsule, implant, rod or plaster.

16. A method for the synthesis of a compound of formula (I) according to any of claims 1 to 7, wherein
a) a compound of formula (IV) 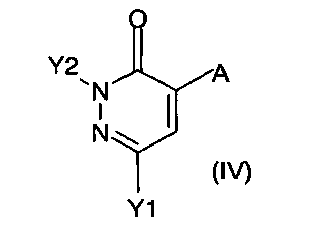 where Y1 is halogen, B(OH)₂ or Sn(C₁-C₁₀-alkyl) and
Y2 is H or a protecting group,
is converted with Ar-Z in presence of a palladium complex, where Z is B(OH)₂,
B(C₁-C₁₀-alkoxy)₂, Sn(C₁-C₁₀-alkyl)₃, Zn-(C₁-C₁₀-alkyl) or halogen,
or
b) with the proviso that in formula (I) A is A1, a compound of formula (II) 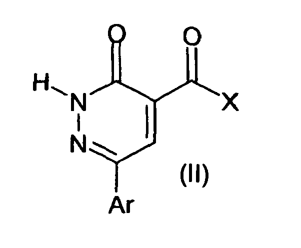
where X is -OH, C₁-C₁₀-alkoxy, chlorine or -O-C(O)O-(C₁-C₁₀-alkyl), is converted with RNH₂ .

## Patentansprüche

1. Verbindungen der Formel (I) 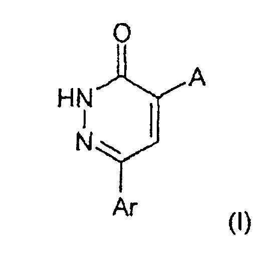 wobei A für Al steht 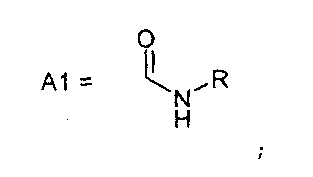
R für unsubstituiertes oder wenigstens einfach substituiertes C₁-C₁₀-Alkyl, Aryl, Aryl-(C₁-C₁₀-alkyl) -, Heteroaryl, Heteroaryl-(C₁-C₁₀-alkyl) -, Heterocyclyl, Heterocyclyl-(C₁-C₁₀-alkyl) -, C₃-C₁₀-Cycloalkyl, Polycyclo-alkyl, C₂-C₁₀-Alkenyl oder C₂-C₁₀-Alkinyl steht,
wobei die Substituenten ausgewählt sind aus Halogen, -CN, C₁-C₁₀-Alkyl, -NO₂, -OR1, -C (O) OR1, -O-C (O) R1, -NR1R2, -NHC (O) R1, -C (O) NR1R2, -SR1, -S (O) R1, -SO₂R1, -NHSO₂R1, -SO₂NR1R2, -C(S)NR1R2, -NHC (S) R1, -O-SO₂R1, - SO₂-O-R1, Oxo, -C (O) R1, -C (NH) NH₂, Hetero-cyclyl, C₃-C₁₀-Cycloalkyl, Aryl- (C₁-C₆-alkyl) -, Aryl, Heteroaryl, Trifluormethyl, Trifluor-methylsulfanyl und Trifluormethoxy,
und Aryl, Heterocyclyl und Heteroaryl ihrerseits wenigstens einfach substituiert sein können durch C₁-C₆-Alkyl , C₁-C₆-Alkoxy, Halogen, Trifluormethyl, Trifluormethoxy oder OH;
Ar für unsubstituiertes oder wenigstens einfach substituiertes Aryl oder Heteroaryl steht;
wobei die Substituenten ausgewählt sind aus Halogen, -CN, NO₂, C₁-C₁₀-Alkyl, -OR1, -C (O) OR1, -O-C (O) R1, -NR1R2, -NHC (O) R1, -C (O) NR1R2, -NHC(S) R1, -C(S)NR1R2, -SR1, -S (O) R1, -SO₂R1, -NHSO₂R1, -SO₂NR1R2, -O-SO₂R1, -SO₂-O-R1, Aryl, Heteroaryl, Aryl- (C₁-C₆-alkyl)-, Formyl, Trifluormethyl und Trifluormethoxy,
und Aryl und Heteroaryl ihrerseits wenigstens einfach substituiert sein können durch C₁-C₆-Alkyl, C₁-C₆-Alkoxy, Halogen, Trifluormethyl, Trifluormethoxy oder OH;
R1 und R2 unabhängig voneinander für
Wasserstoff;
unsubstituiertes oder wenigstens einfach substituiertes C₁-C₁₀-Alkyl, C₃-C₁₀-Cycloalkyl, Aryl, Aryl- (C₁-C₁₀-alkyl) -, C₂-C₁₀-Alkenyl, C₂-C₁₀-Alkinyl, Heterocyclyl, Heterocyclyl-(C₁-C₁₀-alkyl) - oder Heteroaryl stehen, wobei die Substituenten ausgewählt sind aus Halogen, C₁-C₆-Alkyl, C₁-C₆-Alkoxy, CN, NO₂, NH₂, (C₁-C₆-Alkyl) amino-, Di (C₁-C₆-alkyl)amino-, OH, COOH, -COO-(C₁-C₆-Alkyl), -CONH₂, Formyl, Trifluormethyl und Trifluormethoxy;
Heteroaryl für einen 5- bis 10-gliedrigen aromatischen mono- oder bicyclischen Heterocyclus mit einem oder mehreren Heteroatomen ausgewählt aus N, O und S steht;
Aryl für Phenyl, Indanyl, Indenyl oder Naphtyl steht;
Heterocyclyl für einen 5- bis 10-gliedrigen aliphatischen mono- oder bicyclischen Heterocyclus mit einem oder mehreren Heteroatomen ausgewählt aus N, O und S steht;
und deren Racemate, Enantiomere, Diastereoisomere und Mischungen davon, deren Tautomere und die physiologisch unbedenklichen Salze davon;
mit der Maßgabe, daß A nicht für -C(O)NH(C₁-C₆-Alkyl) steht, wenn Ar für Phenyl steht, das wenigstens einfach substituiert ist durch Heterocyclyl oder Heteroaryl, das Stickstoff enthält, und daß die folgenden Verbindungen ausgenommen sind:
3-{4-(3,4,5-Trimethoxyanilinocarbonyl)-3-oxo-2,3-dihydropyridazin-6-yl}-2-phenylpyrazolo[1,5-a]pyridin,
3-{4-(N-Ethoxycarbonylmethyl)carbamoyl-3-oxo-2,3-dihydropyridazin-6-yl}-2-phenylpyrazolo[1,5-a]pyridin,
3-(4-(N-Carboxymethyl)carbamoyl-3-oxo-2,3-dihydropyridazin-6-yl}-2-phenylpyrazolo[1,5-a]pyridin,
6-(4-Cyanophenyl)-4-[(4-carboxybutyl)aminocarbonyl]-(2H)-pyridazin-3-on und
6-(4-Methoxyphenyl)-4-methylcarbamoyl-(2H)-pyridazin-3-on.

2. Verbindungen nach Anspruch 1, wobei in der Formel (I)
R für unsubstituiertes oder wenigstens einfach substituiertes C₁-C₁₀-Alkyl, Aryl, Aryl-(C₁-C₁₀-alkyl)-, Heterocyclyl, Heterocyclyl-(C₁-C₁₀-alkyl)-, C₃-C₁₀-Cycloalkyl, Heteroaryl oder Heteroaryl-(C₁-C₁₀-alkyl) - steht,
wobei die Substituenten ausgewählt sind aus Halogen, -CN, C₁-C₁₀-Alkyl, -NO₂, -OR1, -C(O)OR1, -O-C(O)R1, -NR1R2, -NHC(O)R1, -C(O)NR1R2, -SR1, -S(O)R1, -SO₂R1, -NHSO₂R1, -SO₂NR1R2, -C (S) NR1R2, -NHC (S) R1, -O-SO₂R1, -SO₂-O-R1, Oxo, -C(O)R1, -C(NH)NH₂, Heterocyclyl, C₃-C₁₀-Cycloalkyl, Aryl-(C₁-C₆-lkyl)-, Aryl, Heteroaryl, Trifluormethyl, Trifluormethylsulfanyl und Trifluormethoxy,
und Aryl, Heterocyclyl und Heteroaryl ihrerseits wenigstens einfach substituiert sein können durch C₁-C₆-Alkyl, C₁-C₆-Alkoxy, Halogen, Trifluormethyl, Trifluorethoxy oder OH;
R1 und R2 unabhängig voneinander für
Wasserstoff;
unsubstituiertes oder wenigstens einfach substituiertes C₁-C₁₀-Alkyl, C₃-C₁₀-Cycloalkyl, Aryl, Aryl- (C₁-C₁₀-alkyl) -, C₂-C₁₀-Alkenyl, C₂-C₁₀-Alkinyl, Heterocyclyl, Heterocyclyl(C₁-C₁₀-alkyl) - oder Heteroaryl stehen, wobei die Substituenten ausgewählt sind aus Halogen, C₁-C₆-Alkyl, C₁-C₆-Alkoxy, CN, NO₂, NH₂, (C₁-C₆-Alkyl) amino-, Di (C₁-C₆-alkyl) amino-, OH, COOH, -COO-(C₁-C₆-Alkyl), -CONH₂, Formyl, Trifluormethyl und Trifluormethoxy;
Heteroaryl für einen 5- bis 10-gliedrigen aromatischen mono- oder bicyclischen Heterocyclus mit einem oder mehreren Heteroatomen ausgewählt aus N, O und S steht;
Aryl für Phenyl, Indanyl, Indenyl oder Naphtyl steht;
Heterocyclyl für einen 5- bis 10-gliedrigen aliphatischen mono- oder bicyclischen Heterocyclus mit einem oder mehreren Heteroatomen ausgewählt aus N, O und S steht;
und deren Racemate, Enantiomere, Diastereoisomere und Mischungen davon, deren Tautomere und die physiologisch unbedenklichen Salze davon.

3. Verbindungen nach Anspruch 1 oder 2, wobei in der Formel (I)
Ar für unsubstituiertes oder wenigstens einfach substituiertes Phenyl, Pyridinyl, Pyrimidinyl, Pyrazolyl, Thiophenyl, Isoxazolyl, Benzo[b]thiophenyl, Benzodioxolyl oder Thiazolo[3,2-b][1,2,4]-thiazolyl steht,
wobei die Substituenten ausgewählt sind aus Halogen, -CN, NO₂, C₁-C₁₀-Alkyl, -OR1, -C (O) OR1, -O-C (O) R1, -NR1R2, -NHC (O) R1, -C (O) NR1R2, -NHC(S)R1, -C (S) NR1R2, -SR1, -S (O) R1, -SO₂R1, -NHSO₂R1, -SO₂NR1R2, -O-SO₂R1, -SO₂-O-R1, Aryl, Heteroaryl, Aryl-(C₁-C₆-alkyl)-, Formyl, Trifluormethyl und Trifluormethoxy,
und Aryl und Heteroaryl ihrerseits wenigstens einfach substituiert sein können durch C₁-C₆-Alkyl, C₁-C₆-Alkoxy, Halogen, Trifluormethyl, Trifluormethoxy oder OH;
R1 und R2 unabhängig voneinander für
Wasserstoff;
unsubstituiertes oder wenigstens einfach substituiertes C₁-C₁₀-Alkyl, C₃-C₁₀-Cycloalkyl, Aryl, Aryl- (C₁-C₁₀-alkyl) -, C₂-C₁₀-Alkenyl, C₂-C₁₀-Alkinyl, Heterocyclyl, Heterocyclyl-(C₁-C₁₀-alkyl)- oder Heteroaryl stehen, wobei die Substituenten ausgewählt sind aus Halogen, C₁-C₆-Alkyl, C₁-C₆-Alkoxy, CN, NO₂, NH₂, (C₁-C₆-Alkyl) amino-, Di (C₁-C₆-alkyl)amino-, OH, COOH, -COO-(C₁-C₆-Alkyl), -CONH₂, Formyl, Trifluormethyl und Trifluormethoxy;
Heteroaryl für einen 5- bis 10-gliedrigen aromatischen mono- oder bicyclischen Heterocyclus mit einem oder mehreren Heteroatomen ausgewählt aus N, O und S steht;
Aryl für Phenyl, Indanyl, Indenyl oder Naphtyl steht;
Heterocyclyl für einen 5- bis 10-gliedrigen aliphatischen mono- oder bicyclischen Heterocyclus mit einem oder mehreren Heteroatomen ausgewählt aus N, O und S steht;
und deren Racemate, Enantiomere, Diastereoisomere und Mischungen davon, deren Tautomere und die physiologisch unbedenklichen Salze davon.

4. Verbindungen nach einem der Ansprüche 1 bis 3, wobei in der Formel (I)
R für unsubstituiertes oder wenigstens einfach substituiertes Aryl-(C₁-C₆-alkyl)- oder Heteroaryl-(C₁-C₆-alkyl)- steht,
wobei die Substituenten ausgewählt sind aus Halogen, C₁-C₆-Alkyl, -OH, -O-Aryl, C₁-C₆-Alkoxy, -O- (C₁-C₆-Alkylen) -N (C₁-C₆-alkyl)₂, -C (O) OH, -C (O) O- (C₁-C₆-Alkyl), -NH₂, -N(C₁-C₆-Alkyl)₂, -NH (C₁-C₆-Alkyl) , -NH(C₁-C₁₀-Cycloalkyl), -C(O)NH₂, -C(O)NH-Heteroaryl, -C(O)NH- (C₁-C₆-Alkyl), -SO₂(C₁-C₆-Alkyl), -SO₂NH₂, -C(O)-Heterocyclyl, -C (NH) NH₂, Heterocyclyl, Aryl-(C₁-C₆-alkyl)-, Aryl, Trifluormethyl und Trifluormethoxy,
und Aryl, Heterocyclyl und Heteroaryl ihrerseits wenigstens einfach substituiert sein können durch C₁-C₃-Alkyl, C₁-C₃-Alkoxy, Fluor, Chlor, Brom, Trifluormethyl, Trifluormethoxy oder OH;
Heteroaryl für Imidazolyl, Thiophenyl, Furanyl, Isoxazolyl, Pyridinyl, Pyrimidinyl, Benzoimidazolyl, Indolyl oder Benzodioxolyl steht;
Aryl für Phenyl oder Naphtyl steht;
Heterocyclyl für Morpholinyl, Piperazinyl oder Piperidinyl steht;
und deren Racemate, Enantiomere, Diastereoisomere und Mischungen davon, deren Tautomere und die physiologisch unbedenklichen Salze davon.

5. Verbindungen nach einem der Ansprüche 1 bis 4, wobei in der Formel (I)
Ar für unsubstituiertes oder wenigstens einfach substituiertes Phenyl, Pyridin-4-yl oder Pyrimidin-4-yl steht,
wobei die Substituenten ausgewählt sind aus Halogen, C₁-C₆-Alkyl, -OH, C₁-C₆-Alkoxy, -C(O)OH, -C(O)O-(C₁-C₆-Alkyl),-NH₂, -N(C₁-C₆-Alkyl)₂, -NH (C₁-C₆-Alkyl), -NH(C₁-C₁₀-Cycloalkyl), -NH(Heterocyclyl-(C₁-C₆-alkyl-)), -NH (Aryl- (C₁-C₆-alkyl-) ) , -C (O) NH₂, -C (O)NH-(C₁-C₆-Alkyl), Aryl und Heteroaryl,
und Aryl, Heterocyclyl und Heteroaryl ihrerseits wenigstens einfach substituiert sein können durch C₁-C₃-Alkyl, C₁-C₃-Alkoxy, Fluor, Chlor, Brom, Trifluormethyl, Trifluormethoxy oder OH;
Heteroaryl für Pyridinyl oder Pyrimidinyl steht;
Aryl für Phenyl oder Naphtyl steht;
Heterocyclyl für Morpholinyl, Piperazinyl oder Piperidinyl steht;
und deren Racemate, Enantiomere, Diastereoisomere und Mischungen davon, deren Tautomere und die physiologisch unbedenklichen Salze davon.

6. Verbindungen nach einem der Ansprüche 1 bis 5, wobei in der Formel (I)
R für unsubstituiertes oder wenigstens einfach substituiertes Benzyl, Phenylethyl-, Phenylpropyl-, Pyridinylmethyl-, Pyridinylethyl- oder Pyridinylpropyl- steht,
wobei die Substituenten ausgewählt sind aus Chlor, Brom, Fluor, Trifluormethyl und Carboxy;
Ar für unsubstituiertes oder wenigstens einfach substituiertes Pyridin-4-yl, Pyrimidin-4-yl oder Phenyl steht,
wobei die Substituenten ausgewählt sind aus Methylamino-, Ethylamino-, Propylamino-, Butylamino-, Hydroxy, Methoxy, Ethoxy, Methyl, Ethyl, Propyl, (Phenylethyl)-amino-, Benzylamino- und (Morpholinylethyl)amino-;
und deren Racemate, Enantiomere, Diastereoisomere und Mischungen davon, deren Tautomere und die physiologisch unbedenklichen Salze davon.

7. Verbindungen nach einem der Ansprüche 1 bis 6, ausgewählt aus der aus
6-(2-Butylaminopyrimidin-4-yl)-3-oxo-2,3-dihydropyridazin-4-carbonsäure-(3-pyridin-3-ylpropyl)amid,
6-(4-Hydroxy-3-methoxyphenyl)-3-oxo-2,3-dihydropyridazin-4-carbonsäure-(3-pyridin-3-yl-propyl) amid,
6-(4-Hydroxy-3,5-dimethylphenyl)-3-oxo-2,3-dihydropyridazin-4-carbonsäure-(3-pyridin-3-ylpropyl)amid,
6-(4-Hydroxyphenyl)-3-oxo-2,3-dihydropyridazin-4-carbonsäure-(3-pyridin-3-ylpropyl)amid,
6-(2-Ethylaminopyrimidin-4-yl)-3-oxo-2,3-dihydropyridazin-4-carbonsäure-4-chlorbenzylamid,
6-(3-Chlor-4-hydroxyphenyl)-3-oxo-2,3-dihydropyridazin-4-carbonsäure-4-chlorbenzylamid,
6-(4-Hydroxy-3,5-dimethylphenyl)-3-oxo-2,3-dihydropyridazin-4-carbonsäure-4-chlorbenzylamid,
4-({[6-(4-Hydroxy-3,5-dimethylphenyl)-3-oxo-2,3-dihydropyridazin-4-carbonyl]amino}methyl)benzoesäure,
4-({[6-(4-Hydroxy-3-methoxyphenyl)-3-oxo-2,3-dihydropyridazin-4-carbonyl]amino}methyl)benzoesäure,
6-(2-Butylaminopyrimidin-4-yl)-3-oxo-2,3-dihydropyridazin-4-carbonsäure(pyridin-3-ylmethyl)amid,
6-(3-Fluor-4-hydroxyphenyl)-3-oxo-2,3-dihydropyridazin-4-carbonsäure-4-chlorbenzylamid,
6-(4-Hydroxy-3-methylphenyl)-3-oxo-2,3-dihydropyridazin-4-carbonsäure-4-chlorbenzylamid,
6-[2-(2-Morpholin-4-yl-ethylamino)pyrimidin-4-yl]-3-oxo-2,3-dihydropyridazin-4-carbonsäure-4-chlorbenzylamid,
6-(4-Hydroxy-3-methoxyphenyl)-3-oxo-2,3-dihydropyridazin-4-carbonsäure-4-chlorbenzylamid,
6-(2-Methylaminopyrimidin-4-yl)-3-oxo-2,3-dihydropyridazin-4-carbonsäure-4-chlorbenzylamid, R-3-Oxo-6-[2-(1-phenylethylamino)pyrimidin-4-yl]-2,3-dihydropyridazin-4-carbonsäure-(3-phenylpropyl) amid,
6-(4-Hydroxyphenyl)-3-oxo-2,3-dihydropyridazin-4-carbonsäure-4-chlorbenzylamid,
3-Oxo-6-pyridin-4-yl-N-[4-(trifluormethyl)-benzyl]-2,3-dihydropyridazin-4-carbonsäureamid,
3-Oxo-6-pyridin-4-yl-2,3-dihydropyridazin-4-carbonsäure-4-brombenzylamid,
3-Oxo-6-pyridin-4-yl-N-(pyridin-3-ylmethyl)-2,3-dihydropyridazin-4-carbonsäureamid,
N-(2,4-Dichlorbenzyl)-3-oxo-6-pyridin-4-yl-2,3-dihydropyridazin-4-carbonsäureamid,
3-Oxo-6-pyridin-4-yl-2,3-dihydropyridazin-4-carbonsäure-4-chlor-2-fluorbenzylamid und
N-(4-Chlorbenzyl)-3-oxo-6-pyridin-4-yl-2,3-dihydropyridazin-4-carbonsäureamid bestehenden Gruppe;
und deren Racemate, Enantiomere, Diastereoisomere und Mischungen davon, deren Tautomere und die physiologisch unbedenklichen Salze davon.

8. Verbindungen nach einem der Ansprüche 1 bis 7 und deren physiologisch unbedenkliche Salze zur Verwendung als Pharmazeutika.

9. Verwendung einer Verbindung nach einem der Ansprüche 1 bis 7 oder eines physiologisch unbedenklichen Salzes davon zur Herstellung eines Medikaments zur Prophylaxe und/oder Behandlung von Krankheiten, bei denen eine Phosphorylierung des Tau-Proteins beobachtet wird.

10. Verwendung einer Verbindung nach einem der Ansprüche 1 bis 7, oder eines physiologisch unbedenklichen Salzes davon zur Herstellung eines Medikaments, bei dem es sich um einen Inhibitor von GSK-3β handelt.

11. Verwendung einer Verbindung nach einem der Ansprüche 1 bis 7, oder eines physiologisch unbedenklichen Salzes davon zur Herstellung eines Medikaments zur Prophylaxe und/oder Behandlung von neurodegenerativen Krankheiten, Schlaganfällen, Schädel- und Spinaltraumata und peripheren Neuropathien, Obesitas, Stoffwechselkrankheiten, Typ-II-Diabetes, essentieller Hypertonie, atherosclerotischen Herzkreislaufkrankheiten, Syndrom der polyzystischen Ovarien, Syndrom X, Immundefizienz oder Krebs.

12. Verwendung einer Verbindung nach Anspruch 11, wobei es sich bei der neurodegenerativen Krankheit um Alzheimer-Krankheit, Parkinson-Krankheit, frontoparietale Demenz, kortikobasale Degeneration oder Pick-Krankheit handelt.

13. Verwendung nach Anspruch 11 zur Prophylaxe und/oder Behandlung von Typ-II-Diabetes oder Alzheimer-Krankheit.

14. Pharmazeutische Zubereitung, enthaltend eine wirksame Dosis wenigstens einer Verbindung oder eines physiologisch unbedenklichen Salzes davon nach einem der Ansprüche 1 bis 7 und einen physiologisch unbedenklichen Träger.

15. Pharmazeutische Zubereitung nach Anspruch 14, wobei die pharmazeutische Zubereitung in Form einer Pille, einer Tablette, einer Lutschtablette, eines Dragees, eines Granulats, einer Kapsel, einer Hart- oder Weichgelatinekapsel, einer wäßrigen Lösung, einer alkoholischen Lösung, einer öligen Lösung, einem Sirup, einer Emulsionssuspension, einer Pastille, einem Zäpfchen, einer Injektions- oder Infusionslösung, einer Salbe, einer Tinktur, einer Creme, einer Lotion, eines Pulvers, eines Sprays, eines transdermalen therapeutischen Systems, eines Nasensprays, einer Aerosolmischung, einer Mikrokapsel, eines Implantats, eines Stäbchens oder eines Pflasters vorliegt.

16. Verfahren zur Synthese einer Verbindung der Formel (I) nach einem der Ansprüche 1 bis 7, bei dem man
a) eine Verbindung der Formel (IV) 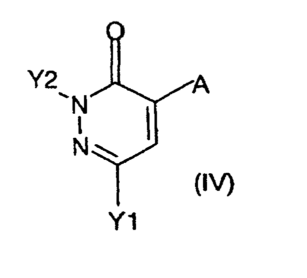 wobei Y1 für Halogen, B(OH)₂ oder Sn(C₁-C₁₀-Alkyl) steht und
Y2 für H oder eine Schutzgruppe steht,
in Gegenwart eines Palladiumkomplexes mit Ar-Z umwandelt, wobei Z für B(OH)₂, B (C₁-C₁₀-Alkoxy)₂, Sn(C₁-C₁₀-Alkyl)₃, Zn-(C₁-C₁₀-Alkyl) oder Halogen steht,
oder
b) mit der Maßgabe, daß in der Formel (I) A für Al steht, eine Verbindung der Formel (II) 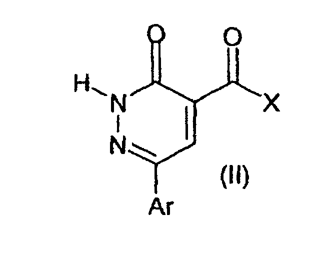 wobei X für -OH, C₁-C₁₀-Alkoxy, Chlor oder -O-C (O) O- (C₁-C₁₀-Alkyl) steht, mit RNH₂ umwandelt.

## Revendications

1. Composé de formule (I) 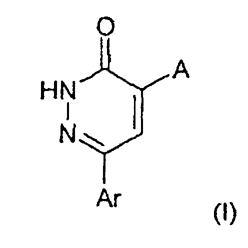 dans laquelle A représente A1 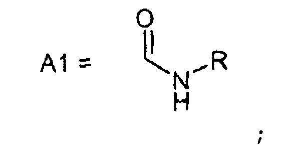
R est C₁-C₁₀-alkyle, aryle, aryl- (C₁-C₁₀-alkyl) -, hétéroaryle, hétéroaryl-(C₁-C₁₀-alkyl)-, hétérocyclyle, hétérocyclyl- (C₁-C₁₀-alkyl) -, C₃-C₁₀-cycloalkyle, polycycloalkyle, C₂-C₁₀-alcényle ou C₂-C₁₀-alcinyle, non substitué ou au moins monosubstitué,
où les substituants sont choisis parmi halogène, -CN, C₁-C₁₀-alkyle, -NO₂, -OR1, -C (O) OR1, -O-C (O) R1, -NR1R2, -NHC (O) R1, -C (O) NR1R2, -SR1, -S (O) R1, -SO₂R1, -NHSO₂R1, -SO₂NR1R2, -C (S) NR1R2, -NHC (S) R1, -O-SO₂R1, -SO₂-O-R1, oxo, -C (O) R1, -C (NH) NH₂, hétérocyclyle, C₃-C₁₀-cycloalkyle, aryl-(C₁-C₆-alkyl)-, aryle, hétéroaryle, trifluorométhyle, trifluorométhylsulfanyle et trifluorométhoxy,
et aryle, hétérocyclyle et hétéroaryle peuvent à leur tour être au moins monosubstitués par C₁-C₆-alkyle, C₁-C₆-alcoxy, halogène, trifluorométhyle, trifluorométhoxy ou OH ;
Ar est aryle ou hétéroaryle non substitué ou au moins monosubstitué ;
où les substituants sont choisis parmi halogène, -CN, NO₂ C₁-C₁₀-alkyle, -OR1, -C (O) OR1, -O-C (O) R1, -NR1R2, -NHC (O) R1, -C (O) NR1R2, -NHC (S) R1, -C (S) NR1R2, -SR1, -S(O)R1, -SO₂R1, -NHSO₂R1, -SO₂NR1R2, -O-SO₂R1, -SO₂-O-R1, aryle, hétéroaryle, aryl-(C₁-C₆-alkyl)-, formyle, trifluorométhyle et trifluorométhoxy,
et aryle et hétéroaryle peuvent à leur tour être au moins monosubstitués par C₁-C₆-alkyle, C₁-C₆-alcoxy, halogène, trifluoro-méthyle, trifluorométhoxy ou OH ;
R1 et R2 sont, indépendamment l'un de l'autre,
hydrogène ;
C₁-C₁₀-alkyle, C₃-C₁₀-cycloalkyle, aryle, aryl-(C₁-C₁₀-alkyl) -, C₂-C₁₀-alcényle, C₂-C₁₀-alcinyle, hétérocyclyle, hétérocyclyl-(C₁-C₁₀-alkyl)- ou hétéroaryle, non substitué ou au moins monosubstitué, où les substituants sont choisis parmi halogène, C₁-C₆-alkyle, C₁-C₆-alcoxy, CN, NO₂, NH₂, (C₁-C₆-alkyl)amino-, di(C₁-C₆-alkyl)amino-, OH, COOH, -COO- (C₁-C₆-alkyle), -CONH₂, formyle, trifluorométhyle et trifluorométhoxy ;
hétéroaryle est un hétérocycle aromatique, mono-ou bicyclique de 5 à 10 chaînons contenant un ou plusieurs hétéroatomes choisis parmi N, O et S;
aryle est phényle, indanyle, indényle ou naphtyle ;
hétérocyclyle est un hétérocycle aliphatique, mono- ou bicyclique de 5 à 10 chaînons contenant un ou plusieurs hétéroatomes choisis parmi N, O et S ;
ou les racémates, les énantiomères, les diastéréo-isomères et les mélanges de celui-ci, les tautomères ou les sels physiologiquement acceptables de ceux-ci ;
à condition que A ne soit pas -C (O) NH (C₁-C₆-alkyle), dans le cas où Ar est phényle qui est au moins monosubstitué par hétérocyclyle ou hétéroaryle contenant de l'azote, et que les composés suivants soient exclus :
la 3-f4-(3,4,5-triméthoxyanilinocarbonyl)-3-oxo-2,3-dihydropyridazin-6-yl}-2-phénylpyrazolo[1,5-a]pyridine,
la 3-{4-(N-éthoxycarbonylméthyl)-carbamoyl-3-oxo-2,3-dihydropyridazin-6-yl}-2-phénylpyrazolo[1,5-a]pyridine,
la 3-(4-(N-carboxyméthyl)-carbamoyl-3-oxo-2,3-dihydropyridazin-6-yl}-2-phénylpyrazolo[1,5-a]pyridine,
la 6-(4-(cyanophényl)-4[(4-carboxybutyl)-amino-carbonyl]-(2H)-pyridazin-3-one et
la 6-(4-méthoxyphényl)-4-méthylcarbamoyl-(2H)-pyridazin-3-one.

2. Composé selon la revendication 1, **caractérisé en ce que** dans la formule (I)
R est C₁-C₁₀-alkyle, aryle, aryl-(C₁-C₁₀-alkyl)- , hétérocyclyle, hétérocyclyl-(C₁-C₁₀-alkyl)- , C₃-C₁₀-cycloalkyle, hétéroaryle ou hétéroaryl-(C₁-C₁₀-alkyl) -, non substitué ou au moins monosubstitué,
où les substituants sont choisis parmi halogène, -CN, C₁-C₁₀-alkyle, -NO₂, -OR1, -C (O) OR1, -O-C (O) R1, -NR1R2, -NHC (O) R1, -C (O)NR1R2, -SR1, -S (O) R1, -SO₂R1, -NHSO₂R1, -SO₂NR1R2, -C (S) NR1R2, -NHC (S) R1, -O-SO₂R1, -SO₂-O-R1, oxo, -C (O) R1, -C (NH) NH₂, hétéro-cyclyle, C₃-C₁₀-cycloalkyle, aryl-(C₁-C₆-alkyl)-, aryle, hétéroaryle, trifluorométhyle, trifluorométhylsulfanyle et trifluorométhoxy,
et aryle, hétérocyclyle et hétéroaryle peuvent à leur tour être au moins monosubstitués par C₁-C₆-alkyle, C₁-C₆-alcoxy, halogène, trifluorométhyle, trifluoroéthoxy ou OH ;
R1 et R2 sont, indépendamment l'un de l'autre,
hydrogène ;
C₁-C₁₀-alkyle, C₃-C₁₀-cycloalkyle, aryle, aryl-(C₁-C₁₀-alkyl) -, C₂-C₁₀-alcényle, C₂-C₁₀-alcinyle, hétérocyclyle, hétérocyclyl-(C₁-C₁₀-alkyl)- ou hétéroaryle, non substitué ou au moins monosubstitué, où les substituants sont choisis parmi halogène, C₁-C₆-alkyle, C₁-C₆-alcoxy, CN, NO₂, NH₂, (C₁-C₆-alkyl)amino-, di(C₁-C₆-alkyl)amino-, OH, COOH, -COO-(C₁-C₆-alkyle), -CONH₂, formyle, trifluorométhyle et trifluorométhoxy ;
hétéroaryle est un hétérocycle aromatique, mono-ou bicyclique de 5 à 10 chaînons contenant un ou plusieurs hétéroatomes choisis parmi N, O et S ;
aryle est phényle, indanyle, indényle ou naphtyle ;
hétérocyclyle est un hétérocycle aliphatique, mono- ou bicyclique de 5 à 10 chaînons contenant un ou plusieurs hétéroatomes choisis parmi N, O et S ;
ou les racémates, les énantiomères, les diastéréo-isomères et les mélanges de celui-ci, les tautomères ou les sels physiologiquement acceptables de ceux-ci.

3. Composé selon la revendication 1 ou 2, **caractérisé en ce que** dans la formule (I)
Ar est phényle, pyridinyle, pyrimidinyle, pyrazolyle, thiophényle, isoxazolyle, benzo[b]thiophényle, benzodioxolyle ou thiazolo[3,2-b][1,2,4]-triazolyle, non substitué ou au moins monosubstitué,
où les substituants sont choisis parmi halogène, -CN, NO₂, C₁-C₁₀-alkyle, -OR1, -C (O) OR1, -O-C (O) R1, -NR1R2, -NHC (O) R1, -C(O)NR1R2, -NHC(S)R1, -C(S)NR1R2, -SR1, -S (O) R1, -SO₂R1, -NHSO₂R1, -SO₂NR1R2, -O-SO₂R1, -SO₂-O-R1, aryle, hétéroaryle, aryl-(C₁-C₆-alkyl)-, formyle, trifluorométhyle et trifluorométhoxy,
et aryle et hétéroaryle peuvent à leur tour être au moins monosubstitués par C₁-C₆-alkyle, C₁-C₆-alcoxy, halogène, trifluoro-méthyle, trifluorométhoxy ou OH;
R1 et R2 sont, indépendamment l'un de l'autre,
hydrogène ;
C₁-C₁₀-alkyle, C₃-C₁₀-cycloalkyle, aryle, aryl-(C₁-C₁₀-alkyl)-, C₂-C₁₀-alcényle, C₂-C₁₀-alcinyle, hétérocyclyle, hétérocyclyl-(C₁-C₁₀-alkyl)- ou hétéroaryle, non substitué ou au moins monosubstitué, où les substituants sont choisis parmi halogène, C₁-C₆-alkyle, C₁-C₆-alcoxy, CN, NO₂ NH₂, (C₁-C₆-alkyl) amino-, di(C₁-C₆-alkyl)amino-, OH, COOH, -COO- (C₁-C₆-alkyle), -CONH₂, formyle, tri-fluorométhyle et trifluorométhoxy ;
hétéroaryle est un hétérocycle aromatique, mono-ou bicyclique de 5 à 10 chaînons contenant un ou plusieurs hétéroatomes choisis parmi N, O et S ;
aryle est phényle, indanyle, indényle ou naphtyle ;
hétérocyclyle est un hétérocycle aliphatique, mono- ou bicyclique de 5 à 10 chaînons contenant un ou plusieurs hétéroatomes choisis parmi N, O et S ;
ou les racémates, les énantiomères, les diastéréo-isomères et les mélanges de celui-ci, les tautomères ou les sels physiologiquement acceptables de ceux-ci.

4. Composé selon l'une quelconque des revendications 1 à 3, **caractérisé en ce que** dans la formule (I)
R est aryl-(C₁-C₁₀-alkyl)- ou hétéroaryl-(C₁-C₆-alkyl)-, non substitué ou au moins monosubstitué,
où les substituants sont choisis parmi halogène, C₁-C₆-alkyle, -OH, -O-aryle, C₁-C₆-alcoxy, -O- (C₁-C₆-alkylène) -N (C₁-C₆-alkyle)₂, -C (O) OH, -C(O)O-(C₁-C₆-alkyle),-NH₂, -N (C₁-C₆-alkyle)₂, -NH (C₁-C₆-alkyle) , -NH(C₁-C₁₀-cycloalkyle), -C(O)NH₂, -C(O)NH-hétéroaryle, -C(O)NH- (C₁-C₆-alkyle) , -SO₂ (C₁-C₆-alkyle) , -SO₂NH₂, -C(O)-hétérocyclyle, -C(NH)NH₂, hétérocyclyle, aryl- (C₁-C₆-alkyl) -, aryle, trifluorométhyle et trifluorométhoxy,
et aryle, hétérocyclyle et hétéroaryle peuvent à leur tour être au moins monosubstitués par C₁-C₃-alkyle, C₁-C₃-alcoxy, fluor, chlore, brome, trifluorométhyle, trifluorométhoxy ou OH ;
hétéroaryle est imidazolyle, thiophényle, furanyle, isoxazolyle, pyridinyle, pyrimidinyle, benzoimidazolyle, indolyle ou benzodioxolyle ;
aryle est phényle ou naphtyle ;
hétérocyclyle est morpholinyle, pipérazinyle ou pipéridinyle ;
ou les racémates, les énantiomères, les diastéréo-isomères et les mélanges de celui-ci, les tautomères ou les sels physiologiquement acceptables de ceux-ci.

5. Composé selon l'une quelconque des revendications 1 à 4, **caractérisé en ce que** dans la formule (I)
Ar est phényle, pyridin-4-yle ou pyrimidin-4-yle, non substitué ou au moins monosubstitué,
où les substituants sont choisis parmi halogène, C₁-C₆-alkyle, -OH, C₁-C₆-alcoxy, -C (O) OH, -C (O) O- (C₁-C₆-alkyle) , -NH₂, -N (C₁-C₆-alkyle)₂, -NH (C₁-C₆-alkyle) , -NH (C₁-C₁₀-cycloalkyle) , -NH(hétérocyclyl-(C₁-C₆-alkyl-)), -NH(aryl-(C₁-C₆-alkyl-)), -C(O)NH₂, -C(O)NH-(C₁-C₆-alkyle), aryle et hétéroaryle,
et aryle, hétérocyclyle et hétéroaryle peuvent à leur tour être au moins monosubstitués par C₁-C₃-alkyle, C₁-C₃-alcoxy, fluor, chlore, brome, trifluorométhyle, trifluorométhoxy ou OH ;
hétéroaryle est pyridinyle ou pyrimidinyle ;
aryle est phényle ou naphtyle ;
hétérocyclyle est morpholinyle, pipérazinyle ou pipéridinyle ;
ou les racémates, les énantiomères, les diastéréo-isomères et les mélanges de celui-ci, les tautomères ou les sels physiologiquement acceptables de ceux-ci.

6. Composé selon l'une quelconque des revendications 1 à 5, **caractérisé en ce que** dans la formule (I)
R est benzyle, phényléthyl-, phénylpropyl-, pyridinylméthyl-, pyridinyléthyl- ou pyridinylpropyl-, non substitué ou au moins monosubstitué,
où les substituants sont choisis parmi chlore, brome, fluor, trifluorométhyle et carboxy ;
Ar est pyridin-4-yle ou pyrimidin-4-yle ou phényle, non substitué ou au moins monosubstitué,
où les substituants sont choisis parmi méthylamino-, éthylamino-, propylamino-, butylamino-, hydroxy, méthoxy, éthoxy, méthyle, éthyle, propyle, (phényléthyl)amino-, benzylamino- et (morpholinyl-éthyl)amino- ;
ou les racémates, les énantiomères, les diastéréo-isomères et les mélanges de celui-ci, les tautomères ou les sels physiologiquement acceptables de ceux-ci.

7. Composé selon l'une quelconque des revendications 1 à 6, choisi dans le groupe constitué de :
(3-pyridin-3-yl-propyl)-amide de l'acide 6-(2-butylamino-pyrimidin-4-yl)-3-oxo-2,3-dihydropyridazine-4-carboxylique,
(3-pyridin-3-yl-propyl)-amide de l'acide 6-(4-hydroxy-3-méthoxyphényl)-3-oxo-2,3-dihydropyridazine-4-carboxylique,
(3-pyridin-3-yl-propyl)-amide de l'acide 6-(4-hydroxy-3,5-diméthylphényl)-3-oxo-2,3-dihydropyridazine-4-carboxylique,
(3-pyridin-3-yl-propyl)-amide de l'acide 6-(4-hydroxyphényl)-3-oxo-2,3-dihydropyridazine-4-carboxylique,
4-chloro-benzylamide de l'acide 6-(2-éthylamino-pyrimidin-4-yl)-3-oxo-2,3-dihydropyridazine-4-carboxylique,
4-chloro-benzylamide de l'acide 6-(3-chloro-4-hydroxyphényl)-3-oxo-2,3-dihydropyridazine-4-carboxylique,
4-chloro-benzylamide de l'acide 6-(4-hydroxy-3,5-diméthylphényl)-3-oxo-2,3-dihydropyridazine-4-carboxylique,
acide 4-({[6-(4-hydroxy-3,5-diméthylphényl)-3-oxo-2,3-dihydropyridazine-4-carbonyl]-amino}-méthyl)-benzoïque,
acide 4-({[6-(4-hydroxy-3-méthoxyphényl)-3-oxo-2,3-dihydropyridazine-4-carbonyl]-amino}-méthyl)-benzoïque,
(pyridin-3-ylméthyl)-amide de l'acide 6-(2-butylamino-pyrimidin-4-yl)-3-oxo-2,3-dihydro-pyridazine-4-carboxylique,
4-chloro-benzylamide de l'acide 6-(3-fluoro-4-hydroxyphényl)-3-oxo-2,3-dihydropyridazine-4-carboxylique,
4-chloro-benzylamide de l'acide 6-(4-hydroxy-3-méthylphényl)-3-oxo-2,3-dihydropyridazine-4-carboxylique,
4-chloro-benzylamide de l'acide 6-[2-(2-morpholin-4-yl-éthylamino)-pyrimidin-4-yl]-3-oxo-2,3-dihydropyridazine-4-carboxylique,
4-chloro-benzylamide de l'acide 6-(4-hydroxy-3-méthoxyphényl)-3-oxo-2,3-dihydropyridazine-4-carboxylique,
4-chloro-benzylamide de l'acide 6-(2-méthylaminopyrimidin-4-yl)-3-oxo-2,3-dihydropyridazine-4-carboxylique,
(3-phényl-propyl)-amide de l'acide R-3-oxo-6-[2-(1-phényléthylamino)-pyrimidin-4-yl]-2,3-dihydropyridazine-4-carboxylique,
4-chloro-benzylamide de l'acide 6-(4-hydroxyphényl)-3-oxo-2,3-dihydropyridazine-4-carboxylique,
3-oxo-6-pyridin-4-yl-N- [4- (trifluorométhyl)benzyl]- 2,3-dihydropyridazine-4-carboxamide
4-bromo-benzylamide de l'acide 3-oxo-6-pyridin-4-yl-2,3-dihydropyridazine-4-carboxylique,
3-oxo-6-pyridin-4-yl-N-(pyridin-3-ylméthyl)-2,3-dihydropyridazine-4-carboxamide,
N-(2,4-dichlorobenzyl)-3-oxo-6-pyridin-4-yl-2,3-dihydropyridazine-4-carboxamide,
4-chloro-2-fluoro-benzylamide de l'acide 3-oxo-6-pyridin-4-yl-2,3-dihydropyridazine-4-carboxylique, et
N-(4-chlorobenzyl)-3-oxo-6-pyridin-4-yl-2,3-dihydropyridazine-4-carboxamide ;
ou les racémates, les énantiomères, les diastéréo-isomères et les mélanges de celui-ci, les tautomères ou les sels physiologiquement acceptables de ceux-ci.

8. Composé selon l'une quelconque des revendications 1 à 7, ou sel physiologiquement acceptable de celui-ci, destiné à être utilisé comme produit pharmaceutique.

9. Utilisation d'un composé selon l'une quelconque des revendications 1 à 7, ou d'un sel physiologiquement acceptable de celui-ci, pour la fabrication d'un médicament destiné à la prophylaxie et/ou au traitement de maladies dans lesquelles la phosphorylation de la protéine Tau est observée.

10. Utilisation d'un composé selon l'une quelconque des revendications 1 à 7, ou d'un sel physiologiquement acceptable de celui-ci, pour la fabrication d'un médicament qui est un inhibiteur de GSK-3β.

11. Utilisation d'un composé selon l'une quelconque des revendications 1 à 7, ou d'un sel physiologiquement acceptable de celui-ci, pour la fabrication d'un médicament destiné à la prophylaxie et/ou au traitement de maladies neuro-dégénératives, des accidents cérébro-vasculaires, des traumatismes crâniens et médullaires et des neuropathies périphériques, de l'obésité, des maladies métaboliques, du diabète de type II, de l'hypertension essentielle, des maladies cardiovasculaires athérosclérotiques, du syndrome des ovaires polykystiques, du syndrome X, de l'immunodéficience ou du cancer.

12. Utilisation selon la revendication 11, **caractérisée en ce que** la maladie neurodégénérative est la maladie d'Alzheimer, la maladie de Parkinson, la démence fronto-pariétale, la dégénérescence corticobasale ou la maladie de Pick.

13. Utilisation selon la revendication 11, pour la prophylaxie et/ou le traitement du diabète de type II ou de la maladie d'Alzheimer.

14. Préparation pharmaceutique comprenant une dose efficace d'au moins un composé, ou un sel physiologiquement acceptable de celui-ci, tel que défini dans l'une quelconque des revendications 1 à 7, et un support physiologiquement acceptable.

15. Préparation pharmaceutique selon la revendication 14, laquelle préparation pharmaceutique est sous forme de pilule, comprimé, tablette, comprimé pelliculé, granulé, capsule, gélule dure ou molle, solution aqueuse, solution alcoolique, solution huileuse, sirop, suspension émulsionnée, pastille, suppositoire, solution injectable ou pour perfusion, pommade, teinture, crème, lotion, poudre, spray, systèmes thérapeutiques transdermiques, spray nasal, mélange aérosol, microcapsule, implant, tige ou pansement adhésif.

16. Méthode de synthèse d'un composé de formule (I) selon l'une quelconque des revendications 1 à 7, **caractérisée en ce que** :
a) un composé de formule (IV) 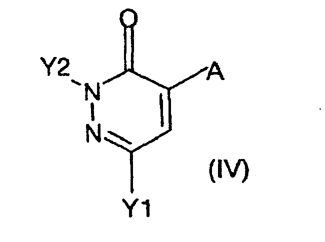 dans laquelle Y1 est halogène, B (OH)₂ ou Sn(C₁-C₁₀-alkyle) et
Y2 est H ou un groupement protecteur,
est transformé avec Ar-Z en présence d'un complexe de palladium, où Z est B(OH)₂, B(C₁-C₁₀-alcoxy)₂, Sn(C₁-C₁₀-alkyle)₃, Zn-(C₁-C₁₀-alkyle) ou halogène,
ou
b) à condition que dans la formule (I) A soit Al, un composé de formule (II) 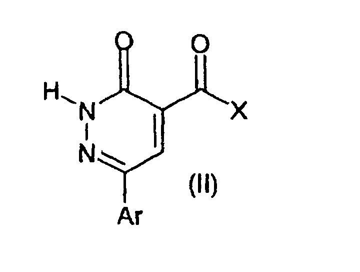 dans laquelle X est -OH, C₁-C₁₀-alcoxy, chlore ou -O-C (O) O-(C₁-C₁₀-alkyle), est transformé avec RNH₂.
